# EUROPEAN PATENT APPLICATION

(11) **EP 4 421 084 A1**
(43) Date of publication of application: **28.08.2024**
(21) Application number: 23382158.6
(22) Date of filing: 21.02.2023
(51) Int. Cl.: C07K 14/245, C07K 14/24, C12Q 1/66, C12Q 1/02

(54) **CHIMERIC PROTEINS AND THEIR USE AS AN ANTIGEN DETECTING SYSTEM**

(71) Applicant: Consejo Superior De Investigaciones Científicas, 28006 Madrid (ES)
(72) Inventor: Fernández Herrero, Luis Ángel, Madrid (ES); Ceballos Munuera, Álvaro, Madrid (ES)
(74) Representative: Pons

(57) **Abstract**

The present invention relates to a chimeric protein and a biosensor system based on said chimeric protein that enable the binding of an extracellular target antigen and the generation of a signal upon recognition. The chimeric protein of the present invention comprises (i) an antigen binding domain, (ii) at least one β-barrel domain from an autotransporter, and (iii) a reporter protein domain comprising a partner of a split protein luminescence system, wherein domain (ii) is bound to domain (i) by one end and to domain (iii) by the other end. The present invention also discloses a host cell comprising at least two chimeric proteins of the invention complementary each other as an *in vitro* antigen detection system.

## Description

The present invention relates to a chimeric protein and a system of bacterial receptors in the outer membrane that would enable the binding of an extracellular target antigen and the generation of a signal upon recognition. Thus, the present invention belongs to the Biotechnology field, particularly, to the detection of antigens using chimeric/fusion proteins.

### BACKGROUND ART

Engineered bacteria offer the potential to dramatically transform and improve approaches to biosensing, diagnostics, and therapeutics. Bacterial biosensors have been widely used for environmental monitoring and remediation, and are now being translated into the field of healthcare to detect pathological biomarkers and diagnose diseases. Bacteria also hold great promise for in vivo diagnostics and therapies. For example, using mouse models, researchers engineered bacteria to detect cancer metastasis, monitor gut inflammation, or specifically target cancer cells. In addition, microbiome engineering could help prevent or treat infectious and autoimmune diseases.

All these applications require sensors enabling bacteria to detect and respond to various signals of interest. Cellular sensors were first engineered by repurposing systems found in nature, like coupling existing transcription factors to a reporter or by rewiring two-component systems. However, a sensor for every molecule of interest might not exist in nature, limiting the range of detectable molecules and related applications. A pressing challenge is thus to engineer synthetic receptors enabling bacteria to detect novel signals.

Several approaches have been used to engineer synthetic receptors. For example, transcription factor specificity can be switched to other ligands by directed evolution. While useful, these sensors are limited to recognizing structurally similar molecules and by the difficulty to preserve allosteric control. Synthetic receptors have also been engineered using conditionally stable ligand-binding domains (LBDs) that are degraded in the absence of their ligands. Finally, synthetic metabolic pathways can transform nondetectable molecules into ligands recognized by known transcription factors.

However, all these systems rely on existing LBDs and are limited to the detection of small molecules. Ideally, synthetic receptors should use versatile LBDs for which recognition specificity can be easily programmed for various applications. In this regard, single-domain antibodies or antibody-like scaffolds are ideal LBDs due to their high stability and solubility, and for which combinatorial libraries can be selected to target many different antigens, from small molecules to proteins. Consequently, single-domain antibodies like camelid VHHs or scFvs have been used to design mammalian transmembrane receptors such as chimeric antigen receptors (CARs), synthetic notch receptors, and protease-based transmembrane receptors.

Therefore, there is a need in the state of the art for systems capable of detecting and quantifying antigens which allow an efficient diagnosis of diseases.

### DESCRIPTION OF THE INVENTION

The inventors have developed a chimeric protein which, once it is expressed in Gram negative bacteria, is anchored to the outer membrane (OM) of bacteria, displaying an antigen binding protein in the bacterium surface and a reporter protein in the periplasm. In this way, by co-expressing two of these chimeric proteins, complementary to each other, in a Gram negative bacteria, the bacteria can function as a biosensor, producing a different activity of the reporter protein that can be monitored (e.g. with a bioluminescence signal, a fluorescence signal, a colorimetric signal, etc.) in the presence of antigen and in a dose-dependent manner. Therefore, the inventors have developed a bacteria system useful as an *in situ* antigen detector.

The inventors engineered different types of chimeric OM protein (OMP) receptors, based on the β-barrel of proteins belonging to the type V secretion system (T5SS) such as intimin, invasin and EhaA, which simultaneously display a nanobody on the bacterial cell surface and the protein fragments of a reporter system in the bacterial periplasm. In this way, functional chimeric proteins that display a nanobody binding the human Epidermal Growth Factor Receptor (EGFR), a tumor-associated antigen, and that express in the periplasm the two fragments of a split luciferase reporter (NanoBiT) or peptides for covalent protein fusions (SpyTag and SnoopTag) were obtained. By co-expressing intimin- and invasin-based chimeric receptors having the anti-EGFR nanobody and the NanoBiT fragments, it is demonstrated that Gram negative bacteria, such as *E. coli,* can function as a biosensor, decreasing its bioluminescence signal in the presence of soluble EGFR and in a dose-dependent manner.

The technology described in this invention uses bacterial cells expressing chimeric protein fusions in the OM for detection of antigens, making unnecessary purification of Abs/Nbs or further conjugation steps. Hence, this antigen detection system is simple to produce, requiring only the cultivation of the bacteria (e.g. *E. coli)* that directly produce the chimeric antigen receptors in the OM. This is a clear advantage over other systems based on purified Abs. The modularity of the chimeric proteins in which the system relies is also advantageous, since it allows for easy substitution of the sequence of the Ab/Nb for a different one, enabling the detection of new antigens as needed. This same modularity allows the substitution of the reporter protein for other reporters based on protein complementation. The activity of the linked reporter protein can be measured directly adding the tested sample on multiwell immunoplates containing these bacteria. Washing steps are not needed to remove the unbound antigen or Ab/Nb before measuring antigen binding signals in a microplate reader. The chimeric receptors in the OM of *E. coli bacteria* reported in this invention facilitate the detection of extracellular antigens, both large and small molecules. They are advantageous for detection of extracellular antigens either soluble or bound to particles (e.g. viruses, exosomes, vesicles), bacteria, cells from pathogens (fungal, protozoa, etc.), tumors, blood, immune system, etc.

Therefore, the invention relates to chimeric proteins expressed in Gram negative bacteria (e.g. *E. coli*) that enable the detection of extracellular antigens that are specifically recognized by these chimeric proteins, and more particularly, to bacterial antigen receptor (BAR) chimeric proteins (chimeric BAR) that comprise three modular regions or functional domains: i) a receptor domain located on the surface of the bacterium and capable of specifically recognize an antigen based on single-domain antibody (e.g. nanobody) or on non-immunoglobulin domains with high affinity for the antigen (e.g. anticalins); ii) a transmembrane domain derived from the proteins intimin, invasin and/or autotransporters, that anchors the chimeric protein to the bacterial outer membrane; and iii) a sensor/reporter domain that is located in the periplasm of the bacterium (the space between the outer and inner membranes in Gram-negative bacteria) and that contains, at least, a peptide fragment of an enzyme and/or protein reporter (e.g. luciferase, protease, β-lactamase, fluorescent protein, etc.).

Once expressed in Gram-negative bacteria such as *E*. *coli,* chimeric BARs of the present invention localize to the outer membrane, with the receptor domain (e.g. nanobody) on the surface and the sensor/reporter domain (e.g. peptide fragment) in the periplasm. By exposing bacteria expressing these chimeric BARs to an extracellular antigen, it will trigger a change in the activity produced by the sensor/reporter domain (e.g., light emission in the case of a luciferase) that is specific and dose-dependent on the antigen concentration. The activity produced by the sensor domains of the chimeric BARs can be detected directly with intact bacteria using different assays (e.g. bioluminescence on multiwell plates, tubes, etc). Chimeric BARs are modular and can be applied for detection of different antigens (e.g. proteins, polysaccharides, etc.) by exchange of the receptor domain (e.g. nanobody) that binds them. The chimeric BAR could be developed for *in vitro* assays detecting specific antigen(s) from viruses, bacteria, tumors, immune and inflammatory responses, etc. These antigens can be detected in samples of different origins, such as from human and animal bodies (eg saliva, blood, plasma, urine, milk etc) or environmental samples (eg water, soil, plants, seeds, etc.).

In view of the above, in one aspect, the present invention relates to a chimeric protein, hereinafter "chimeric protein of the invention", comprising
(i) an antigen binding domain,
(ii) at least one β-barrel domain, wherein the β-barrel domain is
   (a) the β-barrel domain from an intimin protein,
   (b) the β-barrel domain from an invasin protein,
   (c) the β-barrel domain from an EhaA autotransporter protein,
   and
(iii) a reporter domain comprising a partner of a split protein luminescence system, wherein domain (ii) is bound to domain (i) by one end and to domain (iii) by the other end.

In the context of the present invention, the terms "chimeric protein" and "fusion protein" are equivalent and can be used equally throughout the present description. As used herein, the term "chimeric protein" refers to proteins created through the joining of two or more genes which are originally coded for separate or same proteins; translation of this fusion gene results in a single polypeptide with functional properties derived from each of the original proteins.

The chimeric protein of the invention comprises three domains. The term "domain" refers to a set of amino acids or region of a protein's polypeptide chain that is self-stabilizing and that folds independently from the rest. Each domain forms a compact folded three-dimensional structure.

In the chimeric protein of the invention, one of the three domains is an antigen binding domain. As use herein, the term "antigen binding domain" refers to any moiety, construct, or molecule (typically proteinaceous) that can specifically recognize and bind to a target antigen. As used herein, the term "specifically binds" refers to an association or union of a binding domain, or a molecule containing the binding domain to a target molecule (i.e., the target antigen) without significantly binding or associating with distinct antigens. In some embodiments, specific binding is manifested in a binding affinity or Kₐ (i.e., an equilibrium association constant of a particular binding interaction with units of 1/M) equal to or greater than 10⁵ M^{- 1} for the antigen, while not significantly associating with any other distinct antigen. Binding affinity can be classified as "high affinity" or "low affinity". "High affinity" refers to a binding affinity with a Kₐ of at least 10⁷ M⁻¹, at least 10⁸ M⁻¹, at least 10⁹ M⁻¹, at least 10¹⁰ M⁻¹, at least 10¹¹ M⁻¹, at least 10¹² M⁻¹, or at least 10¹³ M⁻¹. "Low affinity" refers to a binding affinity with a Kₐ of up to 10⁷ M, up to 10⁶ M⁻¹, up to 10⁵ M ⁻¹. Alternatively, binding affinity can be defined as an equilibrium dissociation constant (K) of a particular binding interaction with units of M. For example, the antigen binding domain can have a binding affinity within a range characterized by a K_{D} from about 50nM (lower binding affinity) to about 0.001 nM (higher binding affinity).

Exemplary antigen binding domains encompassed by the present invention include, without being limited to, aptamers and antibodies, and antigen-binding fragments or derivatives thereof. As used herein, the term "antibody" encompasses immunoglobulin molecules and antigen binding antibody derivatives and fragments thereof, derived from any antibody-producing animals (e.g., cartilaginous fish, mouse, rat, rabbit, camelids, and primate including human), that specifically bind to an antigen of interest. Exemplary antibodies include monoclonal antibodies, chimeric antibodies (e.g., mouse-rabbit, mouse-human, mouse-primate, primate-human monoclonal antibodies), and humanized antibodies. An antibody "derivative" encompasses fragments, modifications, fusions, or other antibody-related constructs that incorporate structure of at least part of an antibody molecule, typically at least the antigen-binding domain of the antibody molecule. An antigen-binding antibody derivative or fragment will typically contain at least a portion of the complementarity determining regions (CDRs) of the original antibody sufficient to bind to the antigen of interest. Illustrative examples of antibody fragments and derivatives encompassed by the present invention include, without being limited to, Fab, Fab', F(ab)2, F(ab')2 and Fv fragments, linear antibodies, single-chain antibody molecules, multi-specific antibodies formed from antibody fragments, and the like. Single-chain antibodies include single-chain variable fragments (scFv) and single-chain Fab fragments (scFab). A "single-chain Fv" or "scFv" antibody fragment, for example, comprises the VJJ and Vp domains of an antibody, wherein these domains are present in a single polypeptide chain. The Fv polypeptide can further comprise a polypeptide linker between the VJJ and Vp domains, which enables the scFv to form the desired structure for antigen. Single-chain antibodies can also include diabodies, triabodies, and the like. In particular embodiments, heterologous affinity reagent is a "single-domain" antibody (sdAb), also referred to herein as a "nanobody", or an antigen-binding fragment or derivative thereof. Single-domain antibodies are antibody fragments that contain a single monomeric variable domain that can bind to target antigens with high specificity. Single-domain antibodies are characteristic of camelids and cartilaginous fishes. For example, camelid sdAbs are called VₕH fragments and have a single variable domain (V_{H}) of a heavy chain antibody molecule. Design and production of binding domains based on camelid sdAbs widely known in the state of the art. Fish sdAbs are called V_{AAR} ("new antigen receptor" variable) fragments, that similarly have a single variable domain joined with a number of constant domains.

The sdAbs of the present invention can be VHH or V_{AAR} fragments or be derived therefrom. These sdAbs are able to bind to antigens without associated light chain variable domains. Alternatively, sdAbs can be engineered from more conventional or canonical antibody structures, such as by monomerizing the traditional antibody domains by removing or replacing lipophilic residues or by introducing premature stop codons, and screening for binding affinity of the single domain chains.

In another particular embodiment of the chimeric protein of the invention, the antigen binding domain is selected from a single domain antibody, a single-chain Fv (scFv) or a fragment thereof, and an engineering protein binder with affinity for an antigen. Examples of engineering protein binder with affinity for an antigen include, without being limited to, proteins based on ankyrin repeat domains (DARPins) and lipocalins (anticalins) (Sha, F. et al. 2017. Protein Science, 26(5): 940-924).

In a more particular embodiment of the chimeric protein of the invention, the single domain antibody is a nanobody.

Due to the smaller size of sdAbs and other antibody fragments and derivatives (e.g., Fab, Fab', F(ab)2, F(ab')2 and Fv fragments, linear antibodies, single-chain antibody molecules (e.g., scFv)), they are more amenable to heterologous expression in bacterial cells using coding sequence operably linked to an appropriate promoter. In some embodiments, the binding domain (e.g., sdAbs and other antibody fragments and derivatives) comprise the variable domain or domains) are displayed in monomeric form on the surface of the bacterium. In a particular embodiment of the chimeric protein of the invention, alone or in combination with the previous particular embodiments, the antigen binding domain is an antigen monomeric binding domain.

It will be apparent to the skilled person in the art that the binding domain can comprise antigen binding molecules other than antibody-based binding domains, such as peptidobodies, antigen-binding scaffolds (e.g., DARPins, HEAT repeat proteins, ARM repeat proteins, tetratricopeptide repeat proteins, and other scaffolds based on naturally occurring repeat proteins, etc., which include a functional binding domain or antigen-binding fragment thereof.

In a more particular embodiment of the chimeric protein of the invention, alone or in combination with all or each one of the previous particular embodiments, the antigen binding domain comprises, or consists of, an amino acid sequence having an identity of, at least, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99% with the sequence SEQ ID NO: 1. In an even more particular embodiment, the antigen binding domain comprises, or consists of, an amino acid sequence having an identity of 100% with the sequence SEQ ID NO: 1.

SEQ ID NO: 1
MAQVQLQESGGGLVQAGGSLLLSCAASGRTFSSYAMGWFRQAPGKEREFVAAIN
WSGGSTSYADSVKGRFTISRDNTKNTVYLQMNSLKPEDTAAFYCAATYNPYSRDH
YFPRMTTEYDYWGQGTQVTVSSEPKTPKPQP

In the present invention, the term "identity" or "sequence identity" is understood to mean the degree of similarity between two nucleotide or amino acid sequences obtained by aligning the two sequences. Depending on the number of common residues between the aligned sequences, a different degree of identity, expressed as a percentage, will be obtained. The degree of identity between two amino acid sequences may be determined by conventional methods, for example, by standard sequence alignment algorithms known in the state of the art, such as, for example, BLAST [Altschul S.F. et al. Basic local alignment search tool. J Mol Biol. 1990 Oct 5; 215(3): 403-10]. The BLAST programmes, for example, BLASTN, BLASTX, and TBLASTX, BLASTP and TBLASTN, are in the public domain at The National Center for Biotechonology Information (NCBI) website.

Persons skilled in the art understand that mutations in the nucleotide sequence of genes that lead to conservative amino acid substitutions at non-critical positions for the functionality of the protein are evolutionarily neutral mutations which do not affect its global structure or its functionality, giving rise to proteins which although comprise different amino acid sequence perform the same activity. These proteins are considered "functionally equivalent variants" of the sequence SEQ ID NO: 1 and fall within the scope of the present invention. Thus, the term "functionally equivalent variant", as used herein, means an enzyme which is derived from a native enzyme (SEQ ID NO: 1 in the present invention) by one or more deletions, insertions and/or substitutions of one or more amino acids at site(s) within its amino acid sequence and performs the same activity, i.e. can act as antigen binding domain and specifically recognize and bind to a target antigen. Variants can be prepared using any mutagenesis procedure known in the art, such as site-directed mutagenesis, synthetic gene construction, semi-synthetic gene construction, random mutagenesis, shuffling, etc. All proteins having a sequence identity of at least 80% with the amino acid sequence of SEQ ID NO: 1 and capable of specifically recognize and bind to a target antigen are considered functionally equivalent variants in the context of the invention. An example of an assay to check if a given protein is a functionally equivalent variant of the binding domain with sequence SEQ ID NO: 1 is ELISA.

The present invention also encompasses functionally equivalent fragments of the binding domain with sequence SEQ ID NO: 1. The term "functionally equivalent fragment" means a polypeptide/protein having one or more (e.g., several) amino acids absent from the amino and/or carboxy terminus of a native protein (in the present invention the sequence SEQ ID NO: 1) and shows the same activity/function that the native protein (in the present invention, capable of specifically recognize and bind to a target antigen). An example of an assay to check if a fragment of the protein of the invention is a functionally equivalent fragment of the SEQ ID NO: 1 is ELISA.

Another domain of the chimeric protein of the invention is a β-barrel domain. In the context of the present invention, the term "β-barrel" refers to a β-sheet comprised of tandem repeats that twists and coils to form a closed toroidal structure in which the first strand is bonded to the last strand (hydrogen bond), and it is inserted in the outer membrane (OM) by the β-barrel assembly machinery (BAM) complex, so that the β-strands are arranged in the folded domain inserted in the bacterial OM in an antiparallel fashion forming a β-barrel structure.

The β-barrel domain in the chimeric protein of the invention may be the β-barrel domain from a bacteria type V autotransporter protein (or T5SS from Type V Secretion System). Examples of bacteria type V autotransporter protein include, but without limiting to, intimin protein, invasin protein, and EhaA autotransporter.

Therefore, the β-barrel domain in the chimeric protein of the invention may be the β-barrel domain from an intimin protein. As used herein, the term "intimin protein" refers to the inverse autotransporter (AT) intimin found in various diarrheagenic bacterial species, such as the enteropathogenic and enterohemorrhagic *E. coli* (EPEC and EHEC), or *Citrobacterrodentium.* In a particular embodiment of the present invention, alone or in combination with all or each one of the previous particular embodiments, intimin protein is from enterohemorrhagic *E. coli* (EHEC O157:H7).

In another particular embodiment of the chimeric protein of the invention, the β-barrel domain from an intimin protein comprises, or consist of, an amino acid sequence having an identity of, at least, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99% with the sequence SEQ ID NO: 2.

The term "identity" has been defined above. Functionally equivalent variants of the sequence SEQ ID NO: 2, as well as functionally equivalent fragments of the sequence SEQ ID NO: 2, are also encompassed in the context of the present invention. The terms "functionally equivalent variants" and "functionally equivalent fragments" has been defined above. Example of an assay to check if a given protein is a functionally equivalent variant or fragment of the binding domain with sequence SEQ ID NO: 2, i.e. it is capable of being inserted in the outer membrane (OM) by the β-barrel assembly machinery (BAM) and the β-strands arranged in an antiparallel fashion forming a β-barrel structure in the OM, is to transform *E. coli* with a nucleotide sequence encoding the given protein, to culture the transformed *E.coli* to express the nucleotide sequence, to isolate the protein from the OM fraction of the bacteria and to determine the structure of the β-barrel of the protein by structural methods (e.g. X-ray crystallography or cryoelectron microscopy). Another way of checking if a given protein is a functionally equivalent variant or fragment of the binding domain with sequence SEQ ID NO: 2 is by the analysis of its primary sequence by homology (e.g. BLAST programs of the NCBI), specific β-barrel membrane protein prediction methods (e.g. 3DBMPP) or by machine-learning algorithms (e.g. AlphaFold).

In an even more particular embodiment, the β-barrel domain from an intimin protein comprises, or consists of, an amino acid sequence having an identity of 100% with the sequence SEQ ID NO: 2.

SEQ ID NO: 2
KSNMTDDKALNYAAQQAASLGSQLQSRSLNGDYAKDTALGIAGNQASSQLQAWL
QHYGTAEVNLQSGNNFDGSSLDFLLPFYDSEKMLAFGQVGARYIDSRFTANLGAG
QRFFLPANMLGYNVFIDQDFSGDNTRLGIGGEYWRDYFKSSVNGYFRMSGWHES
YNKKDYDERPANGFDIRFNGYLPSYPALGAKLIYEQYYGDNVALFNSDKLQSNPGA
ATVGVNYTPIPLVTMGIDYRHGTGNENDLLYSMQFRYQFDKSWSQQIEPQYVNEL
RTLSGSRYDLVQRNNNIILEYKKQDILSLNIPHDINGTEHSTQKIQLIVKSKYGLDRIV
WDDSALRSQGGQIQHSGSQSAQDYQAILPAYVQGGSNIYKVTARAYDRNGNSSN
NVQLTITVLSNGQVVDQVGVTDFTADKTSAKADNADTITYTATVKKNGVAQANVPV
SFNIVSGTATLGANSAKTDANGKATVTLKSSTPGQVVVSAKTAEMTSALNASAVIFF
D

Alternatively, the β-barrel domain in the chimeric protein of the invention may also be the β-barrel domain from an invasin protein. As used herein, the term "invasin protein" refers to the AT intimin found in the enteropathogenic *Yersinia* species such as *Y. enterocolitica* and *Y. pseudotuberculosis.* In a particular embodiment of the present invention, alone or in combination with all or each one of the previous particular embodiments, invasin protein is from *Yersinia pseudotuberculosis.*

In another particular embodiment of the chimeric protein of the invention, the β-barrel domain from an invasin protein comprises, or consist of, an amino acid sequence having an identity of, at least, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99% with the sequence SEQ ID NO: 3.

SEQ ID NO: 3
KYDANAPQQVQPYSVSSSAFENLHPNNEMESSINPFSASDTERNAAIIDRANKEQET
EAVNKMISTGARLAASGRASDVAHSMVGDAVNQEIKQWLNRFGTAQVNLNFDKNFS
LKESSLDWLAPWYDSASFLFFSQLGIRNKDSRNTLNLGVGIRTLENGWLYGLNTFYD
NDLTGHNHRIGLGAEAWTDYLQLAANGYFRLNGWHSSRDFSDYKERPATGGDLRA

NAYLPALPQLGGKLMYEQYTGERVALFGKDNLQRNPYAVTAGINYTPVPLLTVGVD
QRMGKSSKHETQWNLQMNYRFGESFQSQLSPSAVAGTRLLAESRYNLVDRNNNIV
LEYQKQQVVKLTLSPATISGLPGQVYQVNAQVQGASAVREIVWSDAELIAAGGTLTP
LSTTQFNLVLPPYKRTAQVSRVTDDLTANFYSLSALAVDHQGNRSNSFTLSVTVQQP
Q

The term "identity" has been defined above. Functionally equivalent variants of the sequence SEQ ID NO: 3, as well as functionally equivalent fragments of the sequence SEQ ID NO: 3, are also encompassed in the context of the present invention. The terms "functionally equivalent variants" and "functionally equivalent fragments" have been defined above. Example of an assay to check if a given protein is a functionally equivalent variant or fragment of the β-barrel domain with sequence SEQ ID NO: 3, i.e. it is capable of being inserted in the outer membrane (OM) by the β-barrel assembly machinery (BAM) and the β-strands arranged in an antiparallel fashion forming a β-barrel structure in the OM, has been disclosed above.

In an even more particular embodiment, the β-barrel domain from an invasin protein comprises, or consists of, an amino acid sequence having an identity of 100% with the sequence SEQ ID NO: 3.

Alternatively, the β-barrel domain in the chimeric protein of the invention may also be the β-barrel domain from an EhaA autotransporter protein. As used herein, the term "EhaA autotransporter protein" refers to the EhaA autotransporter protein found in the enterohemorrhagic *E. coli* O157:H7 and other Shiga toxin-producing *E. coli* strains of various serotypes. In a particular embodiment of the present invention, alone or in combination with all or each one of the previous particular embodiments, EhaA autotransporter protein is from enterohemorrhagic *E. coli* O157:H7

In another particular embodiment of the chimeric protein of the invention, the β-barrel domain from a EhaA autotransporter protein comprises, or consist of, an amino acid sequence having an identity of, at least, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99% with the sequence SEQ ID NO: 4.

The term "identity" has been defined above. Functionally equivalent variants of the sequence SEQ ID NO: 4, as well as functionally equivalent fragments of the sequence SEQ ID NO: 4, are also encompassed in the context of the present invention. The terms "functionally equivalent variants" and "functionally equivalent fragments" have been defined above. Examples of equivalent β-barrel domains of autotransporter proteins are described in the Pfam family PF03797 (Pfam Autotransporter beta-domain). Example of an assay to check if a given protein is a functionally equivalent variant or fragment of the β-barrel domain with sequence SEQ ID NO: 4, i.e. it is capable of being inserted in the outer membrane (OM) by the β-barrel assembly machinery (BAM) and the β-strands arranged in an antiparallel fashion forming a β-barrel structure in the OM, has been disclosed above.

In an even more particular embodiment, the β-barrel domain from EhaA autotransporter comprises, or consists of, an amino acid sequence having an identity of 100% with the sequence SEQ ID NO: 4. In an even more particular embodiment, the β-barrel domain from an autotransporter comprises, or consists of, an amino acid sequence having an identity of 100% with a sequence found in Pfam family PF03797 (Pfam Autotransporter beta-domain)

SEQ ID NO: 4
TPTPGPDLNVDNDLRPEAGSYIANLAAANTMFTTRLHERLGNTYYTDMVTGEQKQT
TMWMRHEGGHNKWRDGSGQLKTQSNRYVLQLGGDVAQWSQNGSDRWHVGVMA
GYGNSDSKTISSRTGYRAKASVNGYSTGLYATWYADDESRNGAYLDSWAQYSWFD
NTVKGDDLQSESYKSKGFTASLEAGYKHKLAEFNGSQGTRNEWYVQPQAQVTWM
GVKADKHRESNGTLVHSNGDGNVQTRLGVKTWLKSHHKMDDGKSREFQPFVEVN
WLHNSKDFSTSMDGVSVTQDGARNIAEIKTGVEGQLNANLNVWGNVGVQVADRGY
NDTSAMVGIKWQF

Another domain of the chimeric protein of the invention is a reporter protein domain comprising a partner of a split protein luminescence system. The reporter protein domain of the chimeric protein of the invention is localized at the bacteria periplasm. In the context of the present invention, the terms "reporter domain", "reporter protein domain" or "sensor domain" are equivalents, and they refer to a detectable marker whose presence in the cell is readily observed in the presence of a stimulus. In the present invention, the reporter domain of the chimeric protein comprises a partner of a split protein luminescence system. In this way, the chimeric protein of the invention may be used in Protein-fragment complementation assays (PCAs).

In the context of the present invention, the expression "a partner of split protein luminescence system" refers to one fragment of a full-length reporter protein (or system) that has an enzymatic activity (e.g. luciferase, b-lactamase, protease, etc.) or intrinsic fluorescence (e.g. green fluorescent protein, etc.), and which is complementary to a second fragment of the reporter enzyme. The term "complementary" refers to the fact that when the two fragments (partners) of the full-length reporter protein (split protein) are bound together, there is a change in the enzymatic activity which can be detected.

PCAs are particularly well suited to detect protein interactions in the cell. They are based on the formation of a bimolecular complex when two non-active fragments of a reporter protein are brought together due to an interaction between bait and prey (where both are fused to the split domains of the reporter). The interaction between bait and prey proteins brings the split reporter fragments close enough to enable their non-covalent and specific reassembly followed by the recovery of its native structure and activity (Morell, M.; Ventura, S.; Avilés, F.X. (2009). FEBS Letters 583, 1684-1691). Examples of reporter proteins which can be used in the present invention include, without being limited to, dihydrofolate reductase (DHFR) (Pelletier, J.N., Campbell-Valois, F.X. and Michnick, S.W. (1998) Proc. Natl. Acad. Sci. USA 95, 12141-12146), β-lactamase (Galarneau, A., et al. (2002) Nat. Biotechnol. 20, 619-622), TEV protease (Wehr, M.C., et al. (2008) BMC Biotechnol. 8, 55), green fluorescent protein (GFP) or its variants (Hu, C.D. and Kerppola, T.K. (2003) Nat. Biotechnol. 21, 539-545), luciferase (Villalobos, V., et al. (2007) Annu. Rev. Biomed. Eng. 9, 321-349), etc. If fluorescent or luminescent proteins are used, the signal is of spectroscopic nature. Particularly, when the reporter proteins are fluorescent proteins (FPs), it refers to bimolecular fluorescence complementation (BIFC).

Illustrative examples of split-fluorescent protein systems include, without limited to, are listed below.

**Table 1: split-fluorescent proteins.**

| **Color** | **Base fluorescent protein** | **Description** |
|---|---|---|
| Blue | EBFP2 | EBFP2(1-10) and Capri(1-10) for use with GFP(11) |
| | Cerulean | Cerulean(1-10) for use with GFP(11) |
| | ECFP | C155 of ECFP pairs with N173 derived from Cerulean or Venus |
| Green | sfGFP | Split super-folder GFP, our most-requested GFP(1-10) and GFP(11) |
| | spGFP | Split superpositive GFP |
| | mNeonGreen2 | mNG2(1-10) and mNG2(11) |
| | mNeonGreen3 | mNG3K(1-10) and mNG3A(1-10) for use with mNG2(11) |
| Yellow | Venus | pBiFC-VN173, pBiFC-VC155 |
| | Venus | Improved N-terminal fragment VN155(!152L), for use with VC155 |
| | mVenus | pET-BiFC contains both fragments of split mVenus (aa 155), includes l152L in N-terminal fragment |
| | mVenus | Split mVenus (aa 155), includes l152L in N-terminal fragment |
| | EYFP | Gateway vectors with EYFP split at 175 |
| Red | mScarlet | Split-wrmScarlet variant of mScarlet |
| | mRuby4 | mRuby4(1-10), pairs with sfCherry2(11) |
| | sfCherry2 | sfCherry2(1-10) and sfCherry2(11), also photo-activatable variant PAsfCherry2(1-10) |
| | sfCherry3 | Improved sfCherry3C(1-10) for use with sfCherry2(11) |
| Near-Infrared | iRFP | iSplit, comprised of PAS and GAF domains of iRFP713. Requires presence of heme oxygenase for chromophore formation (abundant in eukaryotes) |
| | iRFP | GAF domains from miRFP709 and miRFP670, pair with common PAS domain. Requires presence of heme oxygenase for chromophore formation (abundant in eukaryotes) |
| Multiple | FAST | Fluorescence-activating and absorption shifting tag (FAST) for use with green-yellow or orange-red fluorogen |
| | HaloTag | TagBiFC allows exogenous labeling for single-molecule imaging |

In a particular embodiment of the chimeric protein of the invention, alone or in combination with all or each one of the previous particular embodiments, the reporter protein domain is a luciferase; the β-lactamase (Verma, V. (2015) Reson 20, 816-821); the TEV protease (Wehr, M., et al. (2006) Nat Methods 3, 985-993); or the AMD fluorescent proteins (Dammeyer T, Tinnefeld P. (2012) Comput Struct Biotechnol J. 22; 3:e201210013) such as GFP (Ishikawa H, et al. (2012) Protein Eng Des Sel. 25(12):813-20). Functionally equivalent fragments of these proteins can also be used in the present invention as long as the functionally of the original protein remains.

Examples of luciferase proteins include, without being limited to, firefly luciferase, renilla luciferase, luciferase coming from *Oploforus gracilirostris* [(Hall, M.P. et al. (2012) ACS Chem. Biol. 7, 11, 1848-1857), NanoLuc^{®}], click beettle luciferase, Italian firefly luciferase, gaussian luciferase, Cypridina, Metridia and Rail worn.

In a more particular embodiment of the chimeric protein of the invention, alone or in combination with all or each one of the previous particular embodiments, the reporter protein domain comprises, or consist of, an amino acid sequence having an identity of, at least, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99% with the sequence SEQ ID NO: 6 or 7.
SEQ ID NO: 5:
   VTGYRLFEEIL
SEQ ID NO: 6

VFTLEDFVGDWEQTAAYNLDQVLEQGGVSSLLQNLAVSVTPIQRIVRSGENALKIDIH
VIIPYEGLSADQMAQIEEVFKVVYPVDDHHFKVILPYGTLVIDGVTPNMLNYFGRPYE
GIAVFDGKKITVTGTLWNGNKIIDERLITPDGSMLFRVTINS

The chimeric protein of the invention comprises three domains, (i) an antigen binding domain, (ii) at least one β-barrel domain, and (iii) a reporter domain, which are arranged in a particular order, i.e. domain (ii) is bound to domain (i) by one end and to domain (iii) by the other end. With this arrangement, once the chimeric protein is expressed in Gram negative bacteria, it is anchored to the outer membrane (OM) of bacteria, displaying an antigen binding protein in the bacterium surface and the reporter protein in the periplasm.

As the skilled person in the art understands, the chimeric protein of the invention may further comprise one or more linker regions, such as poly-Glycine linkers or the like. The linker can be used, e.g., to link the different domains of the chimeric protein. It is also envisaged that two or more linkers are used in the chimeric protein e.g., one linker between the β-barrel domain and the antigen binding domain, and a further linker between the β-barrel domain and the reporter protein domain. The linker can be the same or be different. The terms "bound to", "couple to" and "linked to" are considered equivalents and can be used interchangeably throughout the present description. The term "linker", "peptide linker" or "linker region" as used herein denotes a short segment of an amino acid sequence. Any "linker" group is optional. When present, its chemical structure is not critical, since it serves primarily as a spacer. The linker is preferably made up of amino acids linked together by peptide bonds. Thus, in preferred embodiments, the linker is made up of from 1 to 20 amino acids linked by peptide bonds, wherein the amino acids are selected from the 20 naturally occurring amino acids. Some of these amino acids may be glycosylated, as is well understood by those in the art. In a more preferred embodiment, the 1 to 20 amino acids are selected from glycine, alanine, proline, asparagine, glutamine, and lysine. Even more preferably, a linker is made up of a majority of amino acids that are sterically unhindered, such as glycine and alanine. In order to ensure proper folding of the protein, these linkers should not contain truncated α-helices or β-sheets. Many linkers are known from the state of the art and any of them can be used in the context of the present invention. Suitable linker sequences encoded by a corresponding DNA sequence are widely known in the state of the art. Besides the basic role in linking the different domains together (as in flexible or rigid linkers), linkers may offer many other advantages for the production of chimeric proteins, such as improving biological activity, increasing expression yield, and achieving desirable pharmacokinetic profiles.

As the skilled person in the art understands, the chimeric protein of the invention may comprise a signal peptide bound to the amino-terminal end, so that it can be transported to the OM after its translation by the ribosomes. In a particular embodiment, alone or combination with all or each one of all the previous particular embodiments, the chimeric protein further comprises an N-terminal signal peptide for translocation through the bacterial membrane. In another particular embodiment, alone or combination with all or each one of all the previous particular embodiments, the signal peptide bound to the antigen binding domain.

As used herein, the term "signal peptide", also called "secretion signal", is a peptide sequence, usually present in the N-terminal end of secretory proteins (i.e., which are secreted or exported from the place they are produced) or membrane proteins, involved in the passage of the protein across the cell membrane (in bacteria). Practically any signal peptide may be used in the instant invention. Illustrative, non-limitative, examples of said signal peptide directing the passage of the chimeric protein of the invention include the signal peptide of PeIB, the signal peptide of OmpA, the signal peptide of protein 3 of bacteriophage M13, the signal peptide of the maltose binding protein (MBP), or any other signal peptide of those normally used with autotransporter systems. In a more particular embodiment of the chimeric protein of the invention, alone or in combination with all or each one of the previous particular embodiments, said signal peptide is the signal peptide of invasin or intimin proteins, or PelB. In a more particular embodiment, alone or in combination with all or each one of the previous particular embodiments, the signal peptide of invasin protein comprises, or consists of, the amino acid sequence SEQ ID NO: 7, or the signal peptide of intimin protein comprises, or consists of, the amino acid sequence SEQ ID NO: 8, or the signal peptide of PelB comprises, or consists of, the amino acid sequence SEQ ID NO: 9.
SEQ ID NO: 7
   MMVFQPISEFLLIRNAGMSMYFNKIISFNIISRIVICIFLICGMFMAGASE
SEQ ID NO: 8
   MITHGCYTRTRHKHKLKKTLIMLSAGLGLFFYVNQNSFANGEN
SEQ ID NO: 9
   MKYLLPTAAAGLLLLAAQ

In addition, the chimeric protein of the invention may encompass suitable tags, e.g., FLAG-tags, Myc-tags, His-tags, HA-tags or GST-tags which allow, e.g., for efficient isolation and/or purification of the tagged chimeric protein. The tag(s) can be attached to the chimeric protein via a linker, if appropriate.

The chimeric protein may be manufactured by chemical synthesis or recombinant molecular biology techniques well known for the skilled artisan. In case of using recombinant molecular biology techniques, the method of manufacturing the chimeric protein typically comprises (a) culturing a host cell comprising a polynucleotide encoding the chimeric protein of the invention and (b) obtaining from the said host cell the chimeric protein.

In another particular embodiment of the chimeric protein of the invention, the chimeric protein comprises, or consists of, the amino acid sequence SEQ ID NO: 10, SEQ ID NO: 11, SEQ ID NO: 12, SEQ ID NO: 13, SEQ ID NO: 14 or SEQ ID NO: 15.
SEQ ID NO: 10 (the optional signal peptide is shown underlined) 
MITHGCYTRTRHKHKLKKTLIMLSAGLGLFFYVNQNSFANGENEFGGGSVFTLEDFVGDWEQ
TAAYNLDQVLEQGGVSSLLQNLAVSVTPIQRIVRSGENALKIDIHVIIPYEGLSADQMAQIEEVF
KVVYPVDDHHFKVILPYGTLVIDGVTPNMLNYFGRPYEGIAVFDGKKITVTGTLWNGNKIIDER
LITPDGSMLFRVTINSGGGSPRKSNMTDDKALNYAAQQAASLGSQLQSRSLNGDYAKDTALG
IAGNQASSQLQAWLQHYGTAEVNLQSGNNFDGSSLDFLLPFYDSEKMLAFGQVGARYIDSRF
TANLGAGQRFFLPANMLGYNVFIDQDFSGDNTRLGIGGEYWRDYFKSSVNGYFRMSGWHE
SYNKKDYDERPANGFDIRFNGYLPSYPALGAKLIYEQYYGDNVALFNSDKLQSNPGAATVGV
NYTPIPLVTMGIDYRHGTGNENDLLYSMQFRYQFDKSWSQQIEPQYVNELRTLSGSRYDLVQ
RNNNIILEYKKQDILSLNIPHDINGTEHSTQKIQLIVKSKYGLDRIVWDDSALRSQGGQIQHSGS
QSAQDYQAILPAYVQGGSNIYKVTARAYDRNGNSSNNVQLTITVLSNGQVVDQVGVTDFTAD
KTSAKADNADTITYTATVKKNGVAQANVPVSFNIVSGTATLGANSAKTDANGKATVTLKSSTP
GQVVVSAKTAEMTSALNASAVIFFDVDGAPVPYPDPLEPAQPAMAQVQLQESGGGLVQAGG
SLLLSCAASGRTFSSYAMGWFRQAPGKEREFVAAINWSGGSTSYADSVKGRFTISRDNTKNT
VYLQMNSLKPEDTAAFYCAATYNPYSRDHYFPRMTTEYDYWGQGTQVTVSSEPKTPKPQPA
AAEQKLISEEDAAA
SEQ ID NO: 11 (the optional signal peptide is shown underlined) 
MITHGCYTRTRHKHKLKKTLIMLSAGLGLFFYVNQNSFANGENEFGGGSVTGYRLFEEILGGG
SPRKSNMTDDKALNYAAQQAASLGSQLQSRSLNGDYAKDTALGIAGNQASSQLQAWLQHY
GTAEVNLQSGNNFDGSSLDFLLPFYDSEKMLAFGQVGARYIDSRFTANLGAGQRFFLPANML
GYNVFIDQDFSGDNTRLGIGGEYWRDYFKSSVNGYFRMSGWHESYNKKDYDERPANGFDIR
FNGYLPSYPALGAKLIYEQYYGDNVALFNSDKLQSNPGAATVGVNYTPIPLVTMGIDYRHGTG
NENDLLYSMQFRYQFDKSWSQQIEPQYVNELRTLSGSRYDLVQRNNNIILEYKKQDILSLNIP
HDINGTEHSTQKIQLIVKSKYGLDRIVWDDSALRSQGGQIQHSGSQSAQDYQAILPAYVQGGS
 
NIYKVTARAYDRNGNSSNNVQLTITVLSNGQVVDQVGVTDFTADKTSAKADNADTITYTATVK
KNGVAQANVPVSFNIVSGTATLGANSAKTDANGKATVTLKSSTPGQVVVSAKTAEMTSALNA
SAVIFFDVDGAPVPYPDPLEPAQPAMAQVQLQESGGGLVQAGGSLLLSCAASGRTFSSYAM
GWFRQAPGKEREFVAAINWSGGSTSYADSVKGRFTISRDNTKNTVYLQMNSLKPEDTAAFY
CAATYNPYSRDHYFPRMTTEYDYWGQGTQVTVSSEPKTPKPQPAAAEQKLISEEDAAA
SEQ ID NO: 12 (the optional signal peptide is shown underlined) 
MMVFQPISEFLLIRNAGMSMYFNKIISFNIISRIVICIFLICGMFMAGASEGGGSVFTLEDFVGD
WEQTAAYNLDQVLEQGGVSSLLQNLAVSVTPIQRIVRSGENALKIDIHVIIPYEGLSADQMAQ
IEEVFKVVYPVDDHHFKVILPYGTLVIDGVTPNMLNYFGRPYEGIAVFDGKKITVTGTLWNGN
KIIDERLITPDGSMLFRVTINSGGGSKYDANAPQQVQPYSVSSSAFENLHPNNEMESSINPF
SASDTERNAAIIDRANKEQETEAVNKMISTGARLAASGRASDVAHSMVGDAVNQEIKQWLN
RFGTAQVNLNFDKNFSLKESSLDWLAPWYDSASFLFFSQLGIRNKDSRNTLNLGVGIRTLEN
GWLYGLNTFYDNDLTGHNHRIGLGAEAWTDYLQLAANGYFRLNGWHSSRDFSDYKERPAT
GGDLRANAYLPALPQLGGKLMYEQYTGERVALFGKDNLQRNPYAVTAGINYTPVPLLTVGV
DQRMGKSSKHETQWNLQMNYRFGESFQSQLSPSAVAGTRLLAESRYNLVDRNNNIVLEYQ
KQQVVKLTLSPATISGLPGQVYQVNAQVQGASAVREIVWSDAELIAAGGTLTPLSTTQFNLV
LPPYKRTAQVSRVTDDLTANFYSLSALAVDHQGNRSNSFTLSVTVQQPQVDGAPVPYPDPL
EPAQPAMAQVQLQESGGGLVQAGGSLLLSCAASGRTFSSYAMGWFRQAPGKEREFVAAI
NWSGGSTSYADSVKGRFTISRDNTKNTVYLQMNSLKPEDTAAFYCAATYNPYSRDHYFPR
MTTEYDYWGQGTQVTVSSEPKTPKPQPAAAEQKLISEEDAAA
SEQ ID NO: 13 (the optional signal peptide is shown underlined) 
MMVFQPISEFLLIRNAGMSMYFNKIISFNIISRIVICIFLICGMFMAGASEGGGSVTGYRLFEEILG
GGSKYDANAPQQVQPYSVSSSAFENLHPNNEMESSINPFSASDTERNAAIIDRANKEQETEA
VNKMISTGARLAASGRASDVAHSMVGDAVNQEIKQWLNRFGTAQVNLNFDKNFSLKESSLD
WLAPWYDSASFLFFSQLGIRNKDSRNTLNLGVGIRTLENGWLYGLNTFYDNDLTGHNHRIGL
GAEAWTDYLQLAANGYFRLNGWHSSRDFSDYKERPATGGDLRANAYLPALPQLGGKLMYE
QYTGERVALFGKDNLQRNPYAVTAGINYTPVPLLTVGVDQRMGKSSKHETQWNLQMNYRFG
ESFQSQLSPSAVAGTRLLAESRYNLVDRNNNIVLEYQKQQVVKLTLSPATISGLPGQVYQVNA
QVQGASAVREIVWSDAELIAAGGTLTPLSTTQFNLVLPPYKRTAQVSRVTDDLTANFYSLSAL
AVDHQGNRSNSFTLSVTVQQPQVDGAPVPYPDPLEPAQPAMAQVQLQESGGGLVQAGGSL
LLSCAASGRTFSSYAMGWFRQAPGKEREFVAAINWSGGSTSYADSVKGRFTISRDNTKNTV
YLQMNSLKPEDTAAFYCAATYNPYSRDHYFPRMTTEYDYWGQGTQVTVSSEPKTPKPQPAA
AEQKLISEEDAAA
SEQ ID NO: 14 (the optional signal peptide is shown underlined) 
MKYLLPTAAAGLLLLAAQPAMAQVQLQESGGGLVQAGGSLLLSCAASGRTFSSYAMGWFRQ
APGKEREFVAAINWSGGSTSYADSVKGRFTISRDNTKNTVYLQMNSLKPEDTAAFYCAATYN
PYSRDHYFPRMTTEYDYWGQGTQVTVSSEPKTPKPQPAAAAGAEFVDGAPVPYPDPLEPID
NSAAISMANPRPPTPRTPTPGPDLNVDNDLRPEAGSYIANLAAANTMFTTRLHERLGNTYYTD
MVTGEQKQTTMWMRHEGGHNKWRDGSGQLKTQSNRYVLQLGGDVAQWSQNGSDRWHV
GVMAGYGNSDSKTISSRTGYRAKASVNGYSTGLYATWYADDESRNGAYLDSWAQYSWFDN
TVKGDDLQSESYKSKGFTASLEAGYKHKLAEFNGSQGTRNEWYVQPQAQVTWMGVKADKH
RESNGTLVHSNGDGNVQTRLGVKTWLKSHHKMDDGKSREFQPFVEVNWLHNSKDFSTSMD
GVSVTQDGARNIAEIKTGVEGQLNANLNVWGNVGVQVADRGYNDTSAMVGIKWQFGQGEA
APVVAPAPAPAPVFTLEDFVGDWEQTAAYNLDQVLEQGGVSSLLQNLAVSVTPIQRIVRSGE
NALKIDIHVIIPYEGLSADQMAQIEEVFKVVYPVDDHHFKVILPYGTLVIDGVTPNMLNYFGRPY
EGIAVFDGKKITVTGTLWNGNKIIDERLITPDGSMLFRVTINS
SEQ ID NO: 15 (the optional signal peptide is shown underlined)

MKYLLPTAAAGLLLLAAQPAMAQVQLQESGGGLVQAGGSLLLSCAASGRTFSSYAMGWFR
QAPGKEREFVAAINWSGGSTSYADSVKGRFTISRDNTKNTVYLQMNSLKPEDTAAFYCAAT
YNPYSRDHYFPRMTTEYDYWGQGTQVTVSSEPKTPKPQPAAAAGAEFVDGAPVPYPDPL
EPIDNSAAISMANPRPPTPRTPTPGPDLNVDNDLRPEAGSYIANLAAANTMFTTRLHERLGN
TYYTDMVTGEQKQTTMWMRHEGGHNKWRDGSGQLKTQSNRYVLQLGGDVAQWSQNGS
DRWHVGVMAGYGNSDSKTISSRTGYRAKASVNGYSTGLYATWYADDESRNGAYLDSWAQ
YSWFDNTVKGDDLQSESYKSKGFTASLEAGYKHKLAEFNGSQGTRNEWYVQPQAQVTW
MGVKADKHRESNGTLVHSNGDGNVQTRLGVKTWLKSHHKMDDGKSREFQPFVEVNWLH
NSKDFSTSMDGVSVTQDGARNIAEIKTGVEGQLNANLNVWGNVGVQVADRGYNDTSAMV
GIKWQFGQGGGGSVTGYRLFEEIL

Thus, in another aspect, the present invention relates to a polynucleotide, hereinafter "polynucleotide of the invention", comprising a nucleotide sequence encoding the chimeric protein of the invention as defined above in previous paragraphs.

The term "polynucleotide" or "nucleic acid (molecule)" as used herein refers to single- or double-stranded DNA molecules as well as to RNA molecules. Encompassed by the said term is genomic DNA, cDNA, hnRNA, mRNA as well as all naturally occurring or artificially modified derivatives of such molecular species. The polynucleotide may be, in an aspect, a linear or circular molecule. The polynucleotide sequence codes for the chimeric protein of the invention. Moreover, in addition to the nucleic acid sequences encoding the chimeric protein of the invention, a polynucleotide as used herein may comprise additional sequences required for proper transcription and/or translation such as 5' or 3' UTR sequences. The nucleic acid sequences encoding the chimeric protein of the present invention can be derived from the amino acid sequences by a skilled artisan without undue burden. In light of the degeneracy of the genetic code, optimized codons may be used in the nucleic acid sequences encoding the chimeric protein of the invention. Thereby, optimal expression can be achieved. Thus, in a particular embodiment of the polynucleotide of the invention, the nucleotide sequence encoding the chimeric protein of the invention is 'codon optimised' for expression in *E. coli.*

In another particular embodiment of the polynucleotide of the invention, the polynucleotide comprises, or consists of, the nucleotide sequence SEQ ID NO: 16, SEQ ID NO: 17, SEQ ID NO: 18, SEQ ID NO: 19, SEQ ID NO: 20 or SEQ ID NO: 21.
SEQ ID NO: 16 (the optional signal peptide is shown underlined) 
ATGATTACTCATGGTTGTTATACCCGGACCCGGCACAAGCATAAGCTAAAAAAAACATTGA
TTATGCTTAGTGCTGGTTTAGGATTGTTTTTTTATGTTAATCAGAACTCATTTGCAAATGGT
GAAAATGAATTCGGTGGGGGAAGCGTCTTCACACTCGAAGATTTCGTTGGGGACTGGGA
ACAGACAGCCGCCTACAACCTGGACCAAGTCCTTGAACAGGGAGGTGTGTCCAGTTTGC
TGCAAAATCTCGCCGTGTCCGTAACTCCGATCCAAAGGATTGTCCGGAGCGGTGAAAAT
GCCCTGAAGATCGACATCCATGTCATCATCCCGTATGAAGGTCTGAGCGCCGACCAAAT
GGCCCAGATCGAAGAGGTGTTTAAGGTGGTGTACCCTGTGGATGATCATCACTTTAAGGT
GATCCTGCCCTATGGCACACTGGTAATCGACGGGGTTACGCCGAACATGCTGAACTATTT
CGGACGGCCGTATGAAGGCATCGCCGTGTTCGACGGCAAAAAGATCACTGTAACAGGGA
CCCTGTGGAACGGCAACAAAATTATCGACGAGCGCCTGATCACCCCCGACGGCTCCATG
CTGTTCCGAGTAACCATCAACAGCGGCGGAGGGTCTCCGCGGAAAAGCAACATGACCGA
TGACAAGGCATTAAATTATGCGGCACAACAGGCGGCGAGTCTCGGTAGCCAGCTTCAGT
CGCGATCTCTGAACGGCGATTACGCGAAAGATACCGCTCTTGGTATCGCTGGTAACCAG
GCTTCGTCACAGTTGCAGGCCTGGTTACAACATTATGGAACGGCAGAGGTTAATCTGCAA
AGTGGTAATAACTTTGACGGTAGTTCACTGGACTTCTTATTACCGTTCTATGATTCCGAAA
AAATGCTGGCATTTGGTCAGGTCGGAGCGCGTTACATTGACTCCCGCTTTACGGCAAATT
TAGGTGCGGGTCAGCGTTTTTTCCTTCCTGCAAACATGTTGGGCTATAACGTCTTCATTGA
TCAGGATTTTTCTGGTGATAATACCCGTTTAGGTATTGGTGGCGAATACTGGCGAGACTA
TTTCAAAAGTAGCGTTAACGGCTATTTCCGCATGAGCGGCTGGCATGAGTCATACAATAA
GAAAGACTATGATGAGCGCCCAGCAAATGGCTTCGATATCCGTTTTAATGGCTATCTACC
GTCATATCCGGCATTAGGCGCCAAGCTGATATATGAGCAGTATTATGGTGATAATGTTGC
 
TTTGTTTAATTCTGATAAGCTGCAATCGAATCCTGGTGCGGCGACCGTTGGTGTAAACTAT
ACTCCGATTCCTCTGGTGACGATGGGGATTGATTACCGTCATGGTACGGGTAATGAAAAT
GATCTCCTTTACTCAATGCAGTTCCGTTATCAGTTTGATAAATCGTGGAGCCAGCAAATTG
AACCACAGTATGTTAACGAGTTAAGAACATTATCAGGCAGCCGTTACGATCTGGTTCAGC
GTAATAACAATATTATTCTGGAGTACAAGAAGCAGGATATTCTTTCTCTGAATATTCCGCAT
GATATTAATGGTACTGAACACAGTACGCAGAAGATTCAGTTGATCGTTAAGAGCAAATAC
GGTCTGGATCGTATCGTCTGGGATGATAGTGCATTACGCAGTCAGGGCGGTCAGATTCA
GCATAGCGGAAGCCAAAGCGCACAAGACTACCAGGCTATTTTGCCTGCTTATGTGCAAG
GTGGCAGCAATATTTATAAAGTGACGGCTCGCGCCTATGACCGTAATGGCAATAGCTCTA
ACAATGTACAGCTTACTATTACCGTTCTGTCGAATGGTCAAGTTGTTGACCAGGTTGGGG
TAACGGACTTTACGGCGGATAAGACTTCGGCTAAAGCGGATAACGCCGATACCATTACTT
ATACCGCGACGGTGAAAAAGAATGGGGTAGCTCAGGCTAATGTCCCTGTTTCATTTAATA
TTGTTTCAGGAACTGCAACTCTTGGGGCAAATAGTGCCAAAACGGATGCTAACGGTAAGG
CAACCGTAACGTTGAAGTCGAGTACGCCAGGACAGGTCGTCGTGTCTGCTAAAACCGCC
GAGATGACTTCAGCACTTAATGCCAGTGCGGTTATATTTTTTGATGTCGACGGTGCGCCG
GTGCCGTATCCGGACCCGCTGGAACCGGCCCAGCCGGCCATGGCCCAGGTGCAGCTGC
AGGAGTCTGGGGGAGGATTGGTGCAGGCTGGGGGTTCTCTGTTGCTCTCCTGTGCAGC
CTCTGGACGCACCTTCAGTAGCTATGCCATGGGCTGGTTCCGCCAGGCTCCAGGGAAGG
AGCGTGAGTTTGTAGCAGCTATTAACTGGAGTGGTGGTAGCACAAGCTATGCAGACTCC
GTGAAGGGCCGATTCACAATCTCCAGAGACAACACCAAGAACACGGTGTATCTGCAAATG
AACAGCCTGAAACCTGAGGACACGGCCGCTTTTTACTGTGCAGCTACGTACAATCCATAC
TCGCGTGATCATTACTTCCCGCGCATGACCACTGAGTATGACTACTGGGGCCAGGGGAC
CCAGGTCACCGTCTCCTCAGAACCCAAGACACCAAAACCACAACCAGCGGCCGCCGAAC
AAAAACTCATCTCAGAAGAGGATGCAGCTGCATAA
SEQ ID NO: 17 (the optional signal peptide is shown underlined) 
ATGATTACTCATGGTTGTTATACCCGGACCCGGCACAAGCATAAGCTAAAAAAAACATTGA
TTATGCTTAGTGCTGGTTTAGGATTGTTTTTTTATGTTAATCAGAACTCATTTGCAAATGGT
GAAAATGAATTCGGTGGGGGAAGCGTGACCGGCTACCGGCTGTTCGAGGAGATTCTGG
GCGGAGGGTCTCCGCGGAAAAGCAACATGACCGATGACAAGGCATTAAATTATGCGGCA
CAACAGGCGGCGAGTCTCGGTAGCCAGCTTCAGTCGCGATCTCTGAACGGCGATTACGC
GAAAGATACCGCTCTTGGTATCGCTGGTAACCAGGCTTCGTCACAGTTGCAGGCCTGGTT
ACAACATTATGGAACGGCAGAGGTTAATCTGCAAAGTGGTAATAACTTTGACGGTAGTTC
ACTGGACTTCTTATTACCGTTCTATGATTCCGAAAAAATGCTGGCATTTGGTCAGGTCGGA
GCGCGTTACATTGACTCCCGCTTTACGGCAAATTTAGGTGCGGGTCAGCGTTTTTTCCTT
CCTGCAAACATGTTGGGCTATAACGTCTTCATTGATCAGGATTTTTCTGGTGATAATACCC
GTTTAGGTATTGGTGGCGAATACTGGCGAGACTATTTCAAAAGTAGCGTTAACGGCTATT
TCCGCATGAGCGGCTGGCATGAGTCATACAATAAGAAAGACTATGATGAGCGCCCAGCA
 
AATGGCTTCGATATCCGTTTTAATGGCTATCTACCGTCATATCCGGCATTAGGCGCCAAG
CTGATATATGAGCAGTATTATGGTGATAATGTTGCTTTGTTTAATTCTGATAAGCTGCAATC
GAATCCTGGTGCGGCGACCGTTGGTGTAAACTATACTCCGATTCCTCTGGTGACGATGG
GGATTGATTACCGTCATGGTACGGGTAATGAAAATGATCTCCTTTACTCAATGCAGTTCCG
TTATCAGTTTGATAAATCGTGGAGCCAGCAAATTGAACCACAGTATGTTAACGAGTTAAGA
ACATTATCAGGCAGCCGTTACGATCTGGTTCAGCGTAATAACAATATTATTCTGGAGTACA
AGAAGCAGGATATTCTTTCTCTGAATATTCCGCATGATATTAATGGTACTGAACACAGTAC
GCAGAAGATTCAGTTGATCGTTAAGAGCAAATACGGTCTGGATCGTATCGTCTGGGATGA
TAGTGCATTACGCAGTCAGGGCGGTCAGATTCAGCATAGCGGAAGCCAAAGCGCACAAG
ACTACCAGGCTATTTTGCCTGCTTATGTGCAAGGTGGCAGCAATATTTATAAAGTGACGG
CTCGCGCCTATGACCGTAATGGCAATAGCTCTAACAATGTACAGCTTACTATTACCGTTCT
GTCGAATGGTCAAGTTGTTGACCAGGTTGGGGTAACGGACTTTACGGCGGATAAGACTT
CGGCTAAAGCGGATAACGCCGATACCATTACTTATACCGCGACGGTGAAAAAGAATGGG
GTAGCTCAGGCTAATGTCCCTGTTTCATTTAATATTGTTTCAGGAACTGCAACTCTTGGGG
CAAATAGTGCCAAAACGGATGCTAACGGTAAGGCAACCGTAACGTTGAAGTCGAGTACG
CCAGGACAGGTCGTCGTGTCTGCTAAAACCGCCGAGATGACTTCAGCACTTAATGCCAG
TGCGGTTATATTTTTTGATGTCGACGGTGCGCCGGTGCCGTATCCGGACCCGCTGGAAC
CGGCCCAGCCGGCCATGGCCCAGGTGCAGCTGCAGGAGTCTGGGGGAGGATTGGTGC
AGGCTGGGGGTTCTCTGTTGCTCTCCTGTGCAGCCTCTGGACGCACCTTCAGTAGCTAT
GCCATGGGCTGGTTCCGCCAGGCTCCAGGGAAGGAGCGTGAGTTTGTAGCAGCTATTAA
CTGGAGTGGTGGTAGCACAAGCTATGCAGACTCCGTGAAGGGCCGATTCACAATCTCCA
GAGACAACACCAAGAACACGGTGTATCTGCAAATGAACAGCCTGAAACCTGAGGACACG
GCCGCTTTTTACTGTGCAGCTACGTACAATCCATACTCGCGTGATCATTACTTCCCGCGC
ATGACCACTGAGTATGACTACTGGGGCCAGGGGACCCAGGTCACCGTCTCCTCAGAACC
CAAGACACCAAAACCACAACCAGCGGCCGCCGAACAAAAACTCATCTCAGAAGAGGATG
CAGCTGCATAA
SEQ ID NO: 18 (the optional signal peptide is shown underlined) 
ATGATGGTTTTCCAGCCAATCAGTGAGTTTCTCTTGATAAGGAATGCGGGAATGTCTATGT
ATTTTAATAAAATAATTTCATTTAATATTATTTCACGAATAGTTATTTGTATCTTTTTGATATG
TGGAATGTTCATGGCTGGGGCTTCAGAAGGTGGGGGAAGCGTCTTCACACTCGAAGATT
TCGTTGGGGACTGGGAACAGACAGCCGCCTACAACCTGGACCAAGTCCTTGAACAGGGA
GGTGTGTCCAGTTTGCTGCAAAATCTCGCCGTGTCCGTAACTCCGATCCAAAGGATTGTC
CGGAGCGGTGAAAATGCCCTGAAGATCGACATCCATGTCATCATCCCGTATGAAGGTCT
GAGCGCCGACCAAATGGCCCAGATCGAAGAGGTGTTTAAGGTGGTGTACCCTGTGGATG
ATCATCACTTTAAGGTGATCCTGCCCTATGGCACACTGGTAATCGACGGGGTTACGCCGA
ACATGCTGAACTATTTCGGACGGCCGTATGAAGGCATCGCCGTGTTCGACGGCAAAAAG
ATCACTGTAACAGGGACCCTGTGGAACGGCAACAAAATTATCGACGAGCGCCTGATCAC
 
CCCCGACGGCTCCATGCTGTTCCGAGTAACCATCAACAGCGGCGGAGGGTCTAAATATG
ATGCTAACGCACCGCAACAGGTCCAGCCTTATTCTGTCTCTTCATCTGCATTTGAAAATCT
CCATCCTAATAATGAAATGGAGAGTTCAATCAATCCCTTTTCCGCATCGGATACAGAAAGA
AATGCTGCAATAATAGATCGCGCCAATAAGGAGCAGGAGACTGAAGCGGTGAATAAGAT
GATAAGCACCGGGGCCAGGTTAGCTGCATCAGGCAGGGCATCTGATGTTGCTCACTCAA
TGGTGGGCGATGCGGTTAATCAAGAAATCAAACAGTGGTTAAATCGATTCGGTACGGCTC
AAGTTAATCTGAATTTTGACAAAAATTTTTCGCTAAAAGAAAGCTCTCTTGATTGGCTGGCT
CCTTGGTATGACTCTGCTTCATTCCTCTTTTTTAGTCAGTTAGGTATTCGCAATAAAGACA
GCCGCAACACACTTAACCTTGGCGTCGGGATACGTACATTGGAGAACGGTTGGCTGTAC
GGACTTAATACTTTTTATGATAATGATTTGACCGGCCACAACCACCGTATCGGTCTTGGTG
CCGAGGCCTGGACCGATTATTTACAGTTGGCTGCCAATGGGTATTTTCGCCTCAATGGAT
GGCACTCGTCGCGTGATTTCTCCGACTATAAAGAGCGCCCAGCCACTGGGGGGGATTTG
CGCGCGAATGCTTATTTACCTGCACTCCCACAACTGGGGGGGAAGTTGATGTATGAGCA
ATACACCGGTGAGCGTGTTGCTTTATTTGGTAAAGATAATCTGCAACGCAACCCTTATGC
CGTGACTGCCGGGATCAATTACACCCCCGTGCCTCTACTCACTGTCGGGGTAGATCAGC
GTATGGGGAAAAGCAGTAAGCATGAAACACAGTGGAACCTCCAAATGAACTATCGCTTTG
GCGAGAGTTTTCAGTCGCAACTTAGCCCTTCAGCGGTGGCAGGAACACGTCTACTGGCG
GAGAGCCGCTATAACCTTGTCGATCGTAACAATAATATCGTGTTGGAGTATCAGAAACAG
CAGGTGGTTAAACTGACATTATCGCCAGCAACTATCTCCGGCCTGCCGGGTCAGGTTTAT
CAGGTGAACGCACAAGTACAAGGGGCATCTGCTGTAAGGGAAATTGTCTGGAGTGATGC
CGAACTGATTGCCGCTGGCGGCACATTAACACCACTGAGTACCACACAATTCAACTTGGT
TTTACCGCCTTATAAACGCACAGCACAAGTGAGTCGGGTAACGGACGACCTGACAGCCA
ACTTTTATTCGCTTAGTGCGCTCGCGGTTGATCACCAAGGAAACCGATCTAACTCATTCAC
ATTGAGCGTCACCGTTCAGCAGCCTCAGGTCGACGGTGCGCCGGTGCCGTATCCGGAC
CCGCTGGAACCGGCCCAGCCGGCCATGGCCCAGGTGCAGCTGCAGGAGTCTGGGGGA
GGATTGGTGCAGGCTGGGGGTTCTCTGTTGCTCTCCTGTGCAGCCTCTGGACGCACCTT
CAGTAGCTATGCCATGGGCTGGTTCCGCCAGGCTCCAGGGAAGGAGCGTGAGTTTGTAG
CAGCTATTAACTGGAGTGGTGGTAGCACAAGCTATGCAGACTCCGTGAAGGGCCGATTC
ACAATCTCCAGAGACAACACCAAGAACACGGTGTATCTGCAAATGAACAGCCTGAAACCT
GAGGACACGGCCGCTTTTTACTGTGCAGCTACGTACAATCCATACTCGCGTGATCATTAC
TTCCCGCGCATGACCACTGAGTATGACTACTGGGGCCAGGGGACCCAGGTCACCGTCTC
CTCAGAACCCAAGACACCAAAACCACAACCAGCGGCCGCCGAACAAAAACTCATCTCAG
AAGAGGATGCAGCTGCATAA
SEQ ID NO: 19 (the optional signal peptide is shown underlined) 
ATGATGGTTTTCCAGCCAATCAGTGAGTTTCTCTTGATAAGGAATGCGGGAATGTCTATGT
ATTTTAATAAAATAATTTCATTTAATATTATTTCACGAATAGTTATTTGTATCTTTTTGATATG
TGGAATGTTCATGGCTGGGGCTTCAGAAGGTGGGGGAAGCGTGACCGGCTACCGGCTG
 
TTCGAGGAGATTCTGGGCGGAGGGTCTAAATATGATGCTAACGCACCGCAACAGGTCCA
GCCTTATTCTGTCTCTTCATCTGCATTTGAAAATCTCCATCCTAATAATGAAATGGAGAGTT
CAATCAATCCCTTTTCCGCATCGGATACAGAAAGAAATGCTGCAATAATAGATCGCGCCA
ATAAGGAGCAGGAGACTGAAGCGGTGAATAAGATGATAAGCACCGGGGCCAGGTTAGCT
GCATCAGGCAGGGCATCTGATGTTGCTCACTCAATGGTGGGCGATGCGGTTAATCAAGA
AATCAAACAGTGGTTAAATCGATTCGGTACGGCTCAAGTTAATCTGAATTTTGACAAAAAT
TTTTCGCTAAAAGAAAGCTCTCTTGATTGGCTGGCTCCTTGGTATGACTCTGCTTCATTCC
TCTTTTTTAGTCAGTTAGGTATTCGCAATAAAGACAGCCGCAACACACTTAACCTTGGCGT
CGGGATACGTACATTGGAGAACGGTTGGCTGTACGGACTTAATACTTTTTATGATAATGAT
TTGACCGGCCACAACCACCGTATCGGTCTTGGTGCCGAGGCCTGGACCGATTATTTACA
GTTGGCTGCCAATGGGTATTTTCGCCTCAATGGATGGCACTCGTCGCGTGATTTCTCCGA
CTATAAAGAGCGCCCAGCCACTGGGGGGGATTTGCGCGCGAATGCTTATTTACCTGCAC
TCCCACAACTGGGGGGGAAGTTGATGTATGAGCAATACACCGGTGAGCGTGTTGCTTTAT
TTGGTAAAGATAATCTGCAACGCAACCCTTATGCCGTGACTGCCGGGATCAATTACACCC
CCGTGCCTCTACTCACTGTCGGGGTAGATCAGCGTATGGGGAAAAGCAGTAAGCATGAA
ACACAGTGGAACCTCCAAATGAACTATCGCTTTGGCGAGAGTTTTCAGTCGCAACTTAGC
CCTTCAGCGGTGGCAGGAACACGTCTACTGGCGGAGAGCCGCTATAACCTTGTCGATCG
TAACAATAATATCGTGTTGGAGTATCAGAAACAGCAGGTGGTTAAACTGACATTATCGCCA
GCAACTATCTCCGGCCTGCCGGGTCAGGTTTATCAGGTGAACGCACAAGTACAAGGGGC
ATCTGCTGTAAGGGAAATTGTCTGGAGTGATGCCGAACTGATTGCCGCTGGCGGCACAT
TAACACCACTGAGTACCACACAATTCAACTTGGTTTTACCGCCTTATAAACGCACAGCACA
AGTGAGTCGGGTAACGGACGACCTGACAGCCAACTTTTATTCGCTTAGTGCGCTCGCGG
TTGATCACCAAGGAAACCGATCTAACTCATTCACATTGAGCGTCACCGTTCAGCAGCCTC
AGGTCGACGGTGCGCCGGTGCCGTATCCGGACCCGCTGGAACCGGCCCAGCCGGCCAT
GGCCCAGGTGCAGCTGCAGGAGTCTGGGGGAGGATTGGTGCAGGCTGGGGGTTCTCTG
TTGCTCTCCTGTGCAGCCTCTGGACGCACCTTCAGTAGCTATGCCATGGGCTGGTTCCG
CCAGGCTCCAGGGAAGGAGCGTGAGTTTGTAGCAGCTATTAACTGGAGTGGTGGTAGCA
CAAGCTATGCAGACTCCGTGAAGGGCCGATTCACAATCTCCAGAGACAACACCAAGAAC
ACGGTGTATCTGCAAATGAACAGCCTGAAACCTGAGGACACGGCCGCTTTTTACTGTGCA
GCTACGTACAATCCATACTCGCGTGATCATTACTTCCCGCGCATGACCACTGAGTATGAC
TACTGGGGCCAGGGGACCCAGGTCACCGTCTCCTCAGAACCCAAGACACCAAAACCACA
ACCAGCGGCCGCCGAACAAAAACTCATCTCAGAAGAGGATGCAGCTGCATAA
SEQ ID NO: 20 (the optional signal peptide is shown underlined) 
ATGAAATACCTATTGCCTACGGCAGCCGCTGGATTGTTATTACTCGCGGCCCAGCCGGC
CATGGCCCAGGTGCAGCTGCAGGAGTCTGGGGGAGGATTGGTGCAGGCTGGGGGTTCT
CTGTTGCTCTCCTGTGCAGCCTCTGGACGCACCTTCAGTAGCTATGCCATGGGCTGGTTC
CGCCAGGCTCCAGGGAAGGAGCGTGAGTTTGTAGCAGCTATTAACTGGAGTGGTGGTAG
 
CACAAGCTATGCAGACTCCGTGAAGGGCCGATTCACAATCTCCAGAGACAACACCAAGA
ACACGGTGTATCTGCAAATGAACAGCCTGAAACCTGAGGACACGGCCGCTTTTTACTGTG
CAGCTACGTACAATCCATACTCGCGTGATCATTACTTCCCGCGCATGACCACTGAGTATG
ACTACTGGGGCCAGGGGACCCAGGTCACCGTCTCCTCAGAACCCAAGACACCAAAACCA
CAACCAGCGGCCGCTGCGGGGGCCGAATTCGTCGACGGTGCGCCGGTGCCGTATCCG
GATCCGCTGGAACCGATCGACAATTCAGCCGCAATTAGTATGGCAAATCCACGTCCACCA
ACACCGCGGACACCTACGCCGGGTCCGGATCTGAATGTGGATAATGACCTGCGACCGGA
GGCGGGTAGCTACATTGCGAACCTTGCAGCAGCGAATACCATGTTCACCACGCGTCTGC
ATGAGCGTCTGGGTAATACGTACTATACCGACATGGTGACGGGTGAGCAGAAACAAACC
ACTATGTGGATGCGCCATGAAGGTGGTCATAATAAATGGCGTGATGGCAGCGGCCAGCT
GAAAACCCAAAGCAATCGCTATGTTCTGCAACTGGGAGGCGATGTCGCGCAGTGGAGCC
AAAACGGCAGCGACCGCTGGCATGTTGGGGTCATGGCGGGATATGGCAACAGCGACAG
CAAAACCATTTCCTCGCGAACCGGTTATCGTGCAAAAGCGAGTGTGAACGGATATAGCAC
AGGCCTCTATGCCACCTGGTATGCCGATGACGAGTCGCGTAATGGCGCGTATCTCGACA
GTTGGGCGCAGTACAGCTGGTTTGATAACACAGTGAAAGGGGATGACTTACAAAGTGAAT
CCTATAAATCAAAAGGATTTACCGCTTCACTGGAAGCTGGATACAAACACAAATTAGCTGA
ATTTAATGGCAGCCAGGGAACGCGTAATGAATGGTATGTTCAGCCGCAAGCACAGGTTAC
CTGGATGGGAGTCAAAGCCGATAAGCACCGCGAAAGCAACGGAACCCTCGTTCATAGCA
ACGGTGATGGCAATGTTCAAACCCGACTTGGCGTAAAAACCTGGCTGAAGAGCCACCAT
AAAATGGATGACGGTAAATCCCGCGAGTTCCAGCCGTTTGTAGAAGTGAACTGGCTACAT
AACAGTAAGGATTTCAGCACCAGTATGGATGGCGTGTCTGTCACTCAGGATGGAGCCCG
AAATATTGCTGAGATAAAAACCGGGGTGGAAGGACAGCTAAATGCCAACCTGAATGTCTG
GGGGAATGTGGGCGTTCAGGTTGCCGATAGGGGATATAATGACACCTCTGCAATGGTTG
GCATTAAGTGGCAATTCGGTCAAGGAGAAGCAGCACCTGTGGTTGCGCCTGCACCGGCT
CCTGCACCAGTCTTCACACTCGAAGATTTCGTTGGGGACTGGGAACAGACAGCCGCCTA
CAACCTGGACCAAGTCCTTGAACAGGGAGGTGTGTCCAGTTTGCTGCAAAATCTCGCCG
TGTCCGTAACTCCGATCCAAAGGATTGTCCGGAGCGGTGAAAATGCCCTGAAGATCGAC
ATCCATGTCATCATCCCGTATGAAGGTCTGAGCGCCGACCAAATGGCCCAGATCGAAGA
GGTGTTTAAGGTGGTGTACCCTGTGGATGATCATCACTTTAAGGTGATCCTGCCCTATGG
CACACTGGTAATCGACGGGGTTACGCCGAACATGCTGAACTATTTCGGACGGCCGTATG
AAGGCATCGCCGTGTTCGACGGCAAAAAGATCACTGTAACAGGGACCCTGTGGAACGGC
AACAAAATTATCGACGAGCGCCTGATCACCCCCGACGGCTCCATGCTGTTCCGAGTAAC
CATCAACAGCTAA
SEQ ID NO: 21 (the optional signal peptide is shown underlined)

ATGAAATACCTATTGCCTACGGCAGCCGCTGGATTGTTATTACTCGCGGCCCAGCCGG
CCATGGCCCAGGTGCAGCTGCAGGAGTCTGGGGGAGGATTGGTGCAGGCTGGGGGTT
CTCTGTTGCTCTCCTGTGCAGCCTCTGGACGCACCTTCAGTAGCTATGCCATGGGCTG

GTTCCGCCAGGCTCCAGGGAAGGAGCGTGAGTTTGTAGCAGCTATTAACTGGAGTGGT
GGTAGCACAAGCTATGCAGACTCCGTGAAGGGCCGATTCACAATCTCCAGAGACAACA
CCAAGAACACGGTGTATCTGCAAATGAACAGCCTGAAACCTGAGGACACGGCCGCTTT
TTACTGTGCAGCTACGTACAATCCATACTCGCGTGATCATTACTTCCCGCGCATGACCA
CTGAGTATGACTACTGGGGCCAGGGGACCCAGGTCACCGTCTCCTCAGAACCCAAGAC
ACCAAAACCACAACCAGCGGCCGCTGCGGGGGCCGAATTCGTCGACGGTGCGCCGGT
GCCGTATCCGGATCCGCTGGAACCGATCGACAATTCAGCCGCAATTAGTATGGCAAAT
CCACGTCCACCAACACCGCGGACACCTACGCCGGGTCCGGATCTGAATGTGGATAATG
ACCTGCGACCGGAGGCGGGTAGCTACATTGCGAACCTTGCAGCAGCGAATACCATGTT
CACCACGCGTCTGCATGAGCGTCTGGGTAATACGTACTATACCGACATGGTGACGGGT
GAGCAGAAACAAACCACTATGTGGATGCGCCATGAAGGTGGTCATAATAAATGGCGTG
ATGGCAGCGGCCAGCTGAAAACCCAAAGCAATCGCTATGTTCTGCAACTGGGAGGCGA
TGTCGCGCAGTGGAGCCAAAACGGCAGCGACCGCTGGCATGTTGGGGTCATGGCGGG
ATATGGCAACAGCGACAGCAAAACCATTTCCTCGCGAACCGGTTATCGTGCAAAAGCGA
GTGTGAACGGATATAGCACAGGCCTCTATGCCACCTGGTATGCCGATGACGAGTCGCG
TAATGGCGCGTATCTCGACAGTTGGGCGCAGTACAGCTGGTTTGATAACACAGTGAAA
GGGGATGACTTACAAAGTGAATCCTATAAATCAAAAGGATTTACCGCTTCACTGGAAGC
TGGATACAAACACAAATTAGCTGAATTTAATGGCAGCCAGGGAACGCGTAATGAATGGT
ATGTTCAGCCGCAAGCACAGGTTACCTGGATGGGAGTCAAAGCCGATAAGCACCGCGA
AAGCAACGGAACCCTCGTTCATAGCAACGGTGATGGCAATGTTCAAACCCGACTTGGC
GTAAAAACCTGGCTGAAGAGCCACCATAAAATGGATGACGGTAAATCCCGCGAGTTCCA
GCCGTTTGTAGAAGTGAACTGGCTACATAACAGTAAGGATTTCAGCACCAGTATGGATG
GCGTGTCTGTCACTCAGGATGGAGCCCGAAATATTGCTGAGATAAAAACCGGGGTGGA
AGGACAGCTAAATGCCAACCTGAATGTCTGGGGGAATGTGGGCGTTCAGGTTGCCGAT
AGGGGATATAATGACACCTCTGCAATGGTTGGCATTAAGTGGCAATTCGGACAGGGCG
GTGGGGGAAGCGTGACCGGCTACCGGCTGTTCGAGGAGATTCTGTAA

As the skilled person understands, the polynucleotide of the invention may be introduced into a vector. Thus, in another aspect, the present invention relates to a vector, hereinafter "vector of the invention", comprising the polynucleotide of the invention.

The term "vector", preferably, encompasses phage, plasmid, viral or retroviral vectors as well as artificial chromosomes, such as bacterial artificial chromosomes. Moreover, the term also relates to targeting constructs which allow for random or site-directed integration of the targeting construct into genomic DNA. Such target constructs, preferably, comprise DNA of sufficient length for either homologous or heterologous recombination. The vector encompassing the polynucleotide of the invention, may further comprise selectable markers for propagation and/or selection in a host or host cell.

The vector may be incorporated into a host cell by various techniques well known in the art. For example, a plasmid vector can be introduced in a precipitate such as a calcium phosphate precipitate or rubidium chloride precipitate, or in a complex with a charged lipid or in carbon-based clusters. Alternatively, a plasmid vector may be introduced by heat shock or electroporation techniques. Should the vector be a virus, it may be packaged in vitro using an appropriate packaging cell line prior to application to host cells. Retroviral vectors may be replication competent or replication defective. In the latter case, viral propagation generally will occur only in complementing host/cells. Moreover, the polynucleotide may be operatively linked to expression control sequences allowing expression in prokaryotic or eukaryotic host cells or isolated fractions thereof in the said vector. Expression of the polynucleotide comprises transcription of the polynucleotide into a translatable mRNA. Regulatory elements ensuring expression in host cells are well known in the art. They comprise regulatory sequences ensuring initiation of transcription and/or poly-A signals ensuring termination of transcription and stabilization of the transcript. Additional regulatory elements may include transcriptional as well as translational enhancers. Possible regulatory elements permitting expression in prokaryotic host cells comprise, e.g., the lac-, trp- or tac- promoter in *E. coli.*

In addition, inducible expression control sequences may be used in an expression vector encompassed by the present invention. Inducible expression systems and suitable expression control sequences are well known in the art. For example, the tetracycline - responsive regulatory system for transcriptional transactivation, among others. Such inducible vectors may comprise tet or lac operator sequences or sequences inducible by heat shock or other environmental factors are described in the art. For example, without being limited to, two commonly used inducible expression systems are Tet-Off and Tet-On; They consist of a fusion of the Tet repressor and a VP 16 activation domain to create a transcriptional activator protein (transactivator) rather than a repressor. Gene expression is activated as a result of binding of the Tet-Off or Tet-On protein to tetracycline response elements (TREs) located within an inducible promoter. Beside elements which are responsible for the initiation of transcription such regulatory elements may also comprise transcription termination signals, such as the SV40-poly-A site or the tk-poly-A site, downstream of the polynucleotide.

In this context, suitable expression vectors are known in the art such as Okayama-Berg cDNA expression vector pcDVI (Pharmacia), pBluescript (Stratagene), pCDM8, pRc/CMV, pcDNAI, pcDNA3, pcDNA3.1 (Invitrogen) or pSPORTI (Invitrogen) or baculovirus-derived vectors.

The vector of the invention can be used to transform, transfect or infect cells susceptible of being transformed, transfected or infected by said vector. Thus, in another aspect, the present invention relates to a host cell, hereinafter "host cell of the invention", comprising
- at least one chimeric protein of the invention, wherein domain (i) is displayed on the bacterial surface, domain (ii) is inserted in the outer membrane, and domain (iii) is display in the periplasm; and/or
- at least one polynucleotide of the invention; and/or
- at least one vector of the invention.

Any host cell may be used in the context in the present invention. Nevertheless, in a particular embodiment of the host cell of the invention, the host cell is a bacterium, preferably, a Gram negative bacteria. Examples of Gram negative bacteria include, without being limited to, *Escherichia* sp., *Acinetobacter* sp., *Klebsiella* sp., *Pseudomonas* sp., *Providencia* sp., *Serratia* sp., *Salmonella* sp., *Yersinia* sp., *Shigella* sp., and *Citrobactersp..* In a more particular embodiment, the host cell is *Escherichia coli, Acinetobacter baumannii, Klebsiella pneumoniae, Pseudomonas putida, Pseudomonas aeruginosa, Pseudomonas luteola, Pseudomaonas cloacae, Pseudomonas fluorescens, Pseudomonas oryzihabitants, Providencia rettgeri, Serratia liquefaciens, Citrobacter freundii, Lebsiella oxytoca, Shigella flexneri, Chrysibacterium meningosepticum, Salmonella enteritidis, Salmonella infantis,* and *Yersinia enterocolitica.*

In an even more particular embodiment of the host cell of the invention, the host cell is *E. coli.*

In another particular embodiment of the host cell of the invention, alone or in combination with all or each one of the previous embodiments, the host cell comprises at least two chimeric proteins of the invention, wherein domain (iii) of each chimeric protein (i.e.: the reporter domain) is complementary to each other. In a more particular embodiment of the host cell, domain (iii) of the one chimeric protein is a fragment of a luciferase and domain (iii) of the other chimeric protein is a second luciferase fragment complementary to the one in the previous chimeric protein.

In the context of the present invention, it is understood that "the at least two chimeric proteins of the invention are complementary each other" when the signal produced by the reporter system is only generated when both proteins interact closely with each other, allowing the interaction between the reporter fragments and thus the restoration of their signaling activity, which cannot be produced by any of the reporter fragments in isolation.

In a more particular embodiment of the host cell of the invention, alone or in combination with all or each one of the previous particular embodiments, the reporter domain (iii) of the one chimeric protein comprises, or consists of, an amino acid sequence having an identity of, at least, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99% with the sequence SEQ ID NO: 5, and the reporter domain (iii) of the other chimeric protein comprises, or consist of, an amino acid sequence having an identity of, at least, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99% with the sequence SEQ ID NO: 6.

In another particular embodiment of the host cell of the invention, the chimeric protein comprises, or consists of, the amino acid sequence SEQ ID NO: 10, SEQ ID NO: 11, SEQ ID NO: 12, SEQ ID NO: 13, SEQ ID NO: 14 or SEQ ID NO: 15.

In another particular embodiment of the host cell of the invention, the host cell comprises, or consists of, at least two chimeric proteins of the invention, wherein
- One chimeric protein comprises, or consists of, the amino acid sequence SEQ ID NO: 10 and the other chimeric protein comprises, or consists of, the amino acid sequence SEQ ID NO: 11; or
- One chimeric protein comprises, or consists of, the amino acid sequence SEQ ID NO: 12 and the other chimeric protein comprises, or consists of, the amino acid sequence SEQ ID NO: 13; or
- One chimeric protein comprises, or consists of, the amino acid sequence SEQ ID NO: 14 and the other chimeric protein comprises, or consists of, the amino acid sequence SEQ ID NO: 15.
- One chimeric protein comprises, or consists of, the amino acid sequence SEQ ID NO: 12 and the other chimeric protein comprises, or consists of, the amino acid sequence SEQ ID NO: 11.
- One chimeric protein comprises, or consists of, the amino acid sequence SEQ ID NO: 10 and the other chimeric protein comprises, or consists of, the amino acid sequence SEQ ID NO: 13.

By co-expressing two chimeric proteins of the invention in a Gram negative bacteria, the inventors have demonstrated that the bacteria can function as a biosensor, decreasing its bioluminescence signal in the presence of the antigen and in a dose-dependent manner. Thus, in another aspect, the present invention relates to the use of a host cell according to the present invention comprising at least two chimeric proteins of the invention as an *in vitro* antigen detection/quantification system (hereinafter, *in vitro* antigen detection system of the invention), wherein the "reporter protein domain" of the at least two chimeric proteins of the invention are complementary each other. As the skilled person understands, the particular embodiments disclosed previously for the host cell of the invention are applicable to the present inventive aspect.

Likewise, in another aspect, the present invention relates to an *in vitro* method for detecting/quantifying antigens, hereinafter method of the invention", comprising (a) contacting an isolated sample with a host cell of the invention comprising at least two chimeric proteins of the invention, wherein the reporter protein domain of the at least two chimeric proteins of the invention are complementary each other, and (b) detecting and quantifying the signal emitted by the protein reporter, wherein the intensity of the signal is inversely proportional to the concentration of antigen present in the sample.

As use herein, the term "antigen" relates to a molecule or molecular structure or any foreign particulate matter that can trigger an immune response in the animal body. Any antigen can be detected by the method of the invention. Thus, in a particular embodiment of the method of the invention, the antigen is a viral antigen, parasite antigen, a bacteria antigen, a tumoral antigen, or an inflammation protein antigen.

Examples of viral antigens include, without being limited to, antigens derived from HIV-1, such as gag, p17, p24, p41, p40, nef, pol, RT, p66, env, gp120 or gp160, gp40, p24, gag, vif, vpr, vpu, rev; from human herpes viruses, such as gH, gL gM gB gC gK gE or gD or derivatives thereof or Immediate Early protein such as ICP27, ICP 47, IC P 4, ICP36 from HSV1 or HSV2; from cytomegalovirus, especially Human, (such as gB or derivatives thereof); from Epstein Barr virus (such as gp350 or derivatives thereof), from Varicella Zoster Virus (such as gpl, II, III and IE63), or from a hepatitis virus such as hepatitis B virus (for example Hepatitis B Surface antigen or Hepatitis core antigen or pol), hepatitis C virus antigen and hepatitis E virus antigen; or from other viral pathogens, such as paramyxoviruses: Respiratory Syncytial virus (such as F and G proteins or derivatives thereof), or antigens from parainfluenza virus, measles virus, mumps virus, human papilloma viruses (for example HPV6, 11, 16, 18, eg L1, L2, E!, E2, E3, E4, E5, E6, E7), flaviviruses (e.g. Yellow Fever Virus, Dengue Virus, Tick-borne encephalitis virus, Japanese Encephalitis Virus) or Influenza virus cells, such as HA, NP, NA, or M proteins, or combinations thereof),

Examples of bacteria antigens include, without limiting to, antigens derived from bacterial pathogens such as *Neisseria* spp, including *N. gonorrhea* and *N*. *meningitidis,* eg, transferrin-binding proteins, lactoferrin binding proteins, PiIC, adhesins); *S*. *pyogenes* (for example M proteins or fragments thereof, C5A protease, *S*. *agalactiae, S. mutans; H, ducreyi;* Moraxella spp, including *M. catarrhalis,* also known as *Branhamella catarrhalis* (for example high and low molecular weight adhesins and invasins); *Bordetella* spp, including *B. pertussis* (for example pertactin, pertussis toxin or derivatives thereof, filamenteous hemagglutinin, adenylate cyclase, fimbriae), *B. parapertussis* and *B. bronchiseptica; Mycobacterium* spp., including *M*. *tuberculosis* (for example ESAT6, Antigen 85A, -B or -C, MPT 44, MPT59, MPT45, HSP10,HSP65, HSP70, HSP 75, HSP90, PPD 19kDa [Rv3763], PPD 38kDa [Rv0934] ), *M. bovis, M. leprae, M. avium, M. paratuberculosis, M. smegmatis; Legionella* spp, including *L. pneumophila; Escherichia* spp, including enterotoxic *E*. *coli* (for example colonization factors, heat-labile toxin or derivatives thereof, heat-stable toxin or derivatives thereof), enterohemorragic *E*. *coli,* enteropathogenic *E*. *coli* (for example shiga toxin-like toxin or derivatives thereof); *Vibrio* spp, including *V. cholera* (for example cholera toxin or derivatives thereof); *Shigella* spp, including *S. sonnei, S. dysenteriae, S. flexnerii; Yersinia* spp, including *Y. enterocolitica* (for example a Yop protein), *Y. pestis, Y. pseudotuberculosis; Campylobacter* spp, including *C. jejuni* (for example toxins, adhesins and invasins) and *C. coli; Salmonella* spp, including *S. typhi, S. paratyphi, S. choleraesuis, S. enteritidis; Listeria* spp., including *L. monocytogenes; Helicobacter* spp, including *H. pylori* (for example urease, catalase, vacuolating toxin); *Pseudomonas* spp, including *P. aeruginosa; Staphylococcus* spp., including *S*. *aureus, S. epidermidis; Enterococcus* spp., including *E. faecalis, E. faecium; Clostridium* spp., including *C. tetani* (for example tetanus toxin and derivative thereof), *C. botulinum* (for example botulinum toxin and derivative thereof), *C. difficile* (for example clostridium toxins A or B and derivatives thereof); *Bacillus* spp., including *B. anthracis* (for example botulinum toxin and derivatives thereof); *Corynebacterium* spp., including *C. diphtheriae* (for example diphtheria toxin and derivatives thereof); *Borrelia* spp., including *B. burgdorferi* (for example OspA, OspC, DbpA, DbpB), *B. garinii* (for example OspA, OspC, DbpA, DbpB), B. afzelii (for example OspA, OspC, DbpA, DbpB), *B. andersonii* (for example OspA, OspC, DbpA, DbpB), *B. hermsii; Ehrlichia* spp., including *E. equi and* the agent of the Human Granulocytic Ehrlichiosis; *Rickettsia* spp, including *R. rickettsii; Chlamydia* spp., including *C. trachomatis* (for example MOMP, heparin-binding proteins), *C. pneumoniae* (for example MOMP, heparin-binding proteins), *C. psittaci; Leptospira* spp., including *L. interrogans; Treponema* spp., including *T. pallidum* (for example the rare outer membrane proteins), *T. denticola, T. hyodysenteriae;* M. tuberculosis such as Rv2557, Rv2558, RPFs: Rv0837c, Rv1884c, Rv2389c, Rv2450, Rv1009, aceA (Rv0467), PstSI, (Rv0932), SodA (Rv3846), Rv2031c 16kDal., Tb Ra12, Tb H9, Tb Ra35, Tb38-1, Erd 14, DPV, MTI, MSL, mTTC2 and hTCC1 (V1lO 99/51748); *Streptococcus* spp, including S. *pneumoniae* (PsaA, PspA, streptolysin, choline-binding proteins) and the protein antigen Pneumolysin, and mutant detoxified derivatives thereof; *Haemophilus* spp., including *H. influenzae* type 8 (for example PRP and conjugates thereof), non-typeable *H. influenzae,* for example OMP26, high molecular weight adhesins, P5, P6, protein D and lipoprotein D, and fimbrin and fimbrin derived peptides.

Examples of parasite antigens include, without limited to, antigens derived from parasites such as *Plasmodium* spp., including *P. falciparum* (for example RTS, S, TRAP MSP1, AMAI, MSP3, EBA, GLURP, RAPI, RAP2, Sequestrin, PfEMP1, Pf332, LSA1, LSA3, STARP, SALSA, PfEXP1, Pfs25, Pfs28, PFS27/25, Pfs16, Pfs48/45, Pfs230 and their analogues in Plasmodium spp.); *Toxoplasma* spp., including *T. gondii* (for example SAG2, SAG3, Tg34); *Entamoeba* spp., including *E. histolytica; Babesia* spp., including *B. microti; Trypanosoma* spp., including *T. cruzi; Giardia* spp., including *G. lamblia; leishmania* spp., including *L. major, Pneumocystis* spp., including *P. carinii; Trichomonas* spp., including *T. vaginalis; Schisostoma* spp., including *S. mansoni,* or derived from yeast such as *Candida* spp., including *C. albicans; Cryptococcus* spp., including *C. neoformans.*

Examples of tumoral antigens include, without being limited to, tumour rejection antigens such as those for prostrate, breast, colorectal, lung, pancreatic, renal or melanoma cancers. Exemplary antigens include MAGE 1, 3 and MAGE 4 or other MAGE antigens, PRAME, BAGE, Lage (also known as NY Eos 1) SAGE and HAGE or GAGE. Indeed these antigens are expressed in a wide range of tumour types such as melanoma, lung carcinoma, sarcoma and bladder carcinoma. Prostate antigens include, without being limited to, Prostate specific antigen (PSA), PAP, PSCA, PSMA or antigen known as Prostase, and P501 S. Breast cancer antigens such as Muc- 1, Muc-2, EpCAM, her 2/ Neu, mammaglobin.

Examples of inflammation-related antigens include, without being limited to, Interleukin 1alpha and 1beta (IL-1 α, IL1β), Interleukin 2 (IL-2), Interleukin 6 (IL-6), Interleukin 10 (IL-10), Interleukin 11 (IL-11), Interleukin 12 (IL-12), Interleukin 17 (IL-17), Interleukin 18 (IL-18), Interleukin 22 (IL-22), Tumor necrosis factor alpha (TNF-α), Interferon gamma (IFN-γ), C-reactive protein (CRP), procalcitonin (PCT).

The first step of the method of the invention comprises contacting an isolated sample with a host cell of the invention comprising at least two chimeric proteins of the invention, wherein the reporter protein domain of the at least two chimeric proteins of the invention are complementary each other

As use herein, the term "sample" refers to a small part or quantity of something intended to show what the whole is like. Examples of samples include, without being limited to, the sample is a food- or feed-product sample, a pharmaceutical, veterinary or biotechnological product sample, a water system sample, a fluid sample, a soil sample, a plant sample, or a clinical sample such as saliva, blood, plasma, urine or faeces. The clinical sample may come from a subject, both animal and human. The term "subject" in the context of the present disclosure is a mammal, e.g., a primate, preferably a higher primate, e.g., a human. In a particular embodiment of the method of the invention, alone or in combination with all or each one of the previous particular embodiments, the sample is a saliva sample, a urine sample, a plasma sample, a water sample or a food sample. It is common general knowledge how to obtain these kinds of samples for their analysis.

As used herein, "isolated" will mean material removed from its original environment (e.g., the natural environment in which the material occurs), and thus it is "altered by the hand of man" from its natural environment.

The expression referring to "the reporter protein domain of the at least two chimeric proteins of the invention are complementary each other" has been explained in previous paragraphs and it is applicable to the present inventive aspect together with all the particular embodiments disclosed above for other inventive aspects of the present invention.

Once this first step has been carried out, the method of the invention comprises a second step comprising detecting and quantifying the signal emitted by the protein reporter. Tools and techniques for detecting and quantifying the signal emitted by a protein reporter are widely known in the state of the art, and any of them can be used in the context of the present invention. Examples of these tools include, without being limited to, droplet, test tube and microplate spectrophotometers of ultraviolet and visible light spectra, including colorimeters, fluorimeters and luminescence readers. Tools using plastic, ceramic and metallic chips, cellulose and nitrocellulose membranes, nanoparticles, lasers, light and fluorescence emission sources, heat emission sources, and using visual inspection, photographic cameras, charge-coupled device (CCD) cameras or any other device able to detect an electromagnetic signal or radioactive particle.

Finally, once the signal of the protein report is detected and quantified, the intensity of the signal is inversely proportional to the concentration of antigen present in the sample.

In a last aspect, the present invention also relates to a kit comprising a chimeric protein, the polynucleotide, the vector or the host cell of the invention.

"Kit" as used in the present invention refers to a product containing the different reagents necessary to carry out the method of the invention, which are packaged to enable them to be transported and stored. The kit may further include, without any limitation, buffers, agents to prevent contamination, inhibitors of protein degradation, etc. Suitable materials for packaging the components of the kit include glass, plastic (polyethylene, polypropylene, polycarbonate and the like), bottles, vials, paper, sachets and the like. Additionally, the kits of the invention may contain instructions for simultaneous, sequential or separate use of the different components in the kit. Said instructions may be in the form of printed material or in the form of an electronic medium capable of storing instructions so that they can be read by a subject, such as electronic storage media (magnetic disks, tapes and the like), optical media (CD-ROM, DVD) and the like.

The chimeric protein, the polynucleotide, the vector or the host cell of the invention have been defined and explained previously throughout the present description. Said definitions and explanations, as well as all the particular embodiments, are all applicable to the kit of the invention.

### DESCRIPTION OF THE DRAWINGS

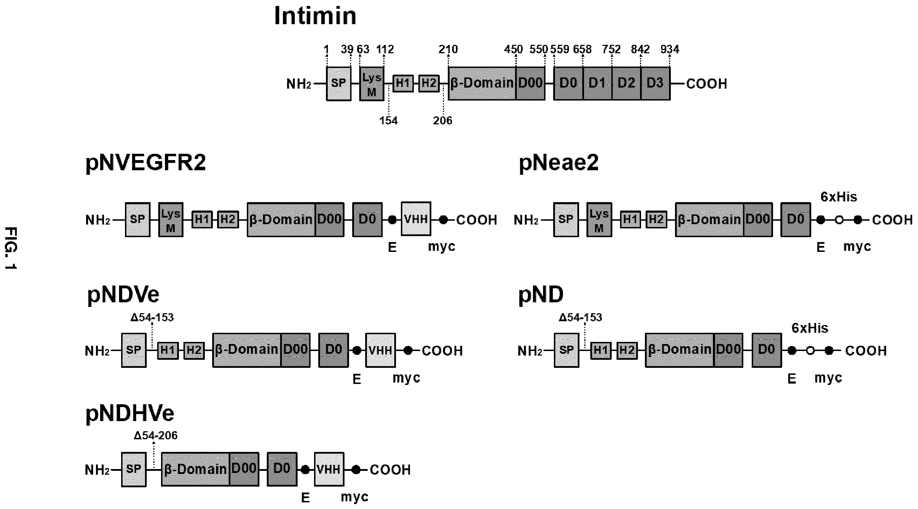

**Figure 1****. Schematic representation of the full-length WT intimin and intimin constructs.** Each box delimits a different protein domain. Numbers relate to the corresponding amino acid in the EHEC intimin WT protein based on its crystal (Fairman *et al.,* 2012). The periplasmic helices region was predicted using secondary structure algorithms. Deletions are marked with the Δ symbol followed by the amino acid range deleted. SP = Signal Peptide, H = helix, D = Immunoglobulin-like domain (D00, D0, etc.), E = E-tag, myc = c-myc-tag, 6xHls = chain of six histidines. Schemes are not at scale to maintain visual clarity.

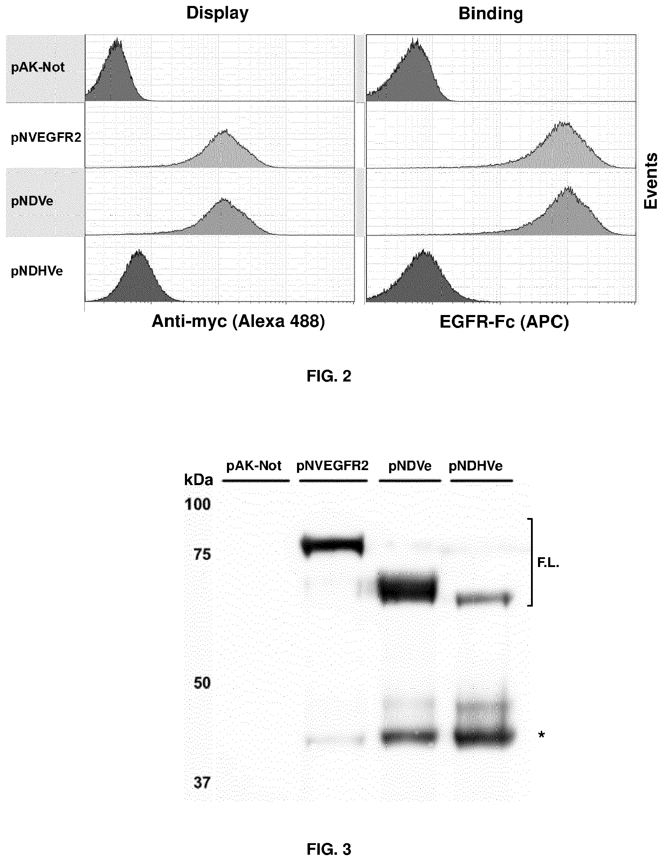

**Figure 2****. Surface display and antigen binding capabilities of the intimin-nanobody constructs with periplasmic deletions.** Flow cytometry analysis of induced *E. coli* EcM1 bacteria carrying the empty vector (pAK-Not), the unmodified intimin-nanobody fusion (pNVEGFR2), the LysM-deletion derivative (pNDVe) or the construct lacking the LysM and α-helices (pNDHVe). Left, display of the intimin proteins on the outer membrane. Histogram shows fluorescence intensity of bacteria stained with anti-myc tag mAb and secondary anti-mouse IgG-Alexa488.Right, binding of antigen by the surface displayed nanobody. Histogram shows fluorescence intensity of bacteria incubated with 50 nM of biotinylated hEGFR-Fc and secondary streptavidin-APC.
**Figure 3****. Western blot analysis of the expression for intimin constructs with periplasmic modifications.** Samples analyzed contain whole-cell protein extracts from induced *E. coli* EcM1 cultures carrying the indicated plasmids. After electrophoresis, proteins were transferred to a membrane and incubated with anti-c-myc mouse mAb and secondary goat anti-mouse polyclonal serum conjugated with peroxidase (POD). The empty vector (pAK-Not) was used as a negative control. Molecular weights in kDa are indicated on the left. Bands corresponding to the full-length proteins are indicated as F.L. Main proteolytic fragments are indicated with an asterisk.

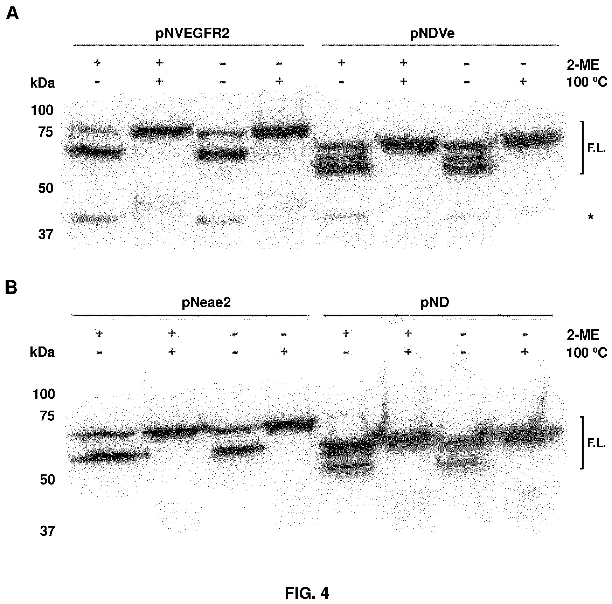

**Figure 4****. Western blot analysis of the effect of the LysM domain on the stability of the intimin b-barrel.** Western blot analysis of whole-cell protein extracts from induced *E. coli* EcM1 cultures carrying **(A)** plasmids with parental intimin-nanobody fusion (pNVEGFR2) and lacking the LysM domain (pNDVe) **(B)** intimin constructs with a C-terminal 6xHis peptide from parental (pNeae2) and LysM-deletion mutant (pND). Samples were boiled or not in a SDS-urea loading buffer with or without β-mercaptoethanol (2-ME). After electrophoresis, proteins were transferred to a membrane and incubated with anti-c-myc mouse mAb and secondary goat anti-mouse polyclonal serum conjugated with peroxidase (POD). Molecular weight standards are indicated on the left (in kDa). Bands corresponding to the full-length proteins are indicated as F.L. Main proteolytic fragments are indicated with an asterisk.

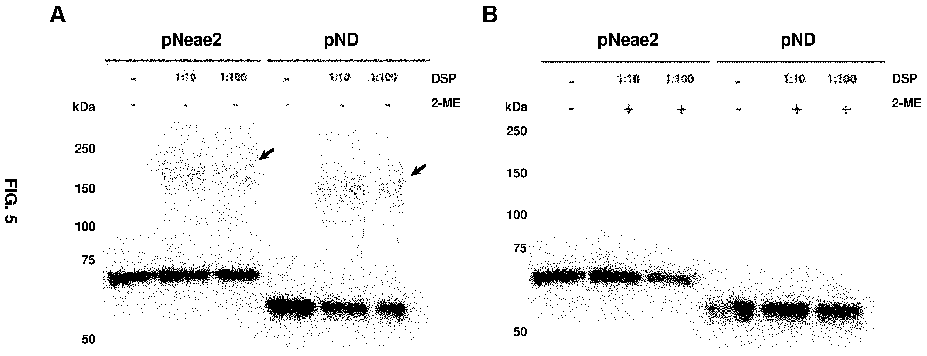

**Figure 5****. Crosslinking of intimin constructs with and without the LysM domain using DSP.** Western blot analysis of whole-cell protein extracts from induced *E. coli* EcM1 cultures carrying plasmids expressing intimin constructs with a C-terminal 6xHis peptide from parental (pNeae2) and LysM-deletion mutant (pND) **(A)** Samples of whole-cell bacteria treated with increasing concentrations of DSP and no 2-ME in the loading buffer. **(B)** Samples of whole-cell bacteria treated with increasing concentrations of DSP and 2-ME in the loading buffer. After electrophoresis, proteins were transferred to a membrane and incubated with anti-c-myc mouse mAb and secondary goat anti-mouse polyclonal serum conjugated with peroxidase (POD). Molecular weight standards are indicated on the left (in kDa). Arrows indicate bands of a molecular weight compatible with the formation of homodimers

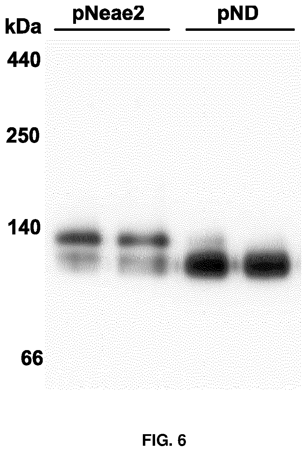

**Figure 6****. BN-PAGE analysis of the quaternary structure of intimin constructs with or without the LysM domain.** Protein samples were extracted from the outer membrane of cultures of *E. coli* EcM1 carrying the intimin-6xHis constructs with (pNeae2) and without (pND) LysM. Extraction was performed using the zwitterionic detergent Zwittergent 3-14 (see Material and methods: Outer membrane protein extraction). Samples were analyzed by electrophoresis in native conditions to maintain any oligomeric interaction (BN-PAGE) For Western blot, the resulting gel was transferred to a membrane and incubated with anti-c-myc mouse mAb and secondary goat anti-mouse polyclonal serum conjugated with peroxidase (POD). Native molecular weights in kDa are indicated on the left.

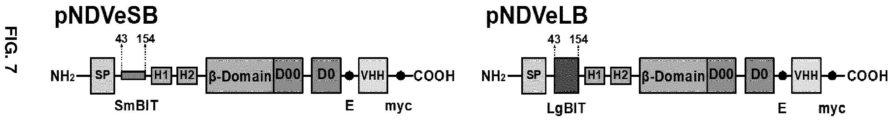

**Figure 7****. Schematic representation of the intimin-nanobody constructs with the LysM deletion fused to the NanoBiT fragments.** Each box delimits a different protein domain. Numbers relate to the corresponding amino acid in the intimin WT protein based on its crystal (Fairman *et al.,* 2012). SP = Signal Peptide, H = helix, D = Immunoglobulin-like domain, E = E-tag, myc = c-myc-tag, Size is not proportional to maintain visual clarity.

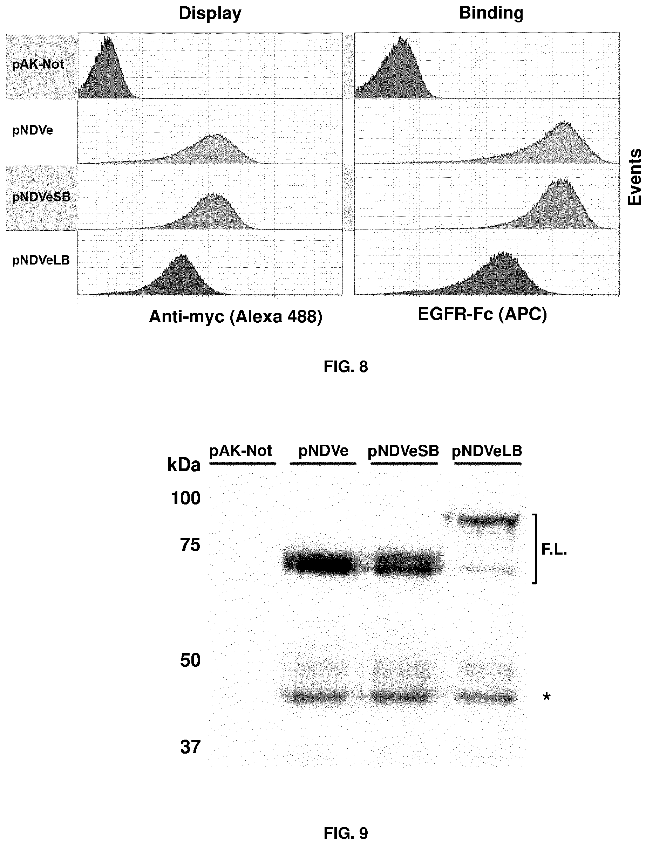

**Figure 8****. Display and antigen binding capabilities of intimin-nanobody constructs with the LysM deletion fused to the NanoBiT fragments.** Flow cytometry analysis of induced *E. coli* EcM1 bacteria carrying the empty vector (pAK-Not) or the intimin construct lacking LysM with no fusion (pNDVe) with the SmBiT fragment (pNDVeSB) or the LgBiT fragment (pNDVeLB. Left, display of the constructs in the outer membrane. Histogram shows fluorescence intensity of bacteria stained with anti-myc tag mAb and secondary anti-mouse IgG-Alexa488. Right, binding of antigen by the displayed nanobody. Histogram shows fluorescence intensity of bacteria incubated with 50 nM of biotinylated hEGFR-Fc and secondary streptavidin-APC.
**Figure 9****. Western blot analysis of the expression of intimin-nanobody constructs lacking LysM with periplasmic fusions to the NanoBiT fragments.** Samples analyzed contain whole-cell protein extracts from induced *E. coli* EcM1 cultures carrying the empty vector (pAK-Not) or the intimin construct lacking LysM with no fusion (pNDVe) with the SmBiT fragment (pNDVeSB) or the LgBiT fragment (pNDVeLB). After electrophoresis, proteins were transferred to a membrane and incubated with anti-c-myc mouse mAb and secondary goat anti-mouse polyclonal serum conjugated with peroxidase (POD). The empty vector (pAK-Not) was used as a negative control. Molecular weights in kDa are indicated on the left. Bands corresponding to the full-length proteins are indicated as F.L. Main proteolytic fragments are indicated with an asterisk.

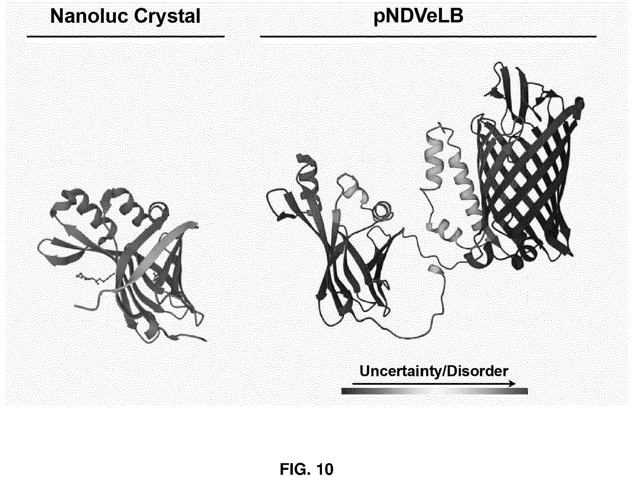

**Figure 10****. AlphaFold prediction of the structure of the intimin-nanobody construct lacking LysM with the periplasmic fusion to the LgBiT fragment.** Detail of the β-barrel, periplasmic helices and LgBiT domain (right). The full-lenght NanoLuc crystal (PDB 7MJB) is shown in the left, with the β-strand corresponding to the SmBiT colored inlight grey.

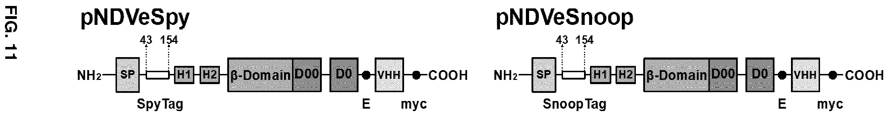

**Figure 11****. Schematic representation of the intimin-nanobody constructs with the LysM deletion fused to SpyTag or SnoopTag.** Each box delimits a different protein domain. Numbers relate to the corresponding amino acid in the intimin WT protein based on its crystal (Fairman *et al.,* 2012). SP = Signal Peptide, H = helix, D = Immunoglobulin-like domain, E = E-tag, myc = c-myc-tag, Schemes not in scale to maintain visual clarity.

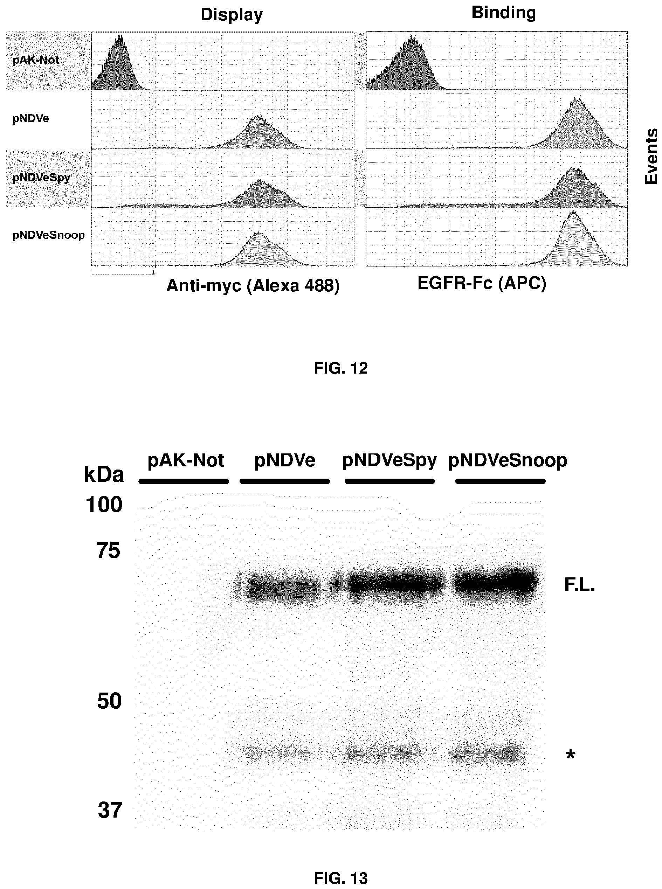

**Figure 12****. Display and antigen binding capabilities of intimin-nanobody constructs with the LysM deletion fused to the Spy and Snoop tags.** Flow cytometry analysis of induced *E. coli* EcM1 bacteria carrying the empty vector (pAK-Not) or the intimin construct lacking LysM with no tag (pNDVe) with the SpyTag (pNDVeSpy) or the SnoopTag (pNDVeSnoop). Left, display of the constructs in the outer membrane. Histogram shows fluorescence intensity of bacteria stained with anti-myc tag mAb and secondary anti-mouse IgG-Alexa488. Right, binding of antigen by the displayed nanobody. Histogram shows fluorescence intensity of bacteria incubated with 50 nM of biotinylated hEGFR-Fc and secondary streptavidin-APC.
**Figure 13****. Western blot analysis of the expression of intimin-nanobody constructs lacking LysM with periplasmic fusions to the Spy and Snoop tags.** Samples analyzed contain whole-cell protein extracts from induced *E. coli* EcM1 cultures carrying the empty vector (pAK-Not) or the intimin construct lacking LysM with no fusion (pNDVe) with the SpyTag (pNDVeSpy) or with the SnoopTag (pNDVeSnoop). After electrophoresis, proteins were transferred to a membrane and incubated with anti-c-myc mouse mAb and secondary goat anti-mouse polyclonal serum conjugated with peroxidase (POD). The empty vector (pAK-Not) was used as a negative control. Molecular weights in kDa are indicated on the left. Bands corresponding to the full-length proteins are indicated as F.L. Main proteolytic fragments are indicated with an asterisk.

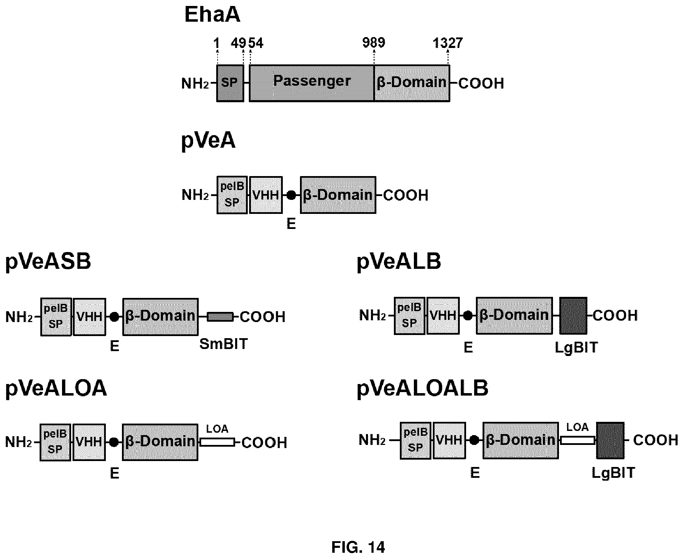

**Figure 14****. Schematic representation of the full-length WT EhaA and EhaA-nanobody constructs.** Each box delimits a different protein domain. Numbers relate to the corresponding amino acid in the EhaA WT protein. SP = Signal Peptide, E = E-tag, LOA= Linker OmpA full-length. Size is not proportional to maintain visual clarity.

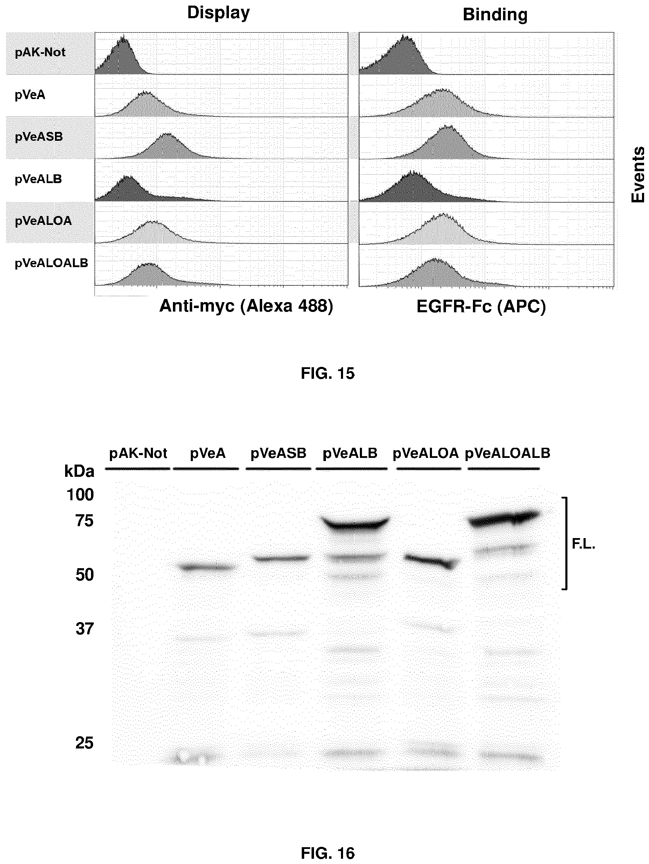

**Figure 15****. Display and antigen binding capabilities of EhaA-nanobody constructs with and without periplasmic fusions.** Flow cytometry analysis of induced *E. coli* EcM1 bacteria carrying the empty vector (pAK-Not) or the EhaA-nanobody constructs with either no periplasmic fusion (pVeA), or a fusion to the SmBIT fragment (pVeASB), the LgBiT domain (pVeALB), the full linker of OmpA (pVeALOA) or the full linker of OmpA and the LgBiT domain (pVeALOALB). Left, display of the constructs in the outer membrane. Histogram shows fluorescence intensity of bacteria stained with anti-E tag mAb and secondary anti-mouse IgG-Alexa488. Right, binding of antigen by the displayed nanobody. Histogram shows fluorescence intensity of bacteria incubated with 50 nM of biotinylated hEGFR-Fc and secondary streptavidin-APC.
**Figure 16****. Western blot analysis of the expression of EhaA-nanobody constructs with or without periplasmic fusions.** Samples analyzed contain whole-cell protein extracts from induced *E. coli* EcM1 cultures carrying the empty vector (pAK-Not) or EhaA-nanobody constructs with either no periplasmic fusion (pVeA), the SmBIT fragment (pVeASB), the LgBiT fragment (pVeALB), the full linker of OmpA (pVeALOA) or the full linker of OmpA and the LgBiT fragment (pVeALOALB) After electrophoresis, proteins were transferred to a membrane and incubated with anti-E mouse mAb and secondary goat anti-mouse polyclonal serum conjugated with peroxidase (POD). The empty vector (pAK-Not) was used as a negative control. Molecular weights in kDa are indicated on the left. Bands corresponding to the full-length proteins are indicated as F.L.

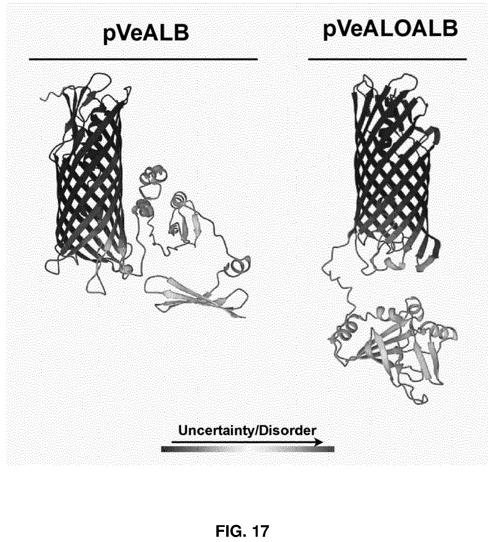

**Figure 17****. AlphaFold prediction of the structures of the EhaA-nanobody constructs with fusions to the LgBiT fragment or the LOA and LgBiT fragment.** Detail of the β-barrel and the periplasmic LgBiT and LOA-LgBiT fusions.

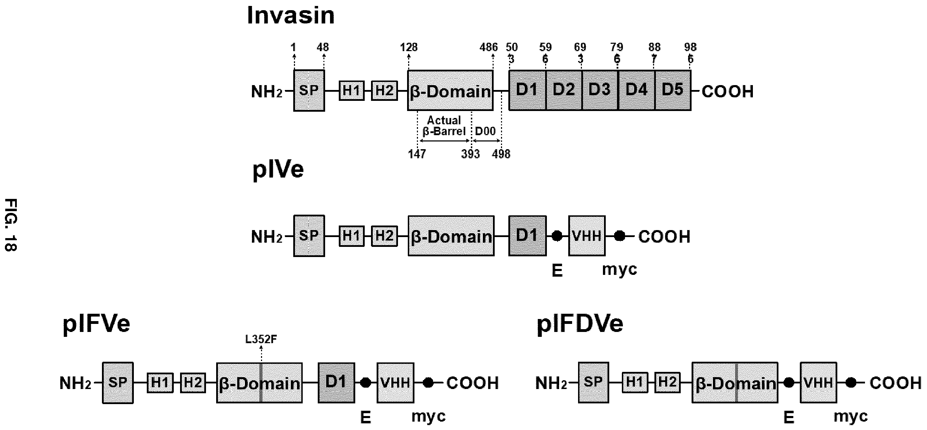

**Figure 18****. Schematic representation of the full-length invasin and invasin-nanobody constructs.** Each box represents a different feature. Numbers relate to the corresponding amino acid in the intimin WT protein based on its crystal (Fairman *et al.,* 2012). The D00 domain was predicted by AlphaFold. SP = Signal Peptide, H = helix, D = Ig-like domain, L352F = Leucine 352 to phenylalanine mutation, E = E-tag, myc = c-myc-tag. Size is not proportional to maintain visual clarity.

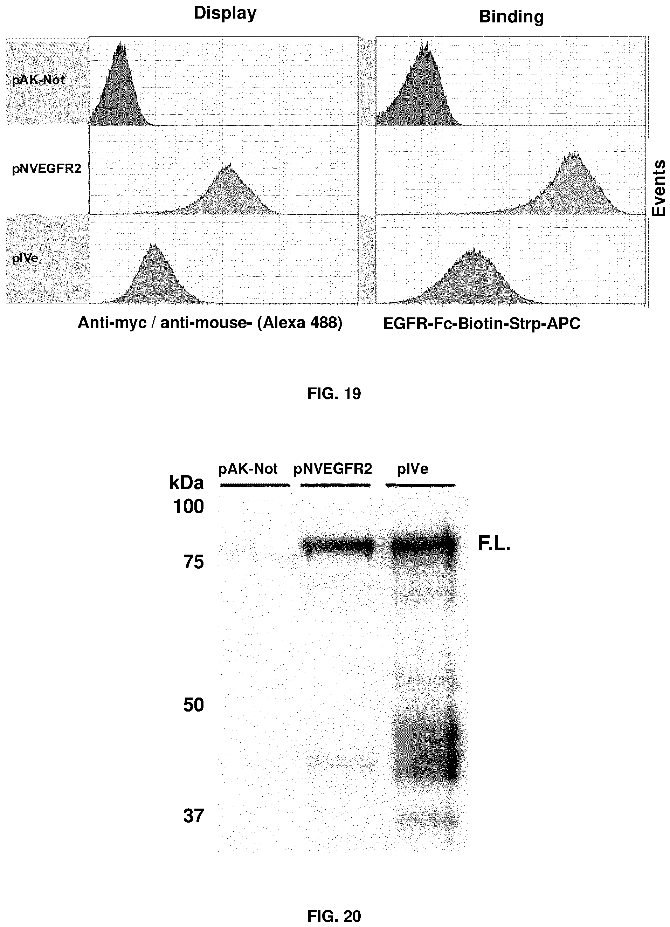

**Figure 19****. Display and antigen binding capabilities of the invasin-nanobody construct.** Flow cytometry analysis of *E. coli* EcM1 bacteria transformed with plasmids expressing either no construct (pAK-Not), the invasin-nanobody construct (pIVe) or the original intimin-nanobody construct with LysM (pNVEGFR2) as positive control. Left, display of the constructs in the outer membrane. Histogram shows fluorescence intensity of bacteria stained with anti-myc tag mAb and secondary anti-mouse IgG-Alexa488. Right, binding of antigen by the displayed nanobody. Histogram shows fluorescence intensity of bacteria incubated with 50 nM of biotinylated hEGFR-Fc and secondary streptavidin-APC.
**Figure 20****. Western blot analysis of the expression for the invasin-nanobody construct.** Samples analyzed contain whole-cell protein extracts from induced *E. coli* EcM1 cultures carrying the indicated plasmids. After electrophoresis, proteins were transferred to a membrane and incubated with anti-c-myc mouse mAb and secondary goat anti-mouse polyclonal serum conjugated with peroxidase (POD). The empty vector (pAK-Not) was used as a negative control. Molecular weights in kDa are indicated on the left. Bands corresponding to the full-length proteins are indicated as F.L.

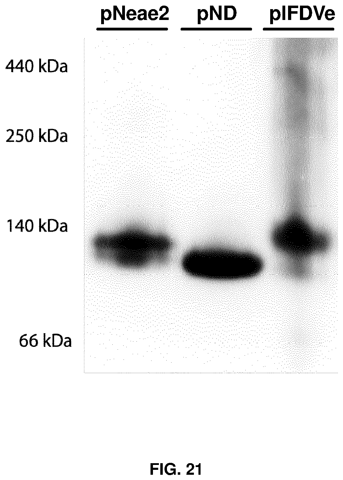

**Figure 21****. BN-PAGE analysis of the quaternary structure of the invasin construct with the L352F mutation and without the D1 domain.** Protein samples were extracted from the outer membrane of cultures of *E. coli* EcM1 carrying the intimin-6xHis constructs with (pNeae2) and without (pND) LysM or the invasin construct with the L352F mutation and without the D1 domain. Native molecular weights in kDa are indicated on the left.

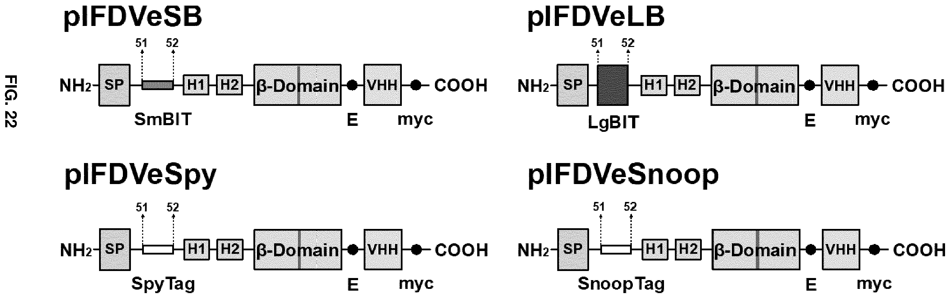

**Figure 22****. Schematic representation of the invasin-nanobody constructs with periplasmic fusions to NanoBiT fragments, SpyTag or SnoopTag.** Each box represents a different feature. Numbers relate to the corresponding amino acid in the intimin WT protein based on its crystal. SP = Signal Peptide, H = helix, D = Ig-like domain, L352F = Leucine to phenylalanine mutation, E = E-tag, myc = c-myc-tag. Schemes are not at scale to maintain visual clarity.

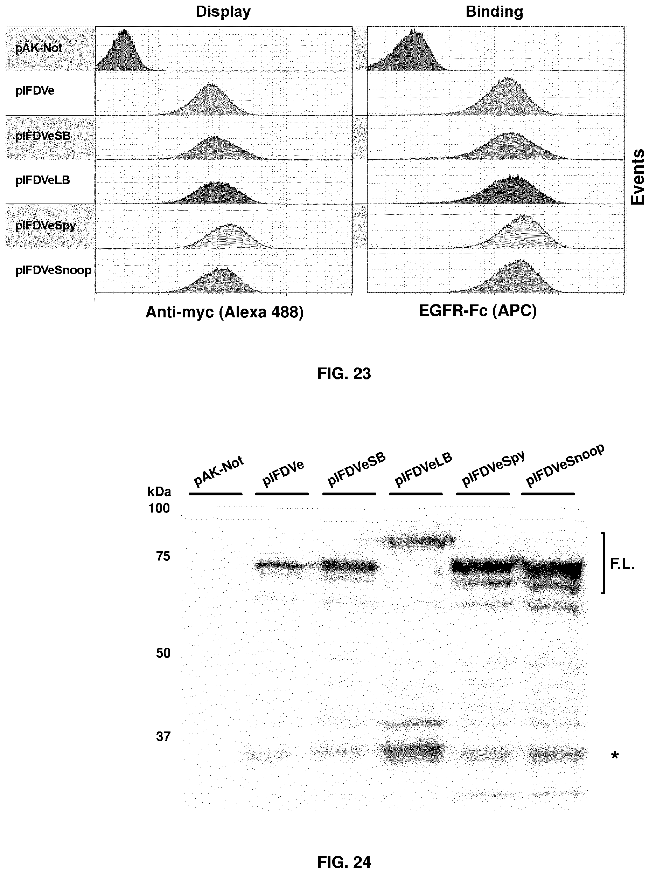

**Figure 23****. Display and antigen binding capabilities of the invasin-nanobody construct IFDVe with periplasmic fusions to NanoBiT fragments, SpyTag or SnoopTag.** Flow cytometry analysis of *E. coli* EcM1 bacteria transformed with plasmids expressing either no construct (pAK-Not), the invasin-nanobody IFDVe construct (pIFDVe) with periplasmic fusions to the SmBiT fragment (pIFDVeSB), LgBiT fragment (pIFDVeLB), Spytag (pIFDVeSpy) or SnoopTag (pIFDVeSnoop). Left, display of the constructs in the outer membrane. Histogram shows fluorescence intensity of bacteria stained with anti-myc tag mAb and secondary anti-mouse IgG-Alexa488. Right, binding of antigen by the displayed nanobody. Histogram shows fluorescence intensity of bacteria incubated with 50 nM of biotinylated hEGFR-Fc and secondary streptavidin-APC.
**Figure 24****. Western blot analysis of the expression for the invasin-nanobody construct IFDVe with periplasmic fusions to NanoBiT fragments, SpyTag or SnoopTag.** Samples analyzed contain whole-cell protein extracts from induced *E. coli* EcM1 cultures carrying the indicated plasmids. After electrophoresis, proteins were transferred to a membrane and incubated with anti-c-myc mouse mAb and secondary goat anti-mouse polyclonal serum conjugated with peroxidase (POD). The empty vector (pAK-Not) was used as a negative control. Molecular weights in kDa are indicated on the left. Bands corresponding to the full-length proteins are indicated as F.L. Main proteolytic fragments are indicated with an asterisk.

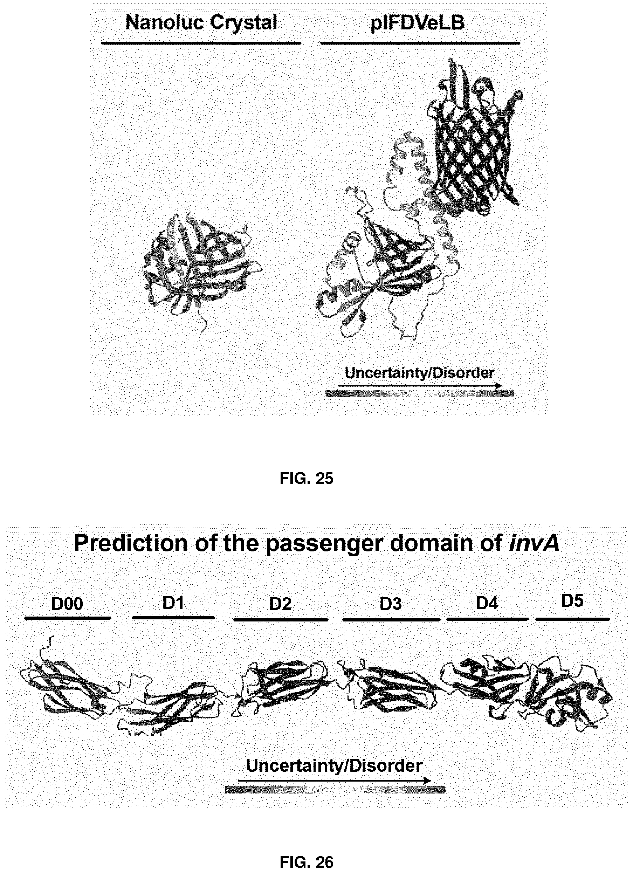

**Figure 25****. AlphaFold prediction of the structure of pIFDVeLB.** Detail of the β-barrel and the periplasmic helices and LgBiT domain (right). The full NanoLuc crystal (PDB 7MJB) is shown in the left to compare the folding. The β-strand corresponding to the SmBiT is colored in light grey.
**Figure 26****. AlphaFold prediction of the passenger domain of the WT invasin.** Each immunoglobulin-like domain is indicated. Notice the appearance of a previously undescribed D00 domain.

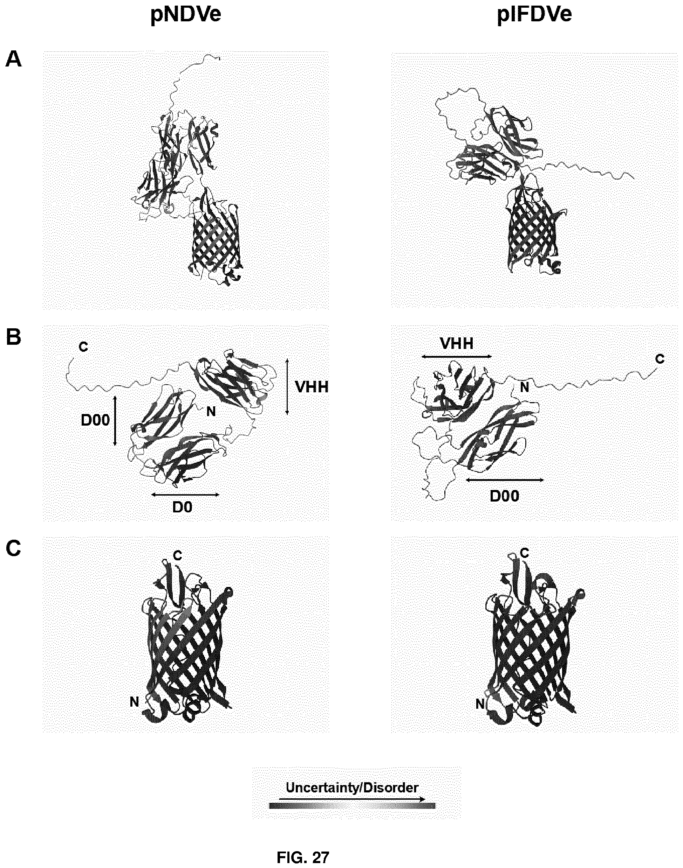

**Figure 27****. Comparison of the AlphaFold structure predictions for pNDVe and pIFDVeLB.** Periplasmic helices have been omitted for visual clarity. **(A)** β-barrel and displayed Ig-like and nanobody domains. (B) Detail of the Ig-like domains and the nanobody **(C)** Detail of the β-barrel.

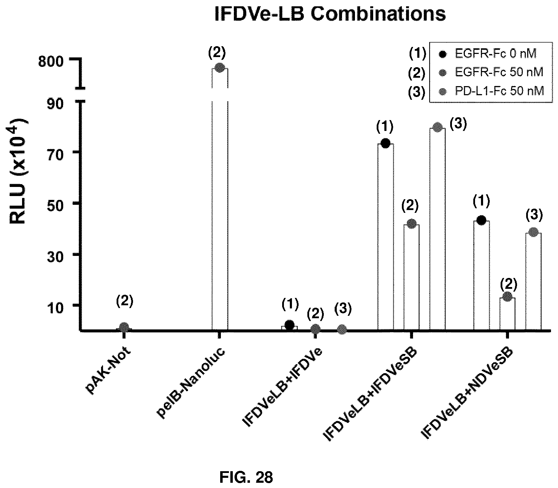

**Figure 28****. Detection of extracellular protein antigen by bacterial antigen receptors.** Relative luminescence units (Lum/OD₆₀₀) of EcM1 bacteria transformed with the indicated combinations of bacterial antigen receptors of EGFR based on invasin LgBiT (IFDVeLB) and SmBIT fusions to invasin (IFDVeSB) or intimin (NDVeSB). Bacteria lacking SmBIT (IFDVe) and carrying the empty vector (pAK-Not) were used as negative controls. A culture of EcM1 bacteria expressing in the periplasm a fusion of the N-terminal SP of PelB to the full-length Nanoluc was used as positive control. When indicated, the protein antigens EGFR-Fc (positive) or PD-L1-Fc (negative) were added at 50 nM to bacteria for 1 h prior to bioluminescence quantification.

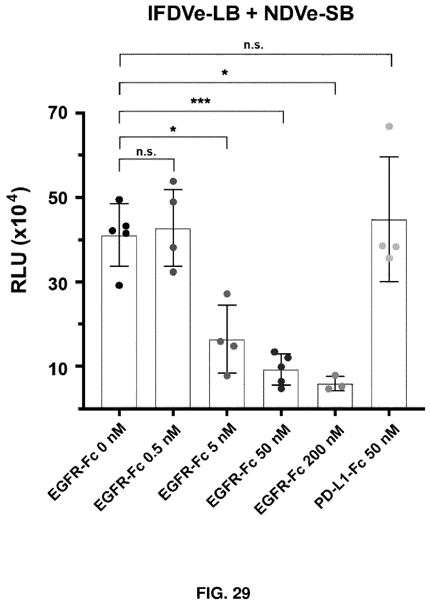

**Figure 29****. Dose-dependent detection of extracellular protein antigen by bacterial antigen receptors.** Relative luminescence units (Lum/OD₆₀₀) of EcM1 bacteria expressing a combination of bacterial antigen receptors of EGFR based on invasin LgBIT (IFDVeLB) and intimin SmBIT (NDVeSB). Different concentrations of the protein antigen EGFR-Fc (from 0 to 200 nM, as indicated) or negative control antigen PD-L1-Fc (50 nM) were added to bacteria for 1 h prior to bioluminescence quantification. Error bars represent the standard deviation. Statistical significance inferred by paired t-test analysis. n.s. non-significant, (*) p-value < 0.05 (***) p-value < 0.001.

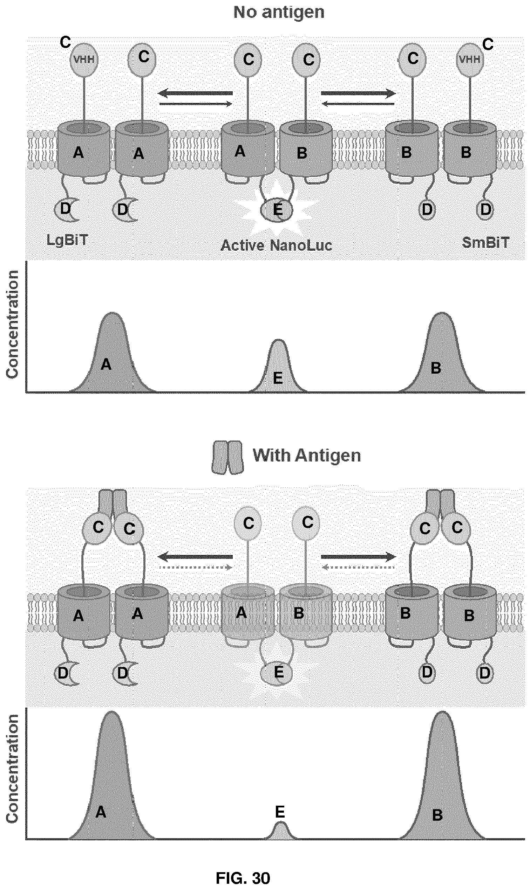

**Figure 30****. Proposed model for the functioning of our antigen biosensor.** In the absence of antigen, the chimeric intimin and invasin proteins form homodimers with a higher frequency than heterodimers. When the dimeric antigen is present, it binds simultaneously to both nanobodies in the pair, stabilizing the homodimers and reducing their capacity to dissociate and form heterodimers. This reduces the luminescence signal produced by the heterodimers. Barrels of invasin (A), barrels of intimin (B), Nanobodies (C), SmBiT (ovals) and LgBiT (half-moons) (D) when inactive and (E) when they complement to produce luminescence.

### EXAMPLE

### Development of an antigen sensing E. coli strain based on antibody-displaying outer membrane proteins.

A system of bacterial receptors in the OM that would enable the binding of an extracellular target antigen and the generation of a signal upon recognition is disclosed below.

### MATERIAL AND METHODS

### 1. Bacterial strains and growth conditions.

*E. coli* strains used in this work can be found in Table 2.

**Table 2.**

| Bacterial strains used in this invention. (Blattner, F.R., et al., 1997. Science 277, 1453-1462; Datsenko, K.A., Wanner, B.L., 2000. Proceedings of the National Academy of Sciences 97, 6640-6645; Salema, V., et al., 2013. PLoS ONE 8, e75126; Durfee, et al., 2008. J Bacteriol 190, 2597-2606) | | |
|---|---|---|
| Strain | Genotype | Reference |
| MG1655 | K-12 (F- λ-) | (Blattner et al., 1997) |
| BW25141 | (F- λ-) *Δ(araD-araB)567, ΔlacZ4787*(::*rrnB-3*), *Δ*(*phoB-phoR*)580*, galU95,* Δ*uidA3::pir, recA1, endA9*(*del-ins*)*::FRT*, *rph-1,* Δ(*rhaD-rhaB*)568, *hsdR51* | (Datsenko and Wanner, 2000) |
| EcM1 | MG1655Δ*fimA-H* | (Salema et al., 2013) |
| DH10B-T1R | (F- λ-) *mcrA Δmrr-hsdRMS-mcrBC cp801acZDM 15 Δ*l*acX74 recA1 endA1 araD139* Δ*(ara, leu)*7697 *galU galK rpsL (StrR) nupG tonA* | (Durfee et al., 2008) |

Bacteria were grown in Lysogeny Broth (LB) liquid medium or LB-agar plates (1.5% w/v) at 37 ºC, unless otherwise specified. Antibiotics and inducers were added to the media at the following concentrations: kanamycin (Km) at 50 µg/mL, chloramphenicol (Cm) at 30 µg/mL, ampicillin (Amp) at 150 µg/mL, anhydrotetracycline (aTc) at 200 ng/mL, isopropyl-β-D-thiogalactopyranoside (IPTG) at 0.1 mM and L-arabinose (L-ara) at 0.4% (w/v). Diaminopimelic acid (DAPA) at 0.3 mM was added to cultures of the *dapA* mutant *E. coli* MFDpir strain. Plasmids carrying the π-dependent R6K origin of replication (Stalker et al., 1982), such as pGE and pGEC derivatives, were cloned, propagated and isolated using *E. coli* BW25141. *E. coli* DH10BT1^{R} was used as a host for the rest of plasmids.

For expression of the different intimin, invasin and autotransporters constructs *E. coli* EcM1 was freshly transformed with the corresponding plasmid and individual colonies harvested from plates were used to start pre-inoculate cultures. Pre-inoculums were grown at 30 ºC, with glucose 2% (w/v) for repression of the P_{*l*ac} promoter and static conditions overnight (O/N). The next day, pre-inoculum cultures were centrifuged (3 minutes, 4000 g) and washed using fresh LB medium twice to remove the glucose and finally diluted to an O.D. of 0.5 at 600 nm (OD₆₀₀). The cultures were later induced using IPTG for 3 h at 30 ºC and 160 rpm.

For expression of GFP using the different tumor-induced promoter candidates, each promoter (P*_{lldP}*, *P_{lldP}*,* P*_{uof}* P*ₕₘₚ* and P*_{norV},)* controlling the GFP^{TCD} using the T7 RBS were integrated into the *csgCG* locus of EcM1. Two different clones from the resulting strains were grown O/N from individual colonies. After growth, cultures were diluted and grown up to 0.4 OD₆₀₀ for their induction. For measuring using a microplate reader (Victor2, PerkinElmer, Shelton, CT, USA) cultures were diluted again to 0.2 OD₆₀₀ using LB containing the specified concentrations of their respective inducer, sodium L-lactate (Sigma-Aldrich), H₂O₂ (Sigma Aldrich) or Spermine NONOate (SPER/NO) (Calbiochem) and grown for 22 h at 30 ºC, each condition with 6 technical replicates. Measurements for fluorescence (535 nm) and absorbance (OD₆₀₀) were taken every 15 minutes after a 30 s orbital shaking. For flow cytometry measuring of the expression, inducers were directly added to the cultures and grown at 37 ºC with agitation at 200 rpm unless otherwise specified.

For expression of integrases, individual colonies of each EcM1 strains carrying the different targets in the *yhjY* locus and the integrases either in plasmid or integrated in the *ypjA* or *ycgV* loci were used to inoculate 96-well plates containing LB medium. After O/N growth at 37 ºC and 200 rpm, cultures were serial diluted (1000-fold total dilution) with fresh medium containing the corresponding inducer and were grown again in the same conditions O/N.

### 2. Plasmids and DNA constructs.

Plasmids used in this work can be found in Table 3.

**Table 3 - Plamids used in this invention. (Veiga, E., et al., 1999. Molecular Microbiology 33, 1232-1243; Salema et al., 2013, cited ad supra; Salema et al., 2016b; Salema, V. et al., 2016b. mAbs 8, 1286-1301; Silva-Rocha et al., 2013. Nucleic Acids Res 41, D666-675).**

| **Name** | **Features** | **References** |
|---|---|---|
| pAK-Not (pAK) | CmR, pBR322 ori, lacIQ-Plac, empty vector used as a parental for all adhesin plasmids | (Veiga et al., 1999) |
| pNeae2 | pAK vector expressing a fusion of EHEC intimin residues 1-654, E-tag, 6xHis tag and c-myc-tag. | (Salema et al., 2013) |
| pNVEGFR2 | pNeae2 with a VHH anti-EGFR substituting the 6xHis tag | (Salema et al., 2016b) |
| pNDVe | pNVEGFR2 with a deletion of the LysM domain (Δ54-153) | This invention |
| pNDHVe | pNVEGFR2 with a deletion of the LysM domain and periplasmic helices (Δ54-206) | This invention |
| pND | pNeae2 with a deletion of the LysM domain (Δ54-153) | This invention |
| pNDVeSB | pNDVe with the SmBiT fragment fused between amino acids 43 and 154 | This invention |
| pNDVeLB | pNDVe with the LgBiT fragment fused between amino acids 43 and 155 | This invention |
| pNDVeSpy | pNDVe with the Spytag fused between amino acids 43 and 156 | This invention |
| pNDVeSnoop | pNDVe with the SnoopTag fused between amino acids 43 and 156 | This invention |
| pVgfpA | pAK vector expressing a fusion of the pelB signal peptide, a VHH anti-GFP, the E-tag and EhaA residues 989-1327 | (Salema et al., 2013) |
| pVeA | pVgfpA with a VHH against EGFR instead of a VHH against GFP | This invention |
| pVeASB | pVeA with the SmBiT fragment fused to the C terminal end using the first three amino acids of the OmpA linker (residues 192-194) | This invention |
| pVeALB | pVeA with the LgBiT fragment fused to the C terminal end using the first three amino acids of the OmpA linker (residues 192-194) | This invention |
| pVeALOA | pVeA with the complete OmpA linker (residues 192-208) fused to the C terminal end | This invention |
| pVeALOALB | pEVe with the LgBiT fragment fused to the C-terminal through the complete OmpA linker (residues 192-208) | This invention |
| pF330Ve | pAK vector expressing a fusion of the pelB pignal peptide, a VHH anti-GFP, the E-tag and the flu (Ag43) residues 710-1039 | This invention |
| pF330VeSB | pF330Ve with the LgBiT fragment fused to the C-terminal end through the complete OmpA linker (residues 192-208). | This invention |
| pF330VeLB | pF330Ve with the SmBiT fragment fused to the C-terminal end through the complete OmpA linker (residues 192-208). | This invention |
| pF487Ve | pAK vector expressing a fusion of the pelB signal peptide, a VHH anti-GFP, the E-tag and flu (Ag43) residues 553-1039 | This invention |
| pF487VeSB | pF487Ve with the LgBiT fragment fused to the C-terminal end through the complete OmpA linker (residues 192-208) | This invention |
| pF487VeLB | pF487Ve with the SmBiT fragment fused to the C-terminal end through the complete OmpA linker (residues 192-208) | This invention |
| pIVe | pAK vector expressing a fusion of invasin residues 1- 596, E-tag, a VHH anti-EGFR and c-myc-tag. | This invention |
| plFVe | plVe with the L352F mutation | This invention |
| plFDVe | plFVe without the D1 Ig-like domain (residues 503-596) | This invention |
| plFDVeSB | plFDVe with the SmBiT fragment fused between invasin residues 51 and 52 | This invention |
| plFDVeLB | plFDVe with the LgBiT fragment fused between invasin residues 51 and 53 | This invention |
| plFDVeSpy | plFDVe with the SpyTag fused between invasin residues 51 and 54 | This invention |
| pAK-pelB-Nanoluc | pAK vector for expression of the full length Nanoluc with an N-terminal pelB signal for periplasmic secretion | This invention |
| pSEVA221 | KmR, RK2-origin | (Silva-Rocha et al., 2013) |
| pSEVA221 -Ptet-NDVe | pSEVA221 with a Ptet promoter for expressing the NDVe construct. | This invention |
| pSEVA221 -Ptet-NDVeSB | pSEVA221 with a Ptet promoter for expressing the NDVeSB construct. | This invention |
| pSEVA221 -Ptet-IFDVe | pSEVA221 with a Ptet promoter for expressing the IFDVe construct. | This invention |
| pSEVA221 -Ptet-IFDVeSB | pSEVA221 with a Pₜₑₜ promoter for expressing the IFDVeSB construct. | This invention |

DNA constructs were produced by standard methods of DNA manipulation, digestion, ligation and modification (Ausubel et al., 2002) or one-step isothermal Gibson assembly (Gibson et al., 2009). DNA synthesis of the tested promoters, the intimin sequence lacking LysM and the LgBiT fragment was performed by ThermoFisher Scientific GeneArt. Oligonucleotides were purchased from Merck or Integrated DNA Technologies. Relevant oligonucleotides can be found in **Table 4.** Designs for integrase targets were kindly provided by Dr. Jérôme Bonnet based on previous works (Bonnet et al., 2013, 2012; Guiziou et al., 2019). For Polymerase Chain Reaction (PCR) either Taq DNA polymerase (NZYTaq II 2X Green Master Mix) for screening or high-fidelity DNA polymerase, Herculase II Fusion DNA polymerase (Agilent Technologies) for cloning were used. All DNA constructs were sequenced by Macrogen, Inc. or ACGT Inc.

**Table 4. Oligonucleotides used in this invention.**

| **Sequence** | **Use** | **SEQ ID NO** |
|---|---|---|
| CATTTTTCACTTCACAGGTC AAGCTT | Cloning in pAK-Not derived vectors | SEQ ID NO: 22 |
| CCGATTCATTAATGCAGCTG GC | Check cloning in pAK-Not derived vectors | SEQ ID NO: 23 |
| GGTAATAAGAAGTCCAGTG AACTACCG | Check cloning of intimin constructs | SEQ ID NO: 24 |
| CCGCGGTTACAACATTATG GAACGGCAGAGG | Cloning of pNDHVe | SEQ ID NO: 25 |
| AATTCGGTGGGGGAAGCGT GACCGGCTACCGGCTGTTC GAGGAGATTCTGGGCGGAG GGTCTCCGC | Cloning of SmBiT in pNDVe | SEQ ID NO: 26 |
| GGAGACCCTCCGCCCAGAA TCTCCTCGAACAGCCGGTA GCCGGTCACGCTTCCCCCA CCG | Cloning of SmBiT in pNDVe | SEQ ID NO: 27 |
| ACTCGGAACAGCATGGAGC C | Check cloning of LgBiT | SEQ ID NO: 28 |
| GCGCATATTGTGATGGTTG ATGCCTATAAACCGACCAA | Cloning of SpyTag in pNDVe | SEQ ID NO: 29 |
| GGGCGGAGGGTCTCCGCG GAAAAGC | | |
| CTTGGTCGGTTTATAGGCAT CAACCATCACAATATGCGC GCTTCCCCCACCGAATTCAT TTTCACC | Cloning of SpyTag in pNdVe | SEQ ID NO: 30 |
| AAACTGGGCGATATTGAATT TATCAAGGTGAACAAAGGC GGAGGGTCTCCGCGGAAAA GC | Cloning of SnoopTag in pNdVe | SEQ ID NO: 31 |
| TTTGTTCACCTTGATAAATT CAATATCGCCCAGTTTGCTT CCCCCACCGAATTCATTTTC ACC | Cloning of SnoopTag in pNdVe | SEQ ID NO: 32 |
| CATAGTGGTTTGTTTCTGCT CACC | Check cloning in pEVe vectors | SEQ ID NO: 33 |
| GGGTAGCTACATTGCGAAC CTTGC | Check cloning in pEVe vectors | SEQ ID NO: 34 |
| GCTTCCCCCACCGCCCTGT CCGAATTGCCACTTAATGCC AACC | Cloning of short linker in pEVeLB | SEQ ID NO: 35 |
| GGTCAAGCTTTTACAGAATC TCCTCGAACAGCCGGTAGC CGGTCACGCTTCCCCCACC GCCCTGTC | Cloning of SmBiT in pEVeLOA | SEQ ID NO: 36 |
| CGGACAGGGCGGTGGGGG AAGCGTCTTCACACTCGAA GATTTCGTTGG | Cloning of LgBiT in pEVeLB | SEQ ID NO: 37 |
| CAAGCTTTTAGCTGTTGATG GTTACTCGGAACAGC | Cloning of LgBiT in pEVe vectors | SEQ ID NO: 38 |
| ACCTGTGGTTGCGCCTGCA CCGGCTCCTGCACCATAAA AGCTTGACCTGTGAAGTGA AAAATGGC | Cloning of LOA in pEVeLOA | SEQ ID NO: 39 |
| AGCCGGTGCAGGCGCAACC ACAGGTGCTGCTTCTCCTT | Cloning of LgBiT in pEVeLOALB | SEQ ID NO: 40 |
| GACCGAATTGCCACTTAATG CC | | |
| ACCTGTGGTTGCGCCTGCA CCGGCTCCTGCACCAGTCT TCACACTCGAAGATTTCGTT GG | Cloning of LOA in pEVeLOA | SEQ ID NO: 41 |
| CACGTCCACCAACACCGCG GGTCCCCCTGTATGCCTCC AT | Cloning of *flu*330 constructs in pAK-Not vectors | SEQ ID NO: 42 |
| ATGGAGGCATACAGGGGGA CCCGCGGTGTTGGTGGACG TG | Cloning of *flu*330 constructs in pAK-Not vectors | SEQ ID NO: 43 |
| CACGTCCACCAACACCGCG GACGAATGTCACTCTCGCC TC | Cloning of *flu487* constructs in pAK-Not vectors | SEQ ID NO: 44 |
| GAGGCGAGAGTGACATTCG TCCGCGGTGTTGGTGGACG TG | Cloning of *flu487* constructs in pAK-Not vectors | SEQ ID NO: 45 |
| CACTTCACAGGTCAAGCTTT TAGAAGGTCACATTCAGTGT GGCC | Cloning of flu constructs without fusions in pAK-Not vectors | SEQ ID NO: 46 |
| GGCCACACTGAATGTGACC TTCTAAAAGCTTGACCTGTG AAGTG | Cloning of flu constructs without fusions in pAK-Not vectors | SEQ ID NO: 47 |
| CCACACTGAATGTGACCTTC GGTCAAGGAGAAGCAGCAC CT | Cloning of LOA-LgBiT in flu constructs | SEQ ID NO: 48 |
| GGTGCTGCTTCTCCTTGAC CGAAGGTCACATTCAGTGT GG | Cloning of LOA-LgBiT in flu constructs | SEQ ID NO: 49 |
| CTGCACCGGCTCCTGCACC AGTGACCGGCTACCGGCTG TT | Cloning of LOA-SmBiT in flu constructs | SEQ ID NO: 50 |
| AACAGCCGGTAGCCGGTCA CTGGTGCAGGAGCCGGTGC AG | Cloning of LOA-SmBiT in flu constructs | SEQ ID NO: 51 |
| ATCGTCGACCTTAACAAAGT GGGTATCAACACTTTGCC | Cloning of invasin constructs maintaining the D1 domain | SEQ ID NO: 52 |
| TTTCTTGATTAACCGCATCG CC | Check cloning of invasin constructs | SEQ ID NO: 53 |
| AAATGAACTATCGCTTTGGC GAGAG | Mutation of invasin leucine 352 to phenylalanine | SEQ ID NO: 54 |
| TCGCCAAAGCGATAGTTCAT TTGG | Mutation of invasin leucine 352 to phenylalanine | SEQ ID NO: 55 |
| ACTGGTCGACCTGAGGCTG CTGAACG | Cloning of invasin constructs without the D1 domain | SEQ ID NO: 56 |
| GTGACCGGCTACCGGCTGT TCGAGGAGATTCTGGGCGG AGGGTCTAAATATGATGCTA ACGCACCGCAACAG | Cloning of SmBiT in plFDVeSB | SEQ ID NO: 57 |
| CAGAATCTCCTCGAACAGC CGGTAGCCGGTCACGCTTC CCCCACCTTCTGAAGCCCC AGCCATGAAC | Cloning of SmBiT in plFDVeSB | SEQ ID NO: 58 |
| TCATGGCTGGGGCTTCAGA AGGTGGGGGAAGCGTCTTC AC | Cloning of LgBiT in plFDVeLB | SEQ ID NO: 59 |
| GTGAAGACGCTTCCCCCAC CTTCTGAAGCCCCAGCCAT GAAC | Cloning of LgBiT in plFDVeLB | SEQ ID NO: 60 |
| TCAACAGCGGCGGAGGGTC TAAATATGATGCTAACGCAC CGCAACAG | Cloning of LgBiT in plFDVeLB | SEQ ID NO: 61 |
| GGTGCGTTAGCATCATATTT AGACCCTCCGCCGCTGTTG A | Cloning of LgBiT in plFDVeLB | SEQ ID NO: 62 |
| GCGCATATTGTGATGGTTG ATGCCTATAAACCGACCAA GGGCGGAGGGTCTAAATAT GATGCTAACGCACCGCAAC AG | Cloning of SpyTag in plFDVeSpy | SEQ ID NO: 63 |
| CTTGGTCGGTTTATAGGCAT CAACCATCACAATATGCGC | Cloning of SpyTag in plFDVeSpy | SEQ ID NO: 64 |
| GCTTCCCCCACCTTCTGAA GCCCCAGCCATGAAC | | |
| AAACTGGGCGATATTGAATT TATCAAGGTGAACAAAGGC GGAGGGTCTAAATATGATG CTAACGCACCGCAACAG | Cloning of SnoopTag in plFDVeSnoop | SEQ ID NO: 65 |
| TTTGTTCACCTTGATAAATT CAATATCGCCCAGTTTGCTT CCCCCACCTTCTGAAGCCC CAGCCATGAAC | Cloning of SnoopTag in plFDVeSnoop | SEQ ID NO: 66 |

### 3. E. coli transformation and chromosome modification.

Replicative plasmids and Gibson assembly reactions were transformed into the appropiate *E. coli* strains using the Transformation and Storage Solution (TSS) method (Chung et al., 1989. Proc Natl Acad Sci U S A 86, 2172-2175). DNA ligations and suicide pGE plasmids were electroporated into electrocompetent bacteria. Conjugation of pGECs plasmids was performed using *E. coli* MFDpir as the donor strain.

Site-directed integration of DNA sequences into the *E. coli* chromosome was done following the marker-less I-*Sce*l endonuclease expression method (Pósfai et al., 2006, Science 312, 1044-1046; Pósfai et al., 1999. Nucleic Acids Res 27, 4409-4415), leaving no antibiotic resistance or recombination sequence in the chromosome of the modified strains. In short, the *E. coli* strain to be modified was previously transformed with the pACBSR plasmid (Cm^{R}) (Herring et al., 2003. Gene 311, 153-163) expressing I-Scel and *λ* Red proteins under control of the L-ara inducible promoter P_{BAD} and subsequently electroporated with the corresponding pGE-based suicide vector (Km^{R}). Co-integrants were selected using LB-Km-Cm plates grown at 37 ºC. Isolated colonies were used to inoculate LB cultures containing Cm and L-arabinose that were grown during up to 6 h at 37 ºC with agitation (200 rpm). Approximately ~1 µL sample of the grown cultures was streaked on LB-Cm plates using an inoculating loop and incubated O/N. Individual colonies were replica-plated in LB-Cm and LB-Km-Cm plates to screen for the Km-sensitive colonies that had deleted the vector sequences after the endonuclease expression. Individual Km-sensitive colonies were checked by PCR using specific oligonucleotides to identify clones with the chromosomal integration in the targeted locus.

### 4. Flow cytometry.

Detection of chimeric proteins with Nb on the bacterial surface and binding to soluble antigen were analyzed by flow cytometry. After induction, a volume of the cultures equivalent to 1 OD₆₀₀ was centrifuged (4000 g, 3 min), washed 3 times with 500 µl of PBS and resuspended in 400 µl of the same buffer. Next, 180 µl of the cell resuspension were incubated for 1 h at room temperature with 20 µl of the primary antibody (for display detection) or biotinylated EGFR fused to Fc (50 nM). For intimin and invasin constructs, the primary antibody was a mouse anti-c-myc monoclonal antibody (1:500; 9B11 clone; Cell Signaling, Ref: 2276) unless indicated, for autotransporter constructs, mouse anti-E-tag antibody (1:500; Phadia) was used. After incubation, bacteria were washed 3 times with PBS and incubated in the same manner with the secondary reagent: for display detection, a goat anti-mouse IgG-Alexa 488 conjugated polyclonal antibody (1:500; Life technologies, Ref: A11029), and for detection of the biotinylated antigen, Streptavidin-APC (1:100; Beckman Coulter, Ref: 733001). Finally, samples were washed 3 times with PBS and resuspended in 500 µl of the same buffer for analysis in a Gallios cytometer (Beckman Coulter, Inc).

For flow cytometry of bacteria expressing GFP under the control of tumor sensing promoters and integrases, grown cultures were diluted 1:100 and directly measured using either a Gallios cytometer (Beckman Coulter Inc.) or a Attune NxT flow cytometer (Thermo Fisher Inc.), respectively.

### 5. Protein electrophoresis and Western blots.

Electrophoresis in denaturing conditions using polyacrylamide gels containing SDS (SDS-PAGE) was performed following standard methods. A volume equivalent to 1 OD₆₀₀ of an induced culture was centrifuged (4000 g, 3 min) and resuspended in 100 µL of Tris-HCl 10 mM pH 8. Next, 100 µL of SDS loading buffer 2x (120 mM Tris-HCl pH 6.8, 2% (p/v) SDS, 2% (v/v) β-mercaptoethanol (2-ME), 10% glycerol and 0.01% (p/v) bromophenol blue) were added. Samples were either boiled (100 ºC, 10 min) or not, as specified, and centrifuged (21.000 g, 10 min) to eliminate any insoluble material (e.g., the peptidoglycan). 100 µL of the supernatants were transferred to new tubes from which up to 20 µL were loaded into 8% polyacrylamide gels for intimin, invasin and flu constructs and 10% polyacrylamide gels for EhaA constructs. Electrophoresis was performed in a Miniprotean III chamber (Bio-Rad).

Due to their high resistance to denaturation of intimin and invasin, samples containing these proteins were prepared in 2x urea-SDS loading buffer (120 mM Tris-HCl pH 6.8, 4% (p/v) SDS, 10 mM EDTA, 8 M urea, 2% (v/v) 2-ME, 10% glycerol and 0.01% (p/v) bromophenol blue). When boiled, these samples were kept at 100 ºC for 30 min. For Western blot analysis gels were transferred to a polyvinylidene difluoride membrane (PVDF, Immobilon-P, Merck-Millipore) in semi-dry conditions as previously reported (Bodelon et al., 2009. J Bacteriol 191, 5169-79; Salema et al., 2013, PLoS ONE 8, e75126). Detection of protein constructs was achieved by incubating the membrane in PBS containing 3% (w/v) non-fat-milk and 1% (w/v) BSA, and the indicated primary antibodies: mouse anti-E-tag mAb (1 :5000; Phadia, Thermo Fisher Scientific), mouse anti-c-myc mAb (1:2500; 9B11 clone; Cell Signaling, Ref: 2276). A goat anti-IgG mouse conjugated to peroxidase (POD) (1:5000; Sigma A2554) was used as a secondary antibody. Membranes were developed by measure of chemiluminescence using Clarity Western ECL Substrate (Bio-Rad ref. 1705061) by ChemiDoc Touch system (Bio-Rad). Precision Plus Protein Dual Color Standards (Bio-Rad) was used as molecular weight marker.

### 6. In vivo protein cross-linking.

Cross-linking of the intimin constructs was performed according to Thanabalu *et al.,* 1998 (Thanabalu et al., 1998 The EMBO Journal 17, 6487-6496). Induced cultures of *E. coli* EcM1 transformed with the different plasmids were centrifuged (4000 g, 3 min) and a 1:10 or 1:100 of the induction volume was resuspended in PBS with 2,5 mM of dithiobis(succinimidyl propionate) (DSP, Pierce) and incubated for 30 min at room temperature. Reaction was stopped using 50 mM Tris-HCl pH 7,5 and after 15 min bacteria were washed twice with 10 mM Tris-CI pH 7,5 and finally resuspended in 200 µL of the same buffer. Samples were divided in two and the same volume of SDS loading buffer 2x, with or without 5% (v/v) 2-ME, was added. The 2-ME reduces the internal disulfide bond of DSP, breaking the covalent crosslinkings.

### 7. Outer membrane protein extraction.

Induced E. *coli* EcM1 cultures expresing intimin, invasin or autotransporters constructs were centrifuged (3000 g, 5 min) and pellets were air-dried and frozen at - 80 ºC. After tawing in ice, the pellets were resuspended in 2 mL TN buffer (20 mM Tris-HCl pH 8, 10 mM NaCl) supplemented with 0.1 mg/mL DNAse I (Roche), 0.1 mg/mL pancreatic RNAse A (Amresco), 0.1 mM phenylmethylsulfonyl fluoride (Sigma) and a protease inhibitor cocktail (Complete EDTA-free, Roche). The resuspended culture was then lysed by sonication (7 pulses of 30 sec, with the tip being cooled between pulses) and the resulting extract was centrifuged to eliminate non-lysed bacteria (1500 g, 10 min, 4 ºC). The supernatant was then ultracentrifuged (100.000 g, 1 h, 4 ºC, Beckman Optima-Max XP) and the pellet, corresponding to the totality of the membranes, was resuspended in 2 mL TN Buffer with 1.5 % (v/v) Triton-X-100 (Sigma) (Schnaitman, 1971, J Bacteriol 108, 545-552) in order to eliminate the fraction corresponding to the inner membrane proteins. Resuspension was aided by using an ultrasonic bath for 10 sec. After a second ultracentrifugation the resulting pellet correspond to the outer membrane and insoluble remains. To obtain the outer membrane proteins, the pellet was resuspended in 1 mL TN buffer with 1% (p/v) Zwittergent 3-14 (Calbiochem) by incubating at 4 ºC using a rotator for 1 h. A final ultracentrifugation results in the outer membrane proteins recovered in the supernatant.

### 8. Blue Native-PAGE.

In order to assess the oligomeric nature of the new adhesins generated in this work, the proteins extracted from the outer membrane were subjected to non-denaturing electrophoresis in polyacrylamide gels using Coomassie Blue G-250 a negative charge provider (Blue Native-PAGE). Glycerol (Merck) and Coomassie Blue G-250 (Bio-Rad) were added to the outer membrane protein extracts to a final concentration of 8.7% (v/v) and 0.5%, respectively, and 20 µL of each sample were loaded into a 4-20% polyacrylamide gradient, Tris-Glycine native gel (Invitrogen). The cathode buffer composition was 50 mM tricine (Fluka), 15 mM Bis/Tris/HCl pH 7 (Fluka) and 0.002% Coomassie Blue G-250, while the anode buffer was 50 mM Bis/Tris/HCl pH 7. Native molecular weight markers (66-669 kDa; GE Bioscience) were resuspended to a final concentration of 2.5 mg/ml in 50 mM Bis/Tris/HCl ph7 and 750 mM aminocaproic acid, and 5 µL were loaded into the gel. Using a Mini-Gel-Tank (Invitrogen), the electrophoresis was first run at 100 V for 45 min, until samples have successfully penetrated the gel, and later at 500 V until the dye front has reached the gel's border. For Western blot analysis, the gel is transferred to a PVDF membrane following standard methods. To unfold the proteins and facilitate antibody access, after transferring the membrane was incubated in 8% acetic acid (Panreac) for 15 min, rinsed in de-ionized water and air dried, after which it was re-wet with methanol (MERCK) for 1 min to remove the excess of Coomassie which helped visualizing the molecular weight markers. The membrane was rinsed in PBS with 0.05% (v/v) Tween 20 (PBS-Tween) before continuing with the standard Western blot protocol.

### 9. Luminescence assays

For detection of luminescence variations due to the presence of EGFR-Fc bacteria were induced for expressing the modified adhesins as above. A volume of the cultures equivalent to 1 OD₆₀₀ was centrifuged (4000 g, 3 min), washed 3 times with 500 µl of PBS and resuspended in 200 µl of PBS and BSA 1% (w/v). A 100 µl volume of each resuspended culture was placed in a 96-well black plate with flat clear bottom (Corning). EGFR-Fc (0.5, 5, 50 or 200 nM) or PD-L1-Fc (50 nM) as a control were added to the wells and incubated at room temperature for 1 h. After the incubation the plate was placed in an orbital shaker at 37 ºC for 5 min and 25 µl of the Nano-Glo^{®} Live Cell Reagent (Promega) were added to each well immediately before measuring luminescence (full spectrum, 2 sec integration time) and OD₆₀₀ using a Spectramax iD5 Multi-mode Microplate Reader (Molecular Devices). Plate was shaken for 3 sec prior to the measurements.

### 10. Software and web tools.

In silico DNA visualization and analysis was done using DNASTAR Lasergene 17 and Benchling softwares. Schematic representation of circuits was based in the Synthetic Biology Open Language (SBOL) Visual Glyphs (Galdzicki et al., 2014. Nat Biotechnol 32, 545-550). Secondary structure prediction for intimin helices was a consensus from the results of the web tools PsiPred (Bio.tools), GOR (Center for Informational Biology, Ochanomizu University) based on (Garnier et al., 1978. Journal of Molecular Biology 120, 97-120), APSSP (Bioinformatics Centre Institute of Microbial Technology) and CFSSP (Ashok Kumar, 2013. CFSSP: Chou and Fasman Secondary Structure Prediction server). Cytometry analysis of integrases experiments used Flow-Jo (Treestar, Inc.), while promoters and adhesins experiments were analyzed using Kaluza (Beckman Coulter, Inc.). Statistical analysis was performed using GraphPad Prism 9.4 (GraphPad Software, LLC.) AlphaFold predictions were kindly provided by Dr. Florencio Pazos from the Computational Systems Biology group (CNB-CSIC), and the results were visualized using Mol* Viewer (Sehnal et al., 2021 Nucleic Acids Research 49, W431-W437).

### RESULTS

For a better understanding, the following references are used in the examples below:

| Protein name | | Amino acid sequence | Nucleotide sequence |
|---|---|---|---|
| NDVeLB | Based on intimin β-barrel | SEQ ID NO: 10 | SEQ ID NO: 16 |
| NDVeSB | | SEQ ID NO: 11 | SEQ ID NO: 17 |
| IFDVeLB | Based on invasin β-barrel | SEQ ID NO: 12 | SEQ ID NO: 18 |
| IFDVeSB | | SEQ ID NO: 13 | SEQ ID NO: 19 |
| VeALOALB | Based on EhaA β-barrel | SEQ ID NO: 14 | SEQ ID NO: 20 |
| VeASB | | SEQ ID NO: 15 | SEQ ID NO: 21 |

### Example 1: Engineering intimin for outer membrane sensors.

### Modification of the periplasmic domain of intimin.

The outer membrane anchoring fragment of a synthetic adhesin is based on the protein intimin, from its N-terminus up to the D0 Ig-like domain. Intimin has been reported to form homodimers and the generation of this dimers has been attributed to the periplasmic segment containing the LysM domain followed by a short peptide spacer. It was first evaluated whether deletion of the LysM and spacer region enabled the production of a monomeric intimin-nanobody fusion. As a model, the plasmid pNVEGFR2, encoding a synthetic adhesin with a nanobody binding the human epidermal growth factor receptor (hEGFR) was used. The LysM domain includes residues 63 to 112 of intimin and the present deletion comprised residues 54 through 153 to also delete the spacer. In addition, in order to facilitate future modifications, an EcoRI restriction site was placed at position 44 and a Sacll site at position 54 of intimin. The resulting plasmid was termed pNDVe. Following the LysM and spacer region, it was noticed that the periplasmic region of intimin also contains two α-helices that are connected to the β-barrel. The role of these α-helices in dimerization and/or folding of intimin has not been reported. Thus, a second construct deleting the LysM domain, the spacer and these two α-helices was also generated. As the α-helices had not been determined in experimentally, four different secondary structure prediction algorithms (PsiPred, GOR, APSSP and CFSSP) were used to determine their more likely position, selecting the amino acids 154 through 206 as consensus. Therefore, this second deletion comprised amino acids 54 through 206 and the plasmid was named pNDHVe. A graphic representation of the different aforementioned constructs can be found in Figure 1.

The above intimin-nanobody constructs were tested for their capacity to be displayed on the bacteria surface, as well as for the functionality of the nanobody to bind its antigen (i.e., hEGFR-Fc) (Figure 2). Flow cytometry analysis of the transformed bacteria showed that the deletion of LysM-spacer in pNDVe did not affect the display nor the binding of the nanobody, with values almost identical to the non-modified construct pNVEGFR2. On the other hand, deletion comprising LysM-spacer and the α-helices severely hindered the capacity for both display and antigen-binding of the bacteria, which were close to the negative control (pAK-Not).

Whole-cell protein samples from the above cultures were subjected to a Western blot analysis with anti-myc monoclonal antibody (mAb) (Figure 3). The Western blot showed that the three proteins were expressed, with protein bands having mobilities of the expected size for the full-length proteins, and of different mass according to their deletions in the N-terminal. Nonetheless, deletions seemed to increase the proteolysis of the fusions *in vivo,* especially for the longer deletion, with a reduced intensity for the full-length protein band and a significant higher intensity of a band of smaller mass corresponding to a proteolytic fragment of ~40 kDa

Taken together, the flow cytometry and Western blot results indicated that deletion of the two α-helices was severely affecting the stability of intimin, suggesting an impaired folding and/or insertion in the outer membrane. At this point, the inventors wondered whether the lack of the LysM-spacer region (LysM-deletion hereafter) could also affect the stability of the protein. Western blot was used to compare the mobility shift on SDS-PAGE of the β-barrels from unmodified intimin and the LysM-deletion mutant under denaturing (i.e., boiled in SDS-urea buffer) and non-denaturing conditions (i.e., samples in SDS-urea buffer at room temperature) (Figure 4A). Without boiling, the unmodified intimin-nanobody fusion showed an intense band of high mobility and a faint band of low mobility on SDS-PAGE. When boiled, the only band present for this parental fusion was that of low mobility, which corresponds to the fully denatured polypeptide. This mobility shift is characteristic of outer membrane proteins containing stable β-barrels and indicate that, without boiling, the intimin β-barrel remains folded even in the presence of SDS-urea. On the other hand, the unboiled sample of the LysM-deletion mutant presented additional protein bands between those of high and low mobility, which indicated the presence of some partial unfolded states of the β-barrel. This partial unfolding in the presence of SDS-urea is suggestive of a lower stability of the β-barrel in the LysM-deletion mutant. To rule out an effect of the nanobody on the folding of the β-barrel, this mobility-shift assay was performed with constructs having a six histidines peptide (6×His) instead of the nanobody fused to the parental intimin fragment (pNeae2) or the LysM-deletion mutant (pND) (Figure 4B). Results were similar to those observed with the fusions having the nanobody, showing that the lower stability of the β-barrel of the LysM-deletion mutant is not due to the nanobody. Interestingly, the proteolytic bands of ~40 kDa were not present here, which suggested that they may result from cleavage of a C-terminal fragment containing the nanobody and myc-tag. We also examined the effect of the presence of β-mercaptoethanol (2-ME) in these samples, but this reducing agent of disulfide bridges did not affect differently these protein samples. Therefore, these results suggested a role of the periplasmic LysM in the stability of the outer membrane β-barrel of intimin. Although a direct interaction between these different domains cannot be excluded, it is more likely that LysM plays an indirect role due to its binding to the peptidoglycan. LysM binding to the peptidoglycan could stabilize intimin in the bacterial envelope and in the SDS-urea sample buffer.

Next, it was tested whether lack of the LysM-spacer region could prevent formation of intimin homodimers. Thus, the oligomerization state of intimin constructs with and without the LysM-spacer region was investigated. To avoid any potential interference of the nanobody in the oligomerization state, the constructs having the 6×His peptide tag were used. In the first approach, a crosslinking assay was performed using bacteria expressing these intimin fusions. Different concentrations of the dithiobis(succinimidyl propionate) (DSP) crosslinker agent were used on the intact bacteria, and the resulting samples were subjected to SDS-PAGE and Western blot. It was found that both wild-type and LysM-deletion constructs presented crosslinking bands with an apparent size close to the expected size of the corresponding protein dimers (Figure 5A). These bands were not present when the crosslinked samples were treated with the reducing agent 2-ME which reduces the disulfide bridge present in the DSP reagent, cleaving the protein crosslinks (Figure 5B). This result was compatible with formation of homodimers by both intimin and the LysM mutant.

To confirm this result, the proteins from the outer membrane fractions (Materials and methods: Outer membrane protein extraction) of induced bacteria having pNeae2 or pND constructs were extracted. Samples were run in native conditions by Blue Native Polyacrylamide Gel Electrophoresis (BN-PAGE) and transferred to a membrane for Western blot developed with anti-myc mAb (Figure 6). This analysis showed that both intimin constructs produced protein bands having apparent molecular masses that are in agreement with the formation of homodimers. Taking together protein crosslink and mobility on native BN-PAGE, this data indicated that deletion of LysM domain in intimin did not prevent its dimerization in the outer membrane. This conclusion meant that intimin-based proteins cannot be used as the only components of an outer membrane sensor relying on antigen-driven dimerization, as the endogenous dimer formation of intimin would trigger a constant activation of the system. However, intimin constructs could still be used in combination with other outer membrane proteins able to display nanobodies.

Modification of the periplasmic region of the intimin LysM-deletion mutant with protein fragments of a reporter system.

Before exploring the use of intimin constructs in combination with other outer membrane proteins, it was still needed to test its capacity to tolerate the fusion in its periplasmic side of proteins fragments that could be employed in the generation of a periplasmic signal. Since deletion of LysM may improve the diffusion of the protein in the outer membrane, and thus, its possibility to interact with other outer membrane proteins, the intimin-nanobody construct lacking LysM (pNDVe) was chosen as the basis of the sensor constructs of the present invention. It is important to remind here that LysM-deletion did not affect the surface display of the intimin construct nor the antigen binding activity of the nanobody.

Next, a protein reporter for testing dimerization of periplasmic domains linked to intimin was chosen. The NanoBiT system (Dixon et al., 2016. ACS Chem. Biol. 11, 400-408) was selected as a reporter candidate. NanoBiT is a protein complementation assay based on a split NanoLuc luciferase with two protein fragments: the small SmBiT, a 1.3 kDa-peptide, and the large LgBiT, an 18 kDa-polypeptide. NanoBiT is able to emit light once the two protein fragments interact to reconstitute the Nanoluc luciferase. However, this association *in vivo* is neglectable unless these fragments are linked to a pair of interacting proteins, which constitute an excellent reporter for protein-protein interactions. Further, the bioluminescence assay of Nanoluc is reported to be very sensitive, simple, fast, and robust at different temperatures.

Both SmBiT and LgBiT were cloned into pNDVe plasmid in frame between the amino acids 43 and 154 of intimin positions, using the inserted EcoRI and Sacll restriction sites. These fusions placed the NanoBiT fragments in the periplasmic domain of the intimin-nanobody fusion, between the N-terminal signal peptide and the periplasmic α-helices. Two Gly-Ser flexible peptide linkers (GGGS) were placed at both ends of the NanoBiT fragments. The plasmid carrying the SmBiT was named pNDVeSB and the one carrying the LgBiT was named pNDVeLB (Figure 7).

Flow cytometry was used to test the display and antigen binding of these constructs (Figure 8). The protein fusion to the SmBiT fragment showed display and antigen binding values almost identical to the parental fusion NVDe, which indicated that the presence of the SmBiT fragment did not affect insertion into the outer membrane nor the functionality of the displayed nanobody. On the contrary, the protein fusion to LgBiT showed a significant reduction in the surface display levels, with values of -35% of the parental, and even a higher difference in antigen binding activity (-20% of the parental). Western blot analysis of whole-cell protein extracts from these cultures (Figure 9) showed similar expression levels of NDVe and NDVeSB fusions, with the small difference in size (11 aas) not detectable by SDS-PAGE. The protein sample from bacteria carrying pNDVeLB presented a main protein band of higher size, as expected for the insertion of the LgBiT 158 residues, and a faint band of lower size, similar to parental fusion, which probably suggested some proteolysis of the LgBiT fragment.

The deep-learning algorithm AlphaFold (Jumper et al., 2021. Nature 596, 583-589.) was used to predict the structure of the protein fusion to the LgBiT fragment. The prediction estimated a correct folding of all the domains, including the LgBiT, which was placed in the periplasm and linked to the β-barrel of the adhesin through highly flexible helices (Figure 10).

The above results suggested that the LysM-deleted intimin-nanobody construct NDVe can be fused to small peptide fusions in its periplasmic region without alterations in surface display or antigen-binding levels, but larger polypeptide fragments are not well tolerated and reduce the expression levels and binding activity of the fusion. The presence of large folded fragments in the periplasm might be interfering with the process of insertion of the β-barrel in the outer membrane through the BAM complex and/or the translocation of its passenger (nanobody). Therefore, the inventors thought of an alternative that could allow the covalent linkage of large protein reporters once the protein fusion was already correctly inserted in the outer membrane. The SpyTag-SpyCatcher and SnoopTag-SnoopCatcher are homologous but orthogonal systems that allow the post-translational fusion of proteins (Hatlem et al., 2019. IJMS 20, 2129). Both are derived from fimbrial subunits from *Streptococcus* species with an intradomain isopeptide bond. These fimbrial domains can be split into a small peptide (the Tag) that form a covalent isopeptide bond with the large fragment of the domain (the Catcher), which is catalytically active. Each of these components of a pair can be fused to different proteins to covalently link them through an isopeptide bond.

In the present system, the Tag-Catcher partners could be used to covalently attach a large fragment of a reporter enzyme in the periplasm fused to a Catcher domain if a small Tag peptide is placed in the periplasmic domain of intimin. Thus, it was decided to test the tolerance of the NVDe fusion to both SpyTag and SnoopTag peptides, generating similar plasmid constructs pNDVeSpy and pNDVeSnoop, respectively (Figure 11). As could be expected from the results with SmBiT fusion, these small peptide Tags did not affect the surface display nor the antigen binding activity of the protein fusions as determined by flow cytometry (Figure 12). Also, the expression levels of these protein fusions were found similar to the parental NVDe construct in Western blot (Figure 13).

Therefore, through this section the inventors have developed a new set of intimin-nanobody fusions that, although maintain the dimeric nature of intimin after deletion of the LysM domain, were able to simultaneously display a functional nanobody on the bacterial surface and a peptide or polypeptide fragment in the periplasm. These constructs hold a great potential for the generation of an outer membrane sensor, but first we needed to find a suitable partner that would not dimerize with intimin.

### Example 2: Engineering autotransporters for outer membrane sensors.

### Engineering the EhaA autotransporter.

First, the nanobody binding human EGFR fused to the C-terminal β-barrel domain of EhaA (C-EhaA) was cloned. To this end, the anti-GFP nanobody found in a similar fusion, encoded by pVgfpA (Salema et al., 2013. PLoS ONE 8, e75126), was replaced, generating the plasmid derivative pVeA. The surface display and antigen binding levels of this construct were found low compared to those of pNDVe (compare Figure 2 with Figure 15).

The outer membrane protein OmpA from *E. coli* is composed of an N-terminal β-barrel embedded in the outer membrane, with a terminal F residue in the last β-strand, followed by a C-terminal periplasmic domain. These two domains are connected through an unstructured 17-aa linker (GQGEAAPVVAPAPAPAP, SEQ D NO: 67) that functions as a peptidoglycan clamp. The first three aa of the linker (GQG) were chosen and used them to connect the C-terminal end of EhaA β-barrel to the NanoBit domains preceded by a flexible GGGS linker, obtaining the plasmids pVeASB and pVeALB (Figure 14). Flow cytometry analysis of these constructs showed that, in a similar situation to intimin-based fusions, the small peptide SmBiT did not hinder the display nor the antigen binding of the nanobody, but the LgBiT fragment made the display and binding signals drop to values close to the negative control. Western blot analysis of whole-cell protein extracts from the induced cultures showed that both constructs were expressed, being the LgBiT chimera even expressed at higher levels than the SmBiT fusion.

It was suspected then that the close presence of a large folded domain could be affecting the insertion and folding of the EhaA β-barrel despite the presence of the short peptide linkers connecting them. To increase the distance between these protein domains, the full-length linker of OmpA (LOA) to directly attach the LgBiT domain to the β-barrel was tested, creating the plasmid pVeALOALB. As a control, the LOA was fused to the barrel without LgBiT, generating pVeALOA. Flow cytometry of induced bacteria with these constructs revealed that the LOA did not affect the surface display nor the antigen binding signals of pVeA (Figure 38). Moreover, the presence of LOA was capable of restoring the surface display and antigen binding activity to the fusion carrying LgBiT, showing values similar to the positive control pVeA. Western blot analysis of bacteria from these cultures showed the expected pattern of protein bands for these constructs according to their mass, with no signs of higher proteolysis (Figure 16). In fact, higher expression levels in the protein fusions carrying LgBiT and LOA were observed.

AlphaFold was used to predict the sequence of these constructs. The algorithm predicted that the β-barrel is correctly folded in all constructs, and expected a flexible unstructured region for the peptides SmBiT and LOA. Interestingly, AlphaFold predicted a highly disorganized LgBiT domain in the case of pVeALB, with limited confidence in the result. The prediction of a folded LgBiT domain improved once the LOA was used to increase the distance between the β-barrel and the LgBiT fragment (Figure 17).

At this point, it was generated a potential partner for the intimin construct with SmBi and LgBi reporter system. EHaa-Based fusion could be engineering to display a nanobody while simultaneously carrying a periplasmic reporter.

### Example 3: Engineering of invasin for outer membrane sensors.

Invasins are inverted autotransporters proteins found in *Yersinia* species that present a high homology to intimin in their overall structure and β-domains. In most cases, invasins do not present a periplasmic LysM domain. We chose the invasin (*invA*) from *Yersinia pseudotuberculosis* to generate a new set of constructs by replacing the intimin Neae fragment encoding the β-domain. The crystal structure of the β-barrel of this invasin has been solved and was reported to be monomeric. We selected the N-terminal 595 residues of invasin, from its signal peptide to the D1 immunoglobulin-like domain, in an equivalent structure to the Neae fragment used in the intimin fusions lacking the LysM domain. The D2 domain of invasin, which had been suggested to be responsible for the oligomerization of the full-length invasin of *Y. pseudotuberculosis,* was excluded, in contrast to other monomeric invasins such as the one from *Y*. *enterocolitica.*

Replacing the intimin backbone in pNVegfr2 for the aforementioned 1-595 fragment from invasin, the construct pIVe (Figure 18) was generated. Flow cytometry analysis comparing surface display and antigen binding of bacteria showed low fluorescence signals for the invasin construct compared to intimin (Figure 19), with values close to those with EhaA constructs. Western blot analysis of the protein samples from these bacteria revealed that invasin-nanobody fusion presented a major band with a similar size and intensity that that of the intimin-nanobody fusion, but also showed a large amount of proteolysis (Figure 20).

Next, oligomerization state of the IFDVe construct was tested. The outer membrane proteins were extracted from induced *E. coli* bacteria carrying pIFVDVe and the samples were subjected to BN-PAGE and Western blot with anti-myc mAb (Figure 21). Migration of this fusion protein produced a protein band of -140 kDa, which is roughly double the expected size for the monomer, leading us to think that this invasion-based construct was also behaving as a dimer similar to intimin. Due to its dimeric nature, for its functioning in a dimerization sensor, this fusion would likely require its co-expression with the intimin or EhaA partners.

Finally, whether peptide and protein domains could be attached to the periplasmic side of the IFDVe protein without affecting its functionality was tested. Several fusions were tested between residues 51 and 52 of IFDVe having the SmBiT (pIFDVeSB), the LgBiT (pIFDVeLB), the SpyTag (pIFDVeSpy) or the SnoopTag (pIFDVeSnoop) (Figure 22). Flow cytometry analysis showed that these fusions did not affect the display nor the antigen binding of the nanobody, with values almost identical to the parental protein fusion without periplasmic modifications (Figure 23). Western blot of the protein samples from these bacteria showed major bands with the expected size for the full-length proteins of all constructs, with just some increased proteolysis for one of the fusions with LgBIT (Figure 24).

Once again, AlphaFold was used to predict the structure of the invasin constructs. The algorithm predicted the correct folding of the invasin β-barrel and of the extracellular domains as well as the presence of flexible α-helices in the periplasm. In the case of plFDVeLB, the LgBiT domain was predicted to be correctly folded (Figure 25). Interestingly, AlphaFold also predicted the presence of an immunoglobulin-like domain between the β-barrel and the D1 domain of invasin, in a similar case to the D00 domain of intimin (Figure 26).

Taken together, these results demonstrate that invasin-based constructs have the capacity to display a functional nanobody on the surface of *E. coli* while having a peptide and/or protein domain anchored in the periplasmic side.

A comparison of the predicted sequences of the β-barrel and displayed domains for the chimeras encoded by pNDVe and plFDVe can be found in Figure 27.

### Example 4: Detection of extracellular antigen by bacteria co-expressing intimin and invasin protein sensors in the outer membrane.

Next, the capacity of the modified nanobody surface display systems disclosed herein, based on intimin and invasin β-barrels and periplasmic split NanoLuc fragments, was investigated to detect an extracellular protein antigen (EGFR-Fc) and to produce a change in the bioluminescence due to dimerization of the β-barrels and interaction of the split NanoLuc fragments. In order to co-express two of these constructs in the same cell, we first cloned the gene fusions from pNDVe, pNDVeSB, plFDVe and plFDVeLB into the backbone of a compatible pSEVA221 vector under control of a tetR-Pₜₑₜ promoter region. The new plasmids (pSEVA221-Ptet-NDVe, pSEVA221-Ptet-NDVeSB, pSEVA221-Ptet-IFDVe and pSEVA221-Ptet-IFDVeLB), were co-transformed with either pNDVeLB or plFDVeLB, into *E. coli* EcM1 strain. Additionally, we generated a positive control (called pAK-pelB-Nanoluc) for the production of bioluminescence by cloning into the pAK-Not vector the full-length NanoLuc gene fused in frame with to the N-terminal signal peptide of PelB (Le Calvez et al., 1996, Gene 170, 51-55; Thie et al., 2008. New Biotechnology 25, 49-54) for its export into the bacterial periplasm. In our preliminary experiments we detected an impaired growth of bacteria carrying pNDVeLB (intimin and LgBiT) and a second plasmid, so we focused on combinations having plFDVeLB (invasin and LgBiT) and a second compatible plasmid.

In an initial experiment, the bioluminescence produced by the different combinations of IFDVeLB (SEQ ID NO:12) with either IFDVe, IFDVeSB (SEQ ID NO: 13) or NDVeSB (SEQ ID NO: 11) in the presence or not of their corresponding antigen, EGFR-Fc, or a control antigen PD-L1-Fc (Figure 28) was compared. As expected, the pair lacking the SmBiT, (IFDVeLB + IFDVe) produced low luminescence levels, comparable to the bacteria with the empty vector pAK-Not (negative control). On the other hand, both the IFDVeLB (SEQ ID NO: 12) + IFDVeSB (SEQ ID NO 13) and IFDVeLB (SEQ ID NO: 12) + NDVeSB (SEQ ID NO: 11) pairs showed light emission, with values ~10-fold below the luminescence found for the full-length NanoLuc in the periplasm (positive control). From the two combinations, the homodimer of invasin β-barrels pair showed higher luminescence. Unexpectedly, both chimeric protein pairs produced higher bioluminescence in the absence of the specific antigen than when EGFR-Fc was added. While this behavior was opposed to the original hypothesis, its potential for generating a biosensing platform was further investigated.

As the IFDVeLB (SEQ ID NO: 12) + NDVeSB (SEQ ID NO: 11) combination showed the higher difference in luminescence between the presence and the absence of EGFR-Fc, we used this pair to test how different concentrations of the antigen affected the emission of light (Figure 29). The results showed that the reduction of the luminescence was dose-dependent, with an inverted relationship between the antigen concentration and the emission of light. Luminescence showed no variation in the presence of the control antigen, nor in the presence of a low concentration of EGFR-Fc (0.5 nM) that is below the equilibrium dissociation constant of the displayed nanobody (K_{D} = 1.92 nM) (Salema et al., 2016b. mAbs 8, 1286-1301). These findings show that the system behaves as a bacterial biosensor that detect the presence of a soluble protein antigen in a dose-dependent manner and directly emits an easily quantifiable signal

### CONCLUSION

Detection of tumor associated protein antigens is also a potential way to specifically trigger diagnostic and therapeutic responses in engineered bacteria. The present invention discloses a whole-cell biosensor *E. coli* that is able to detect the presence of a tumor-associated protein, hEGFR-Fc, through the display in the outer membrane of chimeric proteins pairs consisting in an exposed nanobody fused to a membrane anchored T5SS β-barrel that carries a split enzyme fragment (NanoBiT).

The engineered chimeric proteins of intimin and invasin allowed the inventors to generate a whole-cell biosensor for detecting the presence of soluble hEGFR-Fc in a specific and dosage-dependent manner. The biosensor disclosed herein behaves in the opposite manner to what was expected: the inventors thought that the intimin and invasin constructs would not interact unless a dimeric antigen forced their dimerization, but luminescence was measurable in the absence of antigen and adding the antigen reduced the luminescence instead of increasing it.

The mechanism behind our sensor is clearly different to what we had expected and therefore we must evaluate our results to understand its behavior. BN-PAGE results showed that both our intimin and invasin constructs behave as homodimers. This premise was further supported by our *in vivo* luminescence measuring assays, where light emission was detected if invasin constructs carrying both NanoBiT fragments were co-expressed, indicating that they were interacting in a close-enough manner to reconstitute the split NanoLuc luciferase. This was also true for co-expression of the invasin construct carrying LgBiT and the intimin construct carrying SmBiT, which implies the spontaneous formation of heterodimers. These findings support the idea that heterodimers may be formed, at least in a transient way and lead the inventors to believe that the chimeric proteins of the invention may also be interesting tool for studying OMP interactions. It was also found that luminescence production was higher for a homologous pair than for a heterologous one, which suggest that homologous interactions may be more frequent. Addition of the dimeric antigen reduced luminescence, which must correlate with a reduction in the formation of heterologous pairs. This reduction of luminescence was proportional to the concentration of the antigen.

Taking into account the above findings, a model for the developed biosensor is proposed herein (Figure 30). In the absence of antigen, the nanobody-displaying constructs of intimin and invasin interact mainly as homodimers in the OM. However, they can also transiently form heterodimers, likely because homodimers are in equilibrium with free monomers of each β-barrel. Formation of these heterodimers forces the interaction of NanoBiT fragments generating luminescence in the presence of the luciferase substrate. When the dimeric antigen is added to the media, it may simultaneously bind to both nanobodies in a dimer stabilizing them. Antigen binding may not stabilize heterodimers in the same manner. Although not wishing to be bound by any theory, it might be related to the different symmetry of the displayed nanobodies in the homodimers vs. the heterodimers. In this scenario, increasing the concentration of antigen reduces the number of free monomers since they do not dissociate from the homodimers, which in turn reduces the pool of functional heterodimers. Thus, by increasing the antigen concentration, the production of luminescence is reduced, producing a measurable output.

It is believed that the modularity architecture of this biosensor brings a great potential to the system. Three main components that, in principle, could be modified as independent modules for modifying the sensor characteristics are distinguished: the nanobody, the OMP barrel and the split reporter.

Nanobodies confer to the system the specificity for the detected protein. The β-barrel, which acts as scaffold and OM anchor for the nanobody, and the reporter system. Three different barrels were engineered for their use in our biosensor based on invasin, intimin and the EhaA Ats, and their functionality in the system is demonstrated herein.

The bacterial antigen receptors (BAR) described in the present description are useful for detecting not only soluble antigen, but antigen present in both abiotic and cellular surfaces. Hence, the use of BAR might also signal bacterial adhesion to a tumor cell.

The final module (or domain), the reporter system, is based on the interaction of a split enzyme. Here, the split version of the NanoLuc luciferase, NanoBiT, was chosen due to its fast, simple and sensitive detection method. However, other split proteins have been developed for protein complementation assays (PCA) that measure protein-protein interactions can be used. Since all these split proteins are based in the same principle of interaction between their fused partners, they can be used as reporter systems for the biosensor of the present invention, broadening its potential for *in vivo* and *in vitro* diagnostic applications.

To sum up, in the present invention:
1. Chimeric outer membrane proteins that display a nanobody on the bacterial surface and a protein fusion in the periplasm have been generated. These chimeric proteins are based on the β-barrels of T5SS members intimin, invasin and EhaA. The periplasmic fusions tolerate short peptides (SmBiT, SpyTag, SnoopTag) as well as large fragments of enzymes (LgBiT).
2. It has been shown that intimin and invasin β-barrels form homodimers even in the absence of protein domains reported to participate in dimerization, such as the periplasmic LysM and its spacer on intimin and the surface-exposed D2 domain of invasin from *Y. pseudotuberculosis.*
3. The engineered chimeric outer membrane proteins disclosed in the present invention can generate *E*. *coli* bacteria able to detect the presence of an extracellular tumor associated antigen (i.e., human EGFR). This bacterial biosensor is based on the coexpression of two chimeric proteins consisting of a nanobody binding the protein antigen, a β-barrel domain of intimin or invasin and periplasmic fusions to the SmBiT and LgBiT fragments of NanoLuc luciferase. Bacteria produce a bioluminescent signal that is specifically reduced in the presence of the extracellular tumor antigen in a dose-dependent manner.

## Claims

1. A chimeric protein comprising
(i) an antigen binding domain,
(ii) at least one β-barrel domain, wherein the β-barrel domain is
(a) the β-barrel domain from an intimin protein,
(b) the β-barrel domain from an invasin protein,
(c) the β-barrel domain from EhaA autotransporter protein,
and
(iii) a reporter protein domain comprising a partner of a split protein luminescence system,
wherein domain (ii) is bound to domain (i) by one end and to domain (iii) by the other end.

2. Chimeric protein according to claim 1, wherein the chimeric protein further comprises an N-terminal signal peptide for translocation through the bacterial membrane.

3. Chimeric protein according to claim 1 or 2, wherein the antigen binding domain is selected from a single domain antibody, a single-chain Fv (scFv) or a fragment thereof, and an engineering protein binder with affinity for an antigen.

4. Chimeric protein according to any one of claims 1 to 3, wherein the antigen binding domain comprises an amino acid sequence having an identity of, at least, 80, 81, 82, 83, 84, 85, 86, 87, 88, 89, 90, 91, 92, 93, 94, 95, 96, 97, 98, 99% with the sequence SEQ ID NO: 1.

5. Chimeric protein according to any one of claims 1 to 4, wherein
(a) the β-barrel domain from an intimin protein comprises an amino acid sequence having an identity of, at least, 80, 81, 82, 83, 84, 85, 86, 87, 88, 89, 90, 91, 92, 93, 94, 95, 96, 97, 98, 99% with the sequence SEQ ID NO: 2, or
(b) the β-barrel domain from an invasin protein comprises an amino acid sequence having an identity of, at least, 80, 81, 82, 83, 84, 85, 86, 87, 88, 89, 90, 91, 92, 93, 94, 95, 96, 97, 98, 99%with the sequence SEQ ID NO: 3, or
(c) the β-barrel domain from the EhaA autotransporter comprises an amino acid sequence having an identity of, at least, 80, 81, 82, 83, 84, 85, 86, 87, 88, 89, 90, 91, 92, 93, 94, 95, 96, 97, 98, 99% with the sequence SEQ ID NO: 4.

6. Chimeric protein according to any one of claims 1 to 5, wherein the reporter signal domain is a luminescent protein, preferably luciferase Nanoluc protein; an AMD fluorescent protein, preferably, GFP; a β-lactamase protein; a TEV protease protein; or functionally equivalent fragments thereof.

7. A polynucleotide comprising a nucleotide sequence encoding a chimeric protein according to any one of claims 1 to 6.

8. A vector comprising a polynucleotide according to claim 7.

9. A host cell comprising
- at least one chimeric protein according to any one of claim 1 to 6, wherein domain (i) is displayed on the bacterial surface, domain (ii) is inserted in the outer membrane, and domain (iii) is display in the periplasm; and/or
- at least one polynucleotide according to claim 7; and/or
- at least one vector according to claim 8.

10. Host cell according to claim 9, wherein the host cell is a bacterium, preferably, a Gram-negative bacteria, more preferably, *Escherichia coli.*

11. Use of a host cell according to claim 9 or 10 comprising at least two chimeric proteins according to any one of claims 1 to 6, as an *in vitro* antigen detection/quantification system, wherein the reporter protein domains of the, at least, two chimeric proteins are complementary each other.

12. An *in vitro* method for detecting/quantifying antigens comprising
(a) contacting an isolated sample with a host cell according to claim 9 or 10 comprising at least two chimeric proteins according to any one of claim 1 to 6, wherein domain (iii) of the, at least, two chimeric proteins are complementary each other, and
(b) detecting and quantifying the signal emitted by the reporter,
wherein the intensity of the signal is inversely proportional to the concentration of antigen present in the sample.

13. Method according to claim 12, wherein the antigen is a viral antigen, a bacteria antigen, a tumoral antigen, or an inflammation protein antigen.

14. Method according to claim 12 or 13, wherein the sample is a saliva sample, a urine sample, a plasma sample, a water sample or a food sample.

15. A kit comprising a chimeric protein according to any one of claim 1 to 6, a polynucleotide according to claim 7; a vector according to claim 8 and/or a host cell according to claim 9 or 10.
